# FASCICULE DE BREVET EUROPEEN

(11) **EP 1 144 348 B1**
(45) Date de publication et mention de la délivrance du brevet: **08.02.2006**
(21) Numéro de dépôt: 99943016.8
(22) Date de dépôt: 21.09.1999
(51) Int. Cl.: C07C 323/25, C07C 327/30, C07D 213/64, C07D 317/64

(54) **INHIBITEURS DE LTA4 HYDROLASE**
LTA4 HYDROLASE-HEMMER
LTA 4 HYDROLASE INHIBITORS

(30) Priorité: 21.09.1998 FR 9811758
(43) Date de publication de la demande: 17.10.2001
(73) Titulaire: BIOPROJET, 75003 Paris (FR); INSTITUT NATIONAL DE LA SANTE ET DE LA RECHERCHE MEDICALE (INSERM), 75013 Paris (FR)
(72) Inventeur: DANVY, Denis, F-76190 Yvetot (FR); MONTEIL, Thierry, F-76690 Saint Georges sur Fontaine (FR); PLAQUEVENT, Jean-Christophe, F-76960 Notre-Dame de Bondeville (FR); DUHAMEL, Pierre, F-76130 Mont-Saint-Aignan (FR); DUHAMEL, Lucette, F-76130 Mont-Saint-Aignan (FR); NOEL, Nadine, F-68480 Moernach (FR); GROS, Claude, F-75015 Paris (FR); CHAMARD, Olivier, F-93600 Aulnay Sous Bois (FR); SCHWARTZ, Jean-Charles, F-75014 Paris (FR); LECOMTE, Jeanne-Marie, F-75003 Paris (FR)
(74) Mandataire: Bernasconi, Jean Raymond
(86) Numéro de dépôt international: PCT/FR1999/002240
(87) Numéro de publication internationale: WO 2000/017133

(56) Documents cités:
- WO-A-94/00420
- WO-A-96/30014
- FR-A- 2 604 434
- Y. QIAN ET AL: J. MED. CHEM., vol. 39, no. 1, 1996, pages 217-223, XP002106492
- M.C. FOURNIE-ZALUSKI ET AL: J. MED. CHEM., vol. 35, no. 7, 1992, pages 1259-1266, XP002106493
- H. TOH ET AL: BIOCHEM. BIOPHYS. RES. COMMUN., vol. 171, no. 1, 1990, pages 216-221, XP002106870

## Description

La présente invention concerne des composés tels que définis ci-après, constituant une classe de médicaments présentant principalement une activité anti-inflammatoire et/ou agissant par inhibition de la LTA₄ (leucotriène A₄) hydrolase, enzyme responsable de la biosynthèse du leucotriène LTB₄, un médiateur pro-inflammatoire important.

Elle concerne également des procédés de préparation de ces composés.

La LTA₄ hydrolase (EC 3.3.2.6.) est une enzyme notamment présente dans les neutrophiles dont on a récemment montré que la séquence (Funck et coll., P.N.A.S., 1987, 89: 6677) s'apparentait à celle d'une métallopeptidase à zinc, l'aminopeptidase M (Malfroy et al., B.B.R.C., 1989, 161: 236). En accord avec la suggestion de Malfroy et coll., la LTA₄ hydrolase a été reconnue posséder un atome de zinc essentiel à son activité catalytique, une activité de type aminopeptidasique et une sensibilité à l'action de certains inhibiteurs de métallopeptidases (Heggstrom et coll., B.B.R.C., 1990, 173: 431 ; Minami et coll., B.B.R.C., 1990, 173: 620).

L'inhibition de la LTA₄ hydrolase est de nature à prévenir la formation de LTB₄, un médiateur responsable de l'adhésion des neutrophiles aux cellules endothéliales et de leur chimiotactisme. Il paraît impliqué dans l'étiologie ou la symptomatologie d'une variété d'états inflammatoires et d'affections telles qu'arthrite rhumatoïde, inflammations chroniques de l'intestin, sclérose en plaques, goutte, psoriasis. Dans ces processus, le LTB₄ pourrait agir en synergie avec d'autres métabolites de l'acide arachidonique produits par la 5-lipoxygénase ou les cyclo-oxygénases dont l'inhibition est bien connue pour produire des effets anti-inflammatoires.

Certains composés inhibiteurs de la LTA₄ hydrolase ont été décrits, notamment dans les demandes de brevet WO 94/00420, WO 96/11192, WO 96/10999 et WO 96/27585.

L'objectif de la présente invention est de fournir de nouveaux composés susceptibles d'inhiber la LTA₄ hydrolase.

L'objectif de la présente invention est également de fournir des composés pouvant être utilisés comme médicaments.

A cette fin, l'invention a pour objet des composés de formule (I) telle que définis ci-après.

Elle a également pour objet des compositions pharmaceutiques contenant au moins un tel composé.

Elle a encore pour objet l'utilisation de composés de formule (I) telle que définie en revendication 39 en tant que médicaments agissant comme inhibiteur de l'activité de la-LTA₄ hydrolase, notamment comme anti-inflammatoires, anti-arthrit anti-psoriasique, hépato-protecteur; anti-mitotique ou traitement d'une surproduction de LTB₄ induite notamment par un inhibiteur de cyclooxygénase.

Les inventeurs ont mis en évidence que les composés de formule (I), ou leurs sels obtenus avec des acides minéraux ou organiques thérapeutiquement acceptables ou leurs stéréoisomères, possédaient une activité inhibitrice de la LTA₄ hydrolase.

Les composés (I) selon l'invention présentent par ailleurs une bonne biodisponibilité.

La présente invention décrit une série de composés capables d'inhiber puissamment la LTA₄ hydrolase.

Ces composés présentent en outre une activité biologique telle qu'indiquée ci-après qui leur confère un intérêt thérapeutique.

Les composés selon l'invention répondant à la formule (I) suivante : 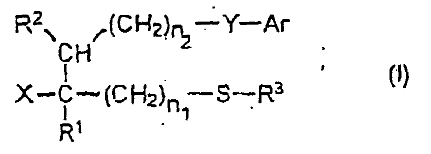
dans laquelle :
- X représente -NH₂
- R¹ et R² sont indépendamment choisis parmi les groupes suivants :
   i) un atome d'hydrogène
   ii) un groupe alkyle inférieur
- n₁ varie de 1 à 4
- n₂ varie de 0 à 10
- R³ est choisi parmi les groupes suivants :
   i) un atome d'hydrogène
   ii) 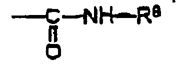
   iii) 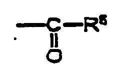
   iv) 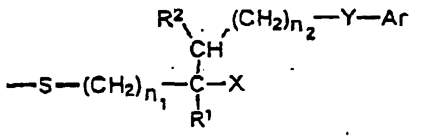
- Y est choisi parmi les groupes suivants :
   i) -O-
   ii) -CH₂-
   iii) -S-
   iv) -OCH₂-
   v) -SCH₂-
- Ar est choisi parmi les groupes suivants :
   i) un groupe phényle, non substitué, ou mono ou polysubstitué avec des substituants choisis parmi les atomes d'halogène et les groupes CF₃, alkyle inférieur, alcoxy inférieur, NH₂, NO₂, CN, OH, CO₂H, OPh, OCH₂Ph, SMe, SEt, Ph, CH₂Ph et NHCOR⁷ où R⁷ est un groupe alkyle inférieur,
   ii) 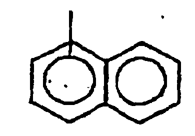
   iii) 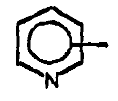
   iv) 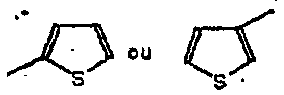
   v) 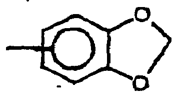
   vi) 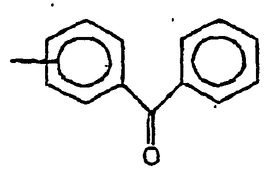
- R⁶ représente un groupe alkyle inférieur ou un groups phényle,

à l'exclusion des composés de formule I dans laquelle:
X est -NH₂, R¹ est R² sont chacun un atome d'hydrogène, n₁ = 1, n₂ = 0,
Y est un groupe -OCH₂- ou -SCH₂-, Ar est un groupe phényle et
R₃ est H ou les radicaux iv) correspondants.

Par groupe alkyle inférieur, on entend un groupe alkyle à chaîne linéraire ou ramifiée contenant de 1 à 6 atomes de carbone tels que méthyle, éthyle, propyle, butyle, pentyle, hexyle et leurs isomères ramifiés.

Par groupe alcoxy inférieur, on entend un groupe alcoxy contenant une chaîne linéaire ou ramifiée de 1 à 6 atomes de carbone tels que méthoxy, éthoxy, propoxy, butoxy, pentoxy, hexoxy et leurs isomères ramifiés.

Les atomes d'halogène sont de préférence choisis parmi le chlore et le fluor.

Lorsque l'on dessine une liaison à travers une liaison de cycle, cela indique que la liaison peut être reliée à tout atome disponible de ce cycle.

L'invention comprend également les stéréoisomères des composés de formule (I) incluant les formes diastéréoisomères et énantiomères.

L'invention s'étend aussi aux sels thérapeutiquement acceptables de ces composés ainsi qu'aux sels de leurs stéréoisomères incluant les formes diastéréoisomères et énantiomères.

Par sels thérapeutiquement acceptables, on entend un sel n'altérant ni la structure chimique, ni les propriétés pharmacologiques des composés de la présente invention. De tels sels incluent les anions minéraux et organiques tels que le chlorhydrate, bromhydrate, acétate, trifluoroacétate, maléate, fumarate; oxalate, bien connus dans la technique. Ces sels sont préparés de manière conventionnelle par neutralisation des composés de formule (I) avec l'acide désiré.

Parmi les composés de formule (I) précitée, ceux pour lesquels X représente NH₂ et/ou R³ représente un atome d'hydrogène sont préférés.

Dans ce groupe, les composés de formule (I) dans laquelle X est NH₂ et R³ est un atome d'hydrogène, sont plus particulièrement préférés.

Les composés, de formule (I) pour lesquels R¹ représente un atome d'hydrogène constituent également un sous-groupe particulièrement préféré selon l'invention.

Les composés de formule (I) avec R¹ différent de l'hydrogène représentent un autre sous-groupe selon l'invention.

Une sous-famille parmi les composés précités est formée par les composés pour lesquels n₁ est égal à 1.

Une autre sous-famille est constituée par les composés pour lesquels n₁ est différent de 1.

Conformément à l'invention, R² représente de préférence un atome d'hydrogène.

Un autre sous-groupe de composés selon l'invention, est formé par les composés où R² est différent d'un atome d'hydrogène Dans ce cas, R² représente de préférence un groupe méthyle.

Une sous-classe de composés selon l'invention est encore constituée par ceux pour lesquels n₂ est égal à zéro. Parmi ces composés, Y représente de préférence -O-, -S-, -OCH₂- ou -SCH₂-.

Une autre sous-classe est formée par les composés pour lesquels n₂ varie de 1 à 4, de préférence de 2 à 4 et de manière plus particulièrement préférée, par les composés où n₂ est égal à 3.

Une autre classe de composés selon l'invention est définie par ceux où n₂ est supérieur à 4.

Du point de vue du symbole Y, les composés pour lesquels celui-ci représente un atome d'oxygène sont particulièrement préférés selon l'invention.

D'autres sous-familles peuvent être définies selon que Y représente -CH₂-, un atome de soufre, un groupe -OCH₂- ou -SCH₂-.

Ar est choisi de préférence parmi un groupe phényle non substitué ou substitué, plus préférentiellement mono-substitué, par un des substituants précités.

Lorsque Ar symbolise un groupe phényle substitué, le ou les substituants sont de préférence choisis parmi les atomes d'halogène, les groupes CF₃, alkyle inférieur, O(alkyle inférieur), NO₂, CN, CO₂H, OPh, OCH₂Ph, Ph et CH₂Ph, les atomes d'halogène ainsi que les groupes alkyle inférieur et O(alkyle inférieur) étant plus particulièrement préférés.

Les composés pour lesquels Ar est un groupe phényle mono ou polysubstitué par -OPh, -OCH₂Ph, -Ph ou -CH₂Ph constituent une autre sous-famille selon l'invention.

D'autres sous-classes sont encore définies selon que Ar représente un groupe naphtyle, pyridinyle ou benzodioxazole.

Pour toutes les sous-familles mentionnées ci-dessus, les substituants non précisés peuvent varier selon leurs définitions générales respectives.

Un groupe particulièrement préféré de composés selon l'invention est constituté des composés répondant à la formule (II) suivante : 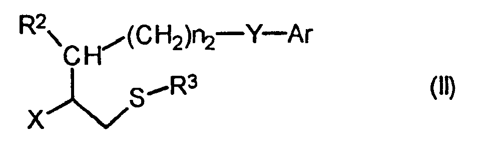
dans laquelle X, R², R³, Y, Ar et n₂ ont la signification précédente.

Les préférences indiquées précédemment pour les composés de formule (I) s'appliquent également à ceux de formule (II).

Un groupe encore plus particulièrement préféré comprend les composés répondant à la formule (III) suivante : 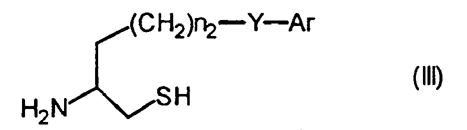
dans laquelle Y, Ar et n₂ ont la signification précédemment indiquée.

Les choix particuliers mentionnés pour les composés de formule (I) du point de vue des symboles Y et Ar, s'appliquent également aux composés de formule (III).

Parmi les composés de formule (II) ou (III), ceux pour lesquels n₂ est égal à 3, Y représente un atome d'oxygène et Ar représente un groupe phényle non substitué ou substitué comme indiqué précédemment, le cas échéant R² représentant un groupe méthyle et R³ représentant le motif iv) précité, sont particulièrement préférés selon l'invention.

Des exemples de composés de la présente invention, sont les :
- chlorhydrate du (S) 2-amino 3-phénoxy 1-propanethiol
- chlorhydrate du (S) 2-amino 3-benzyloxy 1-propanethiol
- chlorhydrate du (R) 2-amino 3-benzyloxy 1-propanethiol
- chlorhydrate du (2S,3R) 2-amino 3-méthyl 3-benzyloxy 1-propanethiol
- chlorhydrate du (2R,3S) 2-amino 3-méthyl 3-benzyloxy 1-propanethiol
- chlorhydrate du (S) 2-amino 3-benzylthio 1-propanethiol
- chlorhydrate du (R,S) 2-amino 7-phényl 1-heptanethiol
- chlorhydrate du (R,S) 2-amino 2-méthyl 6-phénoxy 1-hexanethiol
- chlorhydrate du (S) 2-amino 3-(4-benzylphenoxy)-1-propanethiol
- chlorhydrate du (R,S) 2-amino 4-phényl 1-butanethiol
- chlorhydrate du (R,S) 2-amino 6-phénoxy 1-hexanethiol
- chlorhydrate du (R,S) 2-amino 7-phénoxy 1-heptanethiol
- chlorhydrate du (R,S) 2-amino 7-(4-méthoxy phénoxy) 1-heptanethiol
- chlorhydrate du (R,S) 2-amino 6-(4-méthoxy phénoxy) 1-hexanethiol
- chlorhydrate du (R,S) 2-amino 6-(4-méthyl phénoxy)-1-hexanethiol
- chlorhydrate du (R,S) 2-amino 6-(3-méthyl phénoxy) 1-hexanethiol
- chlorhydrate du (R,S) 2-amino 6-(3-méthoxy phénoxy) 1-hexanethiol
- chlorhydrate du (R,S) 2-amino 6-(4-chloro phénoxy)-1-hexanethiol
- chlorhydrate du (R,S) 2-amino 6-(4-bromo phénoxy)-1-hexanethiol
- chlorhydrate du (R,S) 2-amino 6-(4-fluoro phénoxy)-1-hexanethiol
- chlorhydrate du (R,S) 2-amino 6-(2-méthoxy phénoxy) 1-hexanethiol
- chlorhydrate du (R,S) 2-amino 6-phénylthio 1-hexanethiol
- chlorhydrate du (R,S) 2-amino 6-(3,4-dioxyméthylène phénoxy)-1-hexanethiol
- chlorhydrate du (R,S) 2-amino 6-(4-éthoxy phénoxy)-1-hexanethiol
- chlorhydrate du (R,S) 2-amino 6-(4-éthyl phénoxy)-1-hexanethiol
- chlorhydrate du (R,S) 2-amino 5-phényl 1-pentanethiol
- chlorhydrate du (R,S) 2-amino 4-phénoxy 1-butanethiol
- chlorhydrate du (R,S) 2-amino 6-phényl 1-hexanethiol
- chlorhydrate du (R,S) 2-amino 4-phénylthio 1-butanethiol
- chlorhydrate du (R,S) 2-amino 6-(2,6-diméthyl phénoxy) 1-hexanethiol
- chlorhydrate du (R,S) 2-amino 4-benzylthio 1-butanethiol
- chlorhydrate du (R,S) 2-amino 5-phénoxy 1-pentanethiol
- chlorhydrate du (R,S) 2-amino 6-(2-méthylphénoxy) 1-hexanethiol
- chlorhydrate du (R,S) 2-amino 6-(4-phénoxyphénoxy) 1-hexanethiol
- chlorhydrate du (R,S) 2-amino 6-(4-carboxyphénoxy) 1-hexanethiol
- chlorhydrate du (R,S) 2-amino 6-(4-cyano phénoxy) 1-hexanethiol
- chlorhydrate du [2(R,S)-3(R,S)] 2-amino 3-méthyl 6-phénoxy 1-hexanethiol
- chlorhydrate du (R,S) 2-amino 6-(4-phényl phénoxy) 1-hexanethiol
- chlorhydrate du (R,S) 2-amino 6-(4-benzyloxy phénoxy) 1-hexanethiol
- chlorhydrate du (R,S) 2-amino 6-(2-naphtyloxy) 1-hexanethiol
- chlorhydrate du (R,S) 2-amino 6-(1-naphtyloxy) 1-hexanethiol
- chlorhydrate du (R,S) 2-amino 8-phénoxy 1-octanethiol
- chlorhydrate du (R,S) 2-amino 9-phénoxy 1-nonanethiol
- chlorhydrate du (R,S) 2-amino 6-(3-trifluoro méthyl phénoxy) 1-hexanethiol
- chlorhydrate du (R,S) 2-amino 6-(3-fluoro phénoxy) 1-hexanethiol
- chlorhydrate du (R,S) 2-amino 6-(2,4-difluoro phénoxy) 1-hexanethiol
- chlorhydrate du (R,S) 2-amino 6-(2-fluoro phénoxy) 1-hexanethiol
- chlorhydrate du (R,S) 2-amino 6-pentafluoro phénoxy 1-hexanethiol
- chlorhydrate du (R,S) 2-amino 6-(4-nitro phénoxy) 1-hexanethiol
- chlorhydrate du (R,S) 2-amino 8-phényl 1-octanethiol
- chlorhydrate du (R,S) 2-amino 6-(3,5-diméthoxy phénoxy) 1-hexanethiol
- chlorhydrate du (R,S) 2-amino 6-(4-butoxy phénoxy) 1-hexanethiol
- chlorhydrate du (R,S) 2-amino 6-(4,5-dichloro phénoxy)1-hexanethiol
- chlorhydrate du (R,S) 2-amino 6-(2-pyridoxy) 1-hexanethiol
- chlorhydrate du (R,S) 2-amino 6-(3-cyano phénoxy) 1-hexanethiol
- chlorhydrate du (R,S) 2-amino 5-(4-benzyl phénoxy) 1-pentanethiol
- chlorhydrate du (R,S) 2-amino 6-(3-chloro phénoxy) 1-hexanethiol
- chlorhydrate du (R,S) 2-amino 7-(4-cyano phénoxy) 1-heptanethiol
- chlorhydrate du (R,S) 2-amino 5-(4-cyano phénoxy) 1-pentanethiol
- chlorhydrate du (R,S) 2-amino 6-(3-éthyl phénoxy) 1-hexanethiol
- chlorhydrate du (R,S) 2-amino 6-(4-trifluorométhyl phénoxy) 1-hexanethiol
- chlorhydrate du (R,S) 2-amino 7-(4-benzylphénoxy) 1-heptanethiol
- chlorhydrate du (R,S) 2-amino 6-(4-benzylphénoxy) 1-hexanethiol
- chlorhydrate du (R,S) 2-amino 5-(3-éthyl phénoxy) 1-pentanethiol
- chlorhydrate du (R,S) 2-amino 5-(4-méthyl phénoxy) 1-pentanethiol
- chlorhydrate du (R,S) 2-amino 6-(4-acétamido phénoxy) 1-hexanethiol
- chlorhydrate du (R,S) 2-amino 6-(4-iodo phénoxy) 1-hexanethiol
- chlorhydrate du (R,S) 2-amino 6-(4-propoxy phénoxy) 1-hexanethiol
- chlorhydrate du (R,S) 2-amino 5-(4-benzoyl phénoxy) 1-pentanethiol
- chlorhydrate du (R,S) 2-amino 5-(4-éthoxy phénoxy) 1-pentanethiol
- chlorhydrate du (R,S) 2-amino 7-(4-éthoxy phénoxy) 1-heptanethiol
- chlorhydrate du (+) 2-amino 6-(4-éthoxy phénoxy) 1-hexanethiol
- chlorhydrate du (-) 2-amino 6-(4-éthoxy phénoxy) 1-hexanethiol
- chlorhydrate du (+) 2-amino 6-phénoxy 1-hexanethiol
- chlorhydrate du (-) 2-amino 6-phénoxy 1-hexanethiol.

Les composés de formule (I) ou (II) telles que précédemment définies avec X = NH₂ et/ou
R³ différent d'un atome d'hydrogène c'est-à-dire choisi parmi les groupes
ii) 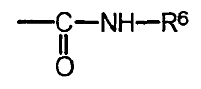
iii) 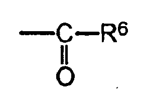
iv)

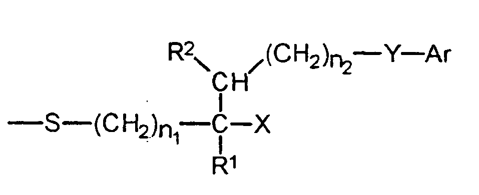

sont préférés en particulier avec R³ représentant le groupe iv) ci-dessus, sont préférées.

Des exemples selon l'invention sont les:
- dichlorhydrate du 1,1-dithio bis (2-(R,S)-amino 6-phénoxy hexane)
- bromhydrate du (R,S) 2-amino 6-phénoxy 1-S-acétylthio hexane
- dichlorhydrate du 1,1-dithiobis (2-(+)-amino-6-phénoxy-hexane) (isomère A)
- dichlorhydrate du 1,1-dithiobis (2-(-)-amino-6-phénoxy-hexane) (isomère B)
- dichlorhydrate du 1,1-dithiobis (2-(R,S)-amino 6-(4-éthoxyphénoxy)-1 hexane)
- dichlorhydrate du 1,1-dithiobis (2-(+)-amino-6-(4-éthoxyphenoxy)-1 hexane) (isomère-A)
- dichlorhydrate du 1,1-dithiobis (2-(-)-amino-6-(4-éthoxyphénoxy)-1 hexane) (isomère B)
- dichlorhydrate du 1,1-dithiobis (2-(R,S)-amino-6-(4-acétamidophénoxyl)-1 hexane
- dichlorhydrate du 1,1-dithiobis (2-(R,S)-amino-6-(4-cyanophénoxy)-1 hexane.

Les composés de la présente invention sont préparés à partir de matières premières facilement disponibles, selon l'un des procédés indiqués ci-après:

Les schémas réactionnels donnés ci-après décrivent des procédés qui peuvent être employés pour là préparation des composés de formule (I), en indiquant les produits de départ, les intermédiaires ainsi que les conditions de synthèse.

Les abréviations utilisées dans la présente description correspondent aux définitions ci-dessous :
- Ac: : acétyle
- Bn: : benzyle
- DCC: : dicyclohexylcarbodiimide
- DEAD: : azodicarboxylate de diéthyle
- DIAD: : azodicarboxylate de diisopropyle
- DMF: : diméthylformamide
- DPPA: : diphénylphosphorylazide
- Et: : éthyle
- EtOH: : alcool éthylique
- Et₂O: : éther éthylique
- HOBT: : hydroxybenzotriazole
- LAH: : aluminohydrure de lithium
- Me: : méthyle
- Ms: : méthanesulfonyle
- n.Bu: : n-butyle
- NBu₄F: : fluorure de tétrabutylammonium
- NEt₃: : triéthylamine
- n.Pr: : n-propyle
- Pd/C: : palladium sur carbone
- Ph: : phényle
- tBu: : tertiobutyle
- TFA: : acide trifluoroacétique
- THF: : tétrahydrofuranne
- Ts: : paratoluènesulfonyle

Les schémas 1 à 3 décrivent la préparation d'amino-alcools substitués. 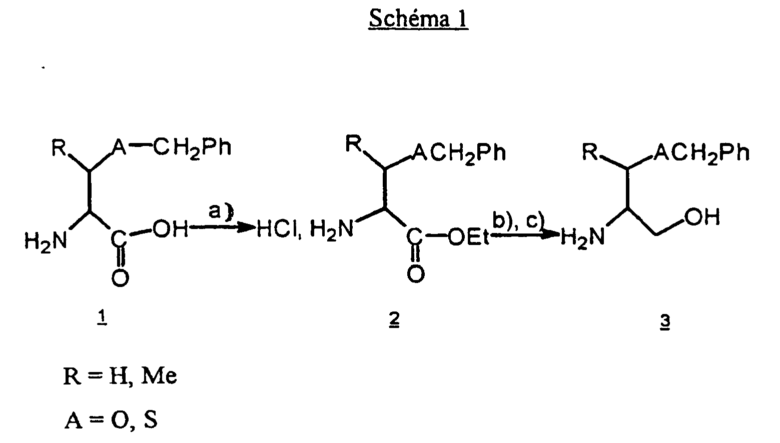
a) SOCl₂, EtOH, reflux
b) NEt₃, Et₂O, CHCl₃
c) LAH, Et₂O

Le schéma 1 décrit une méthode de préparation des composés de formule 3 à partir d'aminoacides commerciaux.

Les composés 2 sont facilement obtenus par estérification, en présence de chlorure de thionyle et d'EtOH, de l'aminoacide 1. L'aminoalcool 3 est obtenu en 2 étapes après libération de l'amine par NEt₃ puis réduction par LAH dans Et₂O à température ambiante. 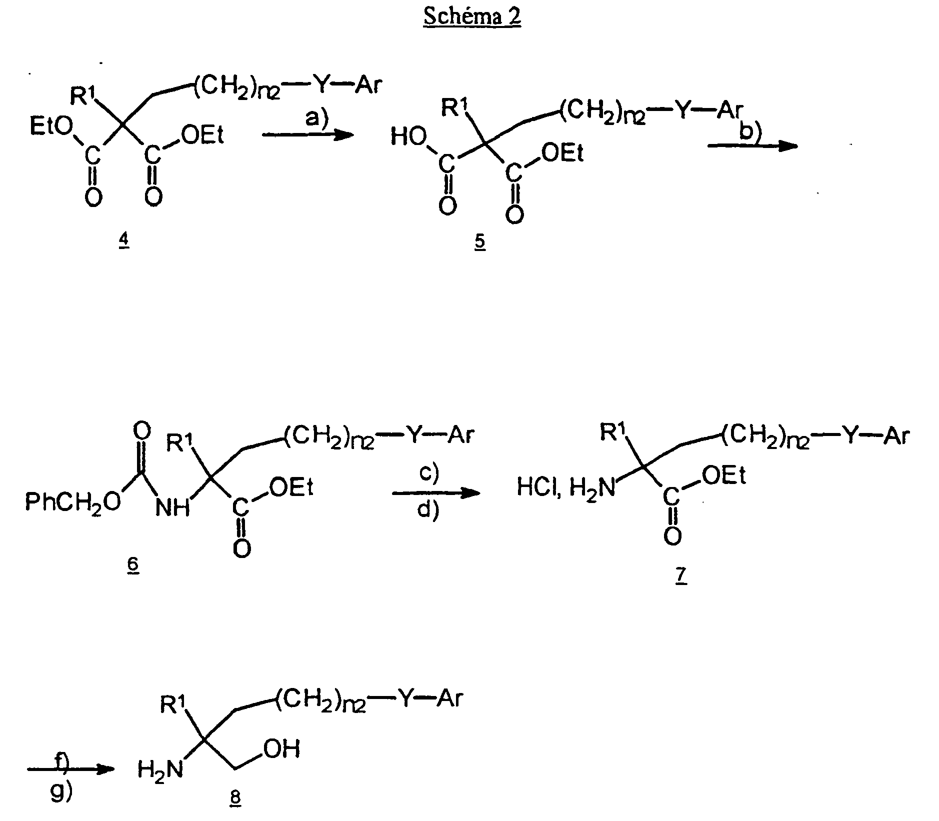
R¹, n2, Y et Ar sont tels que définis dans la formule (I).
a) KOH, EtOH, 0°C
b) DPPA, NEt₃, toluène, alcool benzylique, 80°C
c) H₂, Pd/C, EtOH
d) HCl 3N
f) NEt₃, Et₂O, CHCl₃
g) LAH, Et₂O

Les malonates de formule 4 sont obtenus par alkylation d'un malonate avec les dérivés bromés ou chlorés correspondant en présence d'éthylate de sodium dans l'éthanol au reflux. Une monosaponification à l'aide d'une solution de KOH dans l'EtOH conduit aux composés 5 à qui l'on fait subir une réaction de Curtius en présence de DPPA, de NEt₃ et d'alcool benzylique dans le toluène à 80°C pendant une nuit.

La fonction benzyloxycarbonyle est déprotégée par hydrogénation catalytique dans l'éthanol à l'aide de Pd/C pour conduire aux aminoesters 7. Les aminoalcools 8 sont obtenus comme décrit dans le schéma 1. 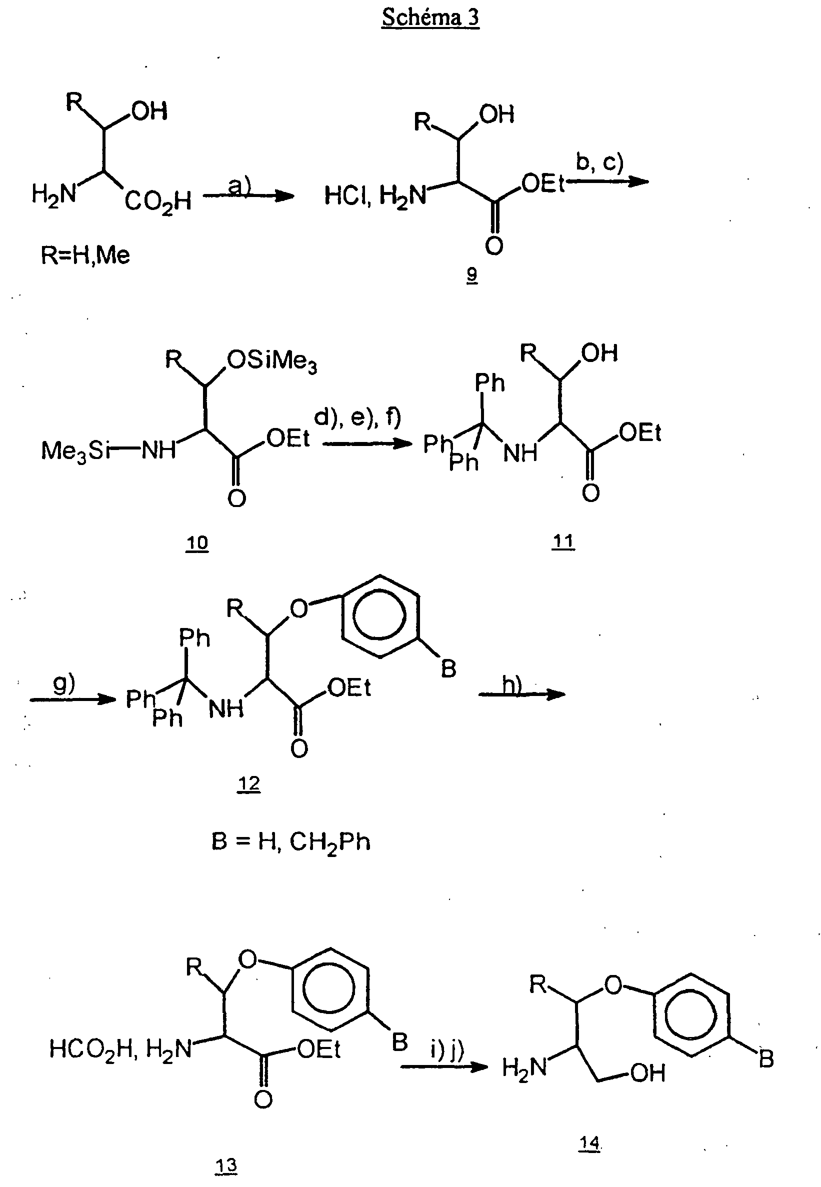
a) SOCl₂, EtOH, reflux
b) NEt₃, Et₂O, CHCl₃
c) Me₃SiCl, NEt₃
d) MeOH
e) Et₃N/(Ph)₃CCl
f) NBu₄F 1M/THF
g) PPh₃, DEAD ou DIAD, 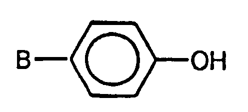
h) HCO₂H
i) NaHCO₃ aq.
j) LAH, Et₂O

La sérine ou la thréonine est estérifiée en présence de chlorure de thionyle et d'EtOH. Le chlorhydrate d'aminoester 9 obtenu est traité par la triéthylamine puis par le chlorure de triméthylsilyle en présence de NEt₃ pour conduire au composé 10. La fonction amino est déprotégée à l'aide de MeOH anhydre puis reprotégée par réaction avec le chlorure de trityle. La fonction hydroxy est ensuite libérée à l'aide du fluorure de tétrabutylammonium pour conduire au composé 11. La fonction hydroxy du composé 11 est substituée selon une réaction de type Mitsunobu par un dérivé phénolique de formule 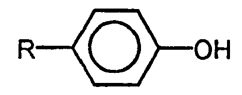
pour conduire au composé 12. Les composés 14 sont obtenus par déprotection par l'acide formique suivie d'une réduction à l'aide de l'aluminohydrure de lithium.

Le schéma 4 décrit une méthode de préparation des composés 17 répondant à la formule (I). 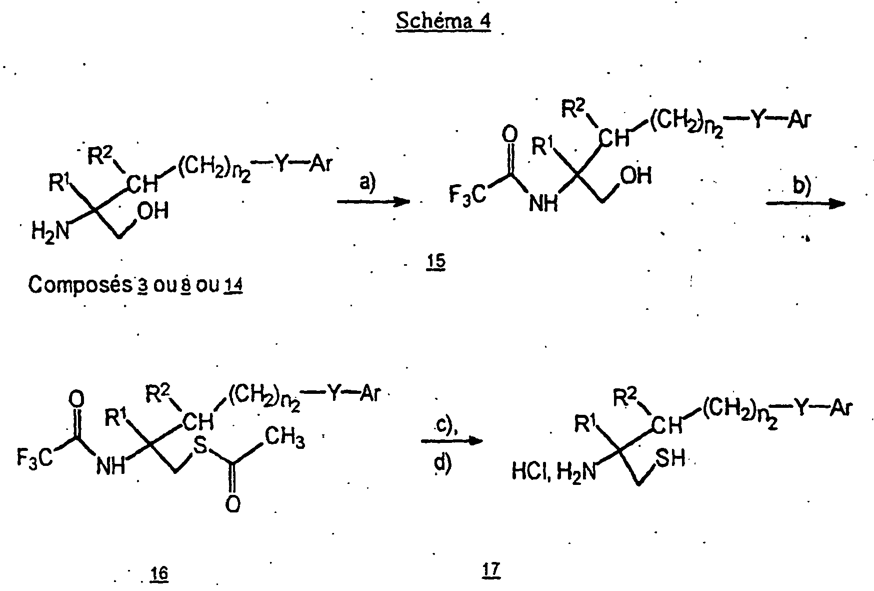
a) trifluoroacétylimidazole, pyridine, 0°C
b) PPh₃, DEAD ou DIAD, CH₃COSH, THF
c) NaOH, EtOH, H₂O, 50°C
d) HCl 3N

La fonction amino des composés 3, 8 ou 14 est protégée à l'aide du trifluoroacétylimidazole dans la pyridine à 0°C. On effectue ensuite sur les composés 15 une réaction de type Mitsunobu en présence de PPh₃, DEAD (ou DIAD) et d'acide thioacétique pour conduire aux composés 16. Enfin, les composés 17 sont obtenus par déprotection à 50°C en milieu basique sous atmosphère inerte suivi d'une acidification par une solution aqueuse d'acide chlrorhydrique.

Pour la synthèse des composés 30 de formule (I), on peut utiliser un procédé via des dérivés mercaptoacide 28.

Ces dérivés mercaptoacide 28 sont préparés à partir de malonates 23 mono-substitués obtenus soit à partir des halogénures commerciaux, soit à partir des halogénures 18, soit à partir des alcools activés 22 (mésylate, tosylate).

Les schémas 5 et 6 décrivent la préparation des halogénures non commerciaux 18 et des alcools activés 22. 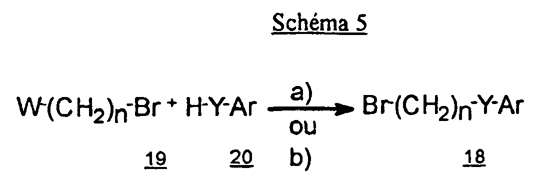
W = Cl, Br
n varie de 3 à 11
Y=O, S
Ar est tel que défini dans la formule (I)
a) NaOH 9N, THF reflux
b) K₂CO₃, DMF, température ambiante

Les composés 18 peuvent être obtenus selon deux voies : par traitement dans la soude 9N au reflux en présence de THF ou par l'utilisation de K₂CO₃ en poudre dans le DMF à température ambiante.

### Schéma 6

Pour la synthèse des alcools activés 22 de formule R²-CH(OZ)-(CH₂)n₂-Y-Ar (Z = Ms, Ts) dans laquelle R², n₂, Y et Ar ont les définitions précitées dans la formule (I), on utilise le procédé suivant : 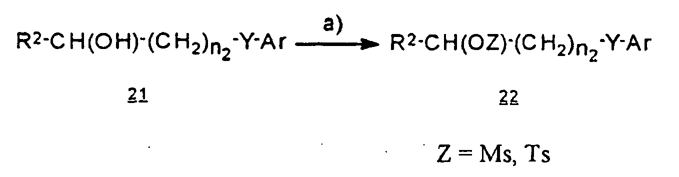
a) MsCl ou TsCl, CH₂Cl₂, température ambiante.

Le schéma 7 décrit la synthèse des malonates mono-substitués 23 obtenus par réaction d'un halogénure commercial, d'un halogénure 18 ou d'un alcool activé 22 sur le diéthyle malonate. 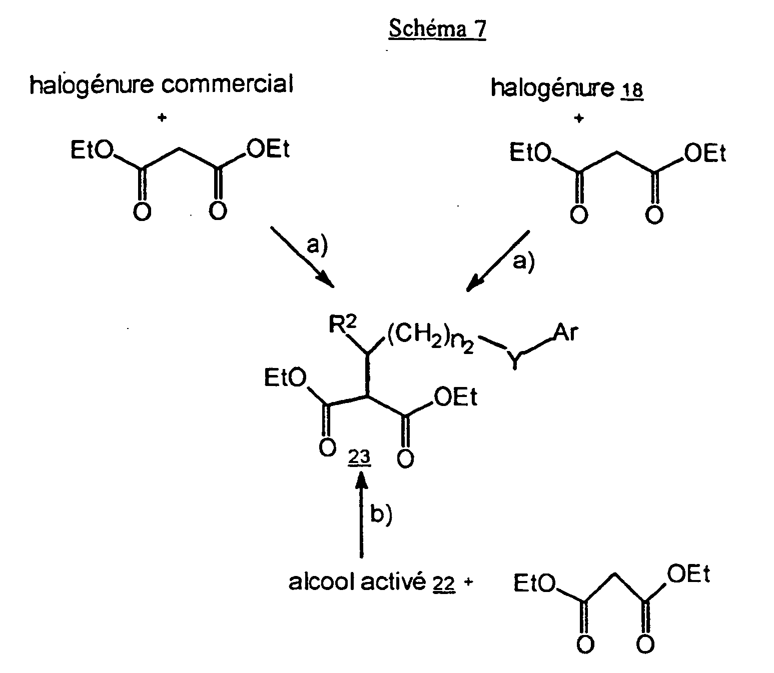
R², n₂, Y et Ar ont les définitions précitées dans la formule (I).
a) NaOEt 1,7 M dans EtOH, reflux
b) CsF, DMF, 60°C

Les malonates 23 sont obtenus soit à partir des halogénures commerciaux, soit à partir des halogénures 18 par réaction avec le diéthylmalonate en présence d'éthylate de sodium dans l'éthanol au reflux soit à partir des alcools activés 22 par réaction avec le diéthyl malonate en présence du fluorure de césium dans le DMF à 60°C.

Les malonates 23 sont transformés en dérivés mercaptoacides 28 selon le schéma 8. 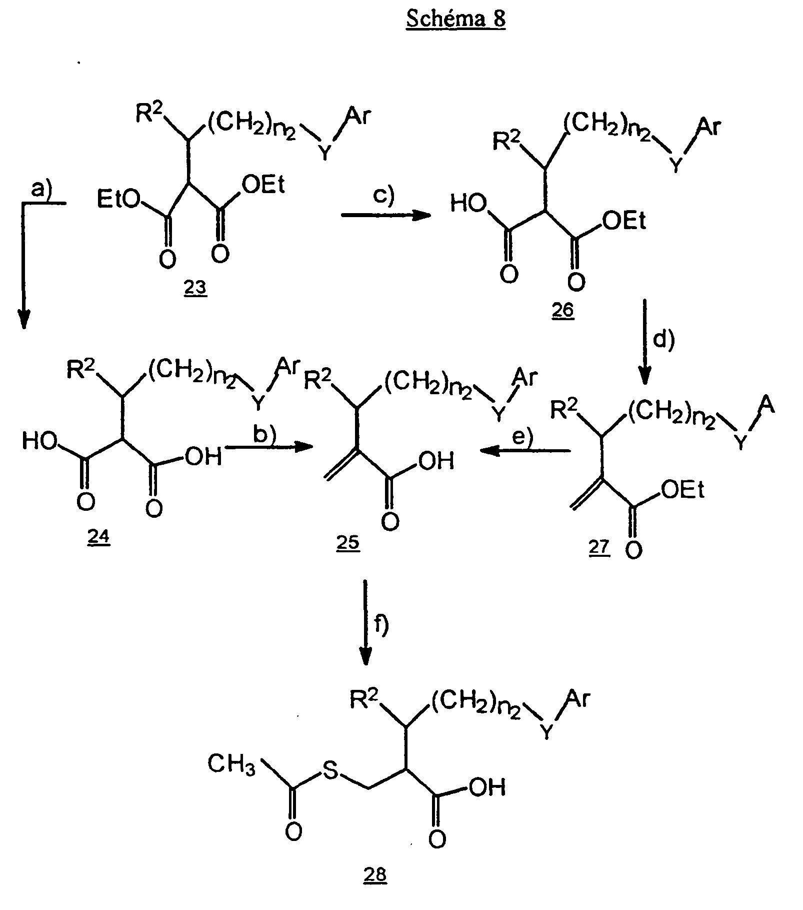
R², n₂, Y et Ar ont les définitions précitées dans la formule (I)
a) NaOH 6N, reflux
b) Paraformaldéhyde, HNEt₂, AcOEt
c) KOH, EtOH 0°C
d) formol 37 %, HNEt₂
e) NaOH 2N, acétone, H₂O
f) CH₃COSH, 70°C

Les acides acryliques 25 sont obtenus selon deux voies :
- une voie en deux étapes via les diacides 24 obtenus par saponification dans la soude 6N au reflux, puis une réaction de Mannich en présence de paraformaldéhyde, de diéthylamine dans l'acétate d'éthyle au reflux ;
- une voie en trois étapes via un monoacide 26 obtenu par monosaponification dans la potasse éthanolique à 0°C suivie d'une réaction de Mannich conduisant aux ester acryliques 27 puis d'une saponification par de la soude 2N dans un mélange acétone-eau.

Les dérivés 28 sont obtenus par addition de Michael de l'acide thioacétique sur les acides acryliques 25 à 70°C.

Les dérivés 28 sont transformés en dérivés mercaptoamines 30 selon le schéma 9. 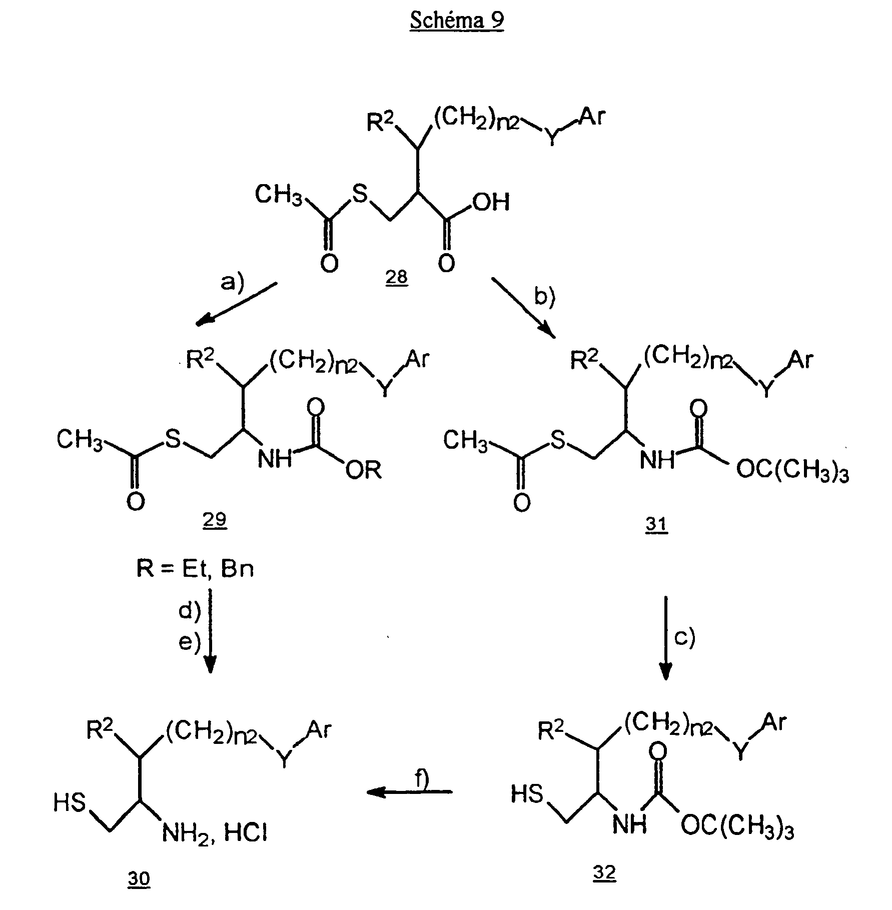
a) DPPA, NEt₃, toluène, ROH, 80°C
   R=Et,Bn
b) DPPA, NEt₃, tBuOH, reflux
c) NaOH 2N, MeOH, atmosphère inerte
d) NaOH 10N, EtOH, reflux, atmosphère inerte
e) HCl 3N
f) HCl gaz, MeOH, -10°C

Les aminothiols de formule 30 sont obtenus selon deux voies :
- une voie en deux étapes qui consiste à effectuer une réaction de Curtius sur les acides 28 à l'aide de DPPA, NEt₃ dans le toluène et d'un alcool tel que l'éthanol ou l'alcool benzylique à 80°C pendant une nuit. Les carbonates 29 sont ensuite déprotégés par une solution de soude 10N en présence d'éthanol, à reflux pendant 2 heures sous atmosphère inerte. Après acidification par HCl 3N, on obtient les aminothiols 30 ;
- une voie en trois étapes où la réaction de Curtius est effectuée dans le tBuOH au reflux pour conduire au carbamate terbutylique 31. La fonction thioester est ensuite saponifiée par une solution aqueuse de soude en présence de MeOH pour conduire aux dérivés 32. Le groupe terbutoxycarbonyle est ensuite déprotégé par une solution d'HCl gazeux dans le MeOH à -10°C. On obtient ainsi les aminothiols 30.

### Schéma 10

Le schéma 10 montre la préparation de disulfures 34 répondant à la formule (I) à partir des carbonates 31 décrits dans le schéma 9. 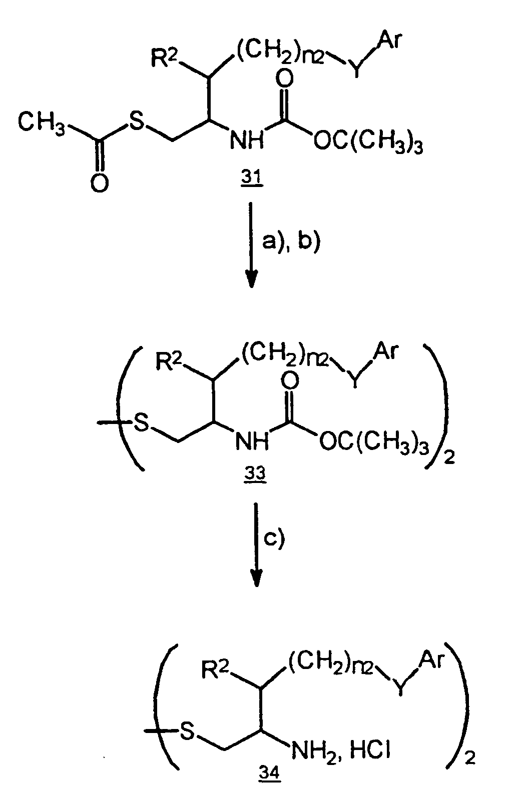
R², n₂, Y et Ar ont les définitions précitées dans la formule (I)
a) NaOH N, EtOH
b) I₂, EtOH
c) HCl gaz, MeOH, -10°C

Les carbamates 31 sont soumis à l'action de soude N dans l'éthanol puis à l'action d'une solution 0,3 M d'iode dans l'éthanol pour conduire aux disulfures N-protégés 33. Le groupe terbutoxycarbonyle est ensuite déprotégé par une solution d'HCl gazeux dans le MeOH à -10°C et on obtient ainsi les disulfures 34.

### Schéma 11

Le schéma 11 montre la préparation des composés S-acétylés 35 répondant à la formule (I) à partir des carbamates 29 décrits dans le schéma 9. 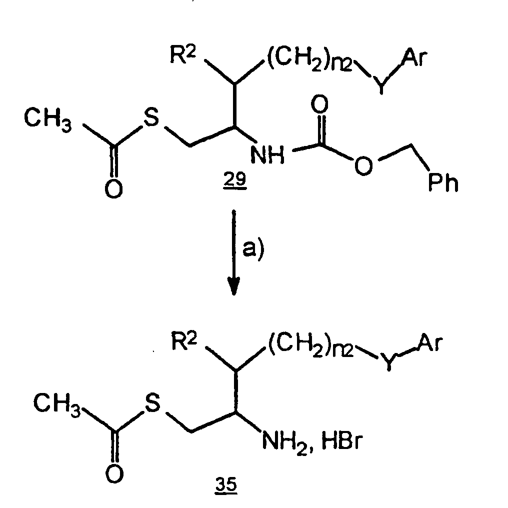
R², n₂, Y et Ar ont les définitions précitées dans la formule (I)
a) HBr 30 % / AcOH

Le groupe benzyloxycarbonyle est déprotégé par une solution d'HBr gazeux à 30 % dans l'acide acétique à température ambiante, pour conduire aux dérivés 35.

Les inventeurs ont montré que les composés (I) selon l'invention présentent des propriétés inhibitrices de la LTA₄ hydrolase.

Ils possèdent une intéressante activité thérapeutique en particulier dans le domaine des traitements anti-inflammatoires.

Ils possèdent également une intéressante activité anti-arthritique.

Les composés de l'invention présentent aussi des propriétés anti-psoriasiques.

Par ailleurs, les inventeurs ont montré que les composés de l'invention préviennent l'augmentation des taux tissulaires de LTB₄ induite par les inhibiteurs de cyclooxygénase.

Ils sont ainsi utiles pour prévenir certains effets secondaires pro-inflammatoires paradoxaux des inhibiteurs de cyclooxygénase.

Enfin, le LTB₄ étant le ligand endogène du récepteur induisant une prolifération des peroxisomes, les composés de l'invention trouvent aussi des applications dans les domaines de l'hépatoprotection et de l'action antimitotique.

La présente invention a ainsi également pour objet l'utilisation des composés de formule (I) en tant que médicaments agissant comme inhibiteurs de l'activité de la LTA₄ hydrolase, en particulier pour un traitement anti-inflammatoire, ou anti-arthritique.

Elle a aussi pour objet l'utilisation des composés de l'invention en tant que médicaments anti-psoriasiques.

Elle a encore pour objet leur utilisation en tant que médicaments hépato-protecteurs ou antimitotiques.

Elle a encore pour objet l'utilisation de tels composés en tant que médicaments destinés au traitement d'une surproduction de LTB₄, induite notamment par des inhibiteurs de cyclooxygénase.

Elle a encore pour objet l'utilisation de tels composés (I) pour la préparation de médicaments destinés à inhiber l'activité de la LTA₄ hydrolase.

Elle a en particulier pour objet leur utilisation pour la préparation de médicaments destinés aux traitements pré-cités.

Les composés de formule (I) peuvent être administrés dans un véhicule ou excipient physiologiquement acceptable.

Aussi, la présente invention a encore pour objet des compositions pharmaceutiques comprenant une quantité thérapeutiquement efficace d'au moins un composé de formule (I) en combinaison avec un véhicule ou excipient physiologiquement acceptable.

Les composés (I) de l'invention peuvent aussi être utilisés en combinaison avec des inhibiteurs de cyclooxygénase.

L'invention concerne ainsi des médicaments ou compositions pharmaceutiques contenant une quantité thérapeutiquement efficace d'un composé (I) et une quantité thérapeutiquement efficace d'un composé inhibiteur de cyclooxygénase, éventuellement en combinaison avec un véhicule ou excipient physiologiquement acceptable.

Des exemples d'inhibiteurs de cyclooxygénase utiles selon l'invention sont l'aspirine (acide acétylsalicylique), l'ibuprofène, le dichlofénac.

Les médicaments ou compositions pharmaceutiques selon l'invention peuvent avantageusement être administrés par voies locales cutanées ou occulaires, par voie parentérale ou par voie orale, cette dernière étant préférée.

L'invention a également pour objet un procédé de traitement pour inhiber l'activité de la LTA₄ hydrolase chez l'homme.

Elle a encore pour objet un tel procédé pour les traitements indiqués précédemment.

Elle a aussi pour objet un procédé de traitement d'une surproduction de LTB₄, notamment induite par des inhibiteurs de cyclooxygénase.

D'autres avantages et caractéristiques de la présente invention apparaîtront à la lecture des exemples de préparation de composés de formule (I) donnés à titre illustratif ainsi que des résultats biologiques donnés ci-après.

### EXEMPLES

Un tableau récapitulatif des exemples de composés de formule (I) est donné ci-dessous :

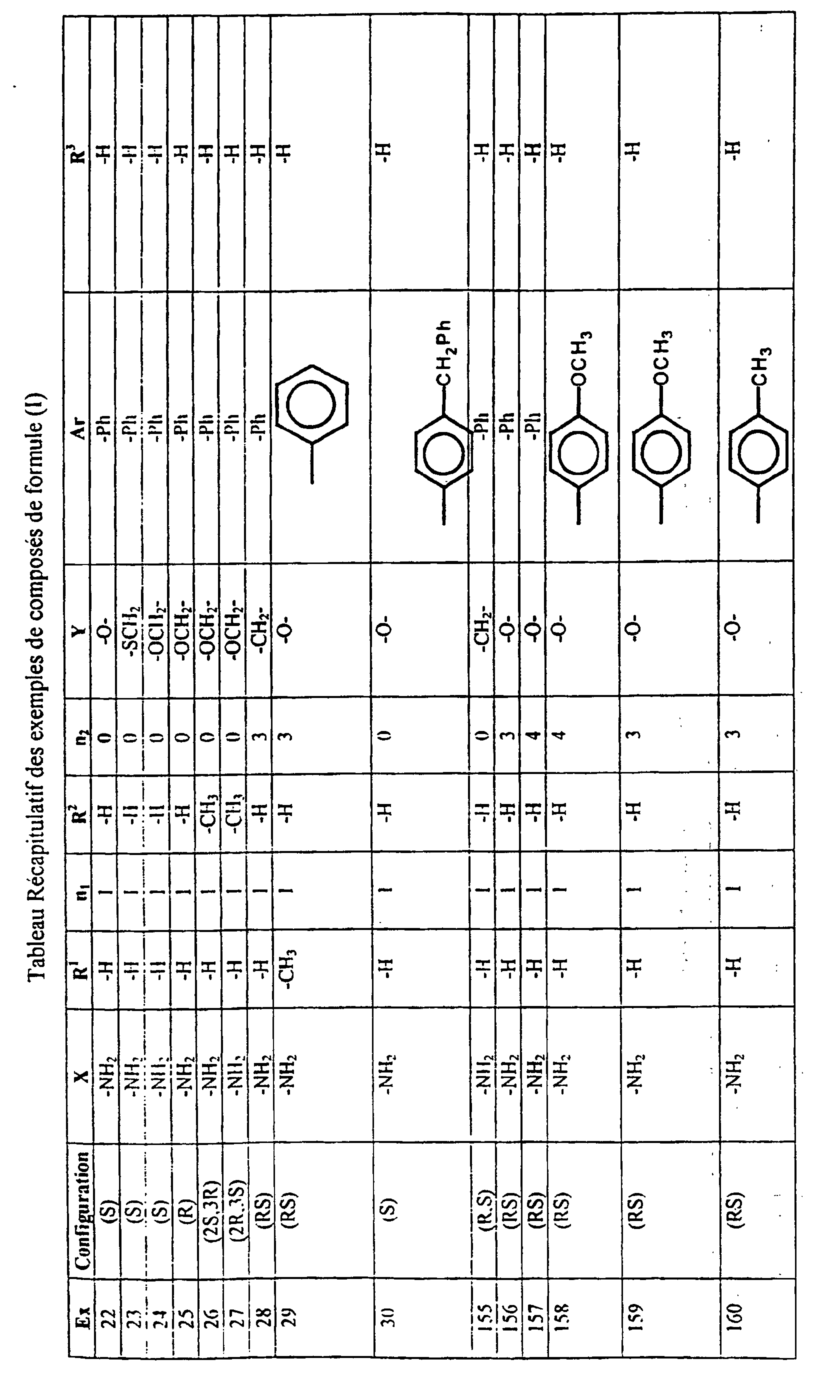

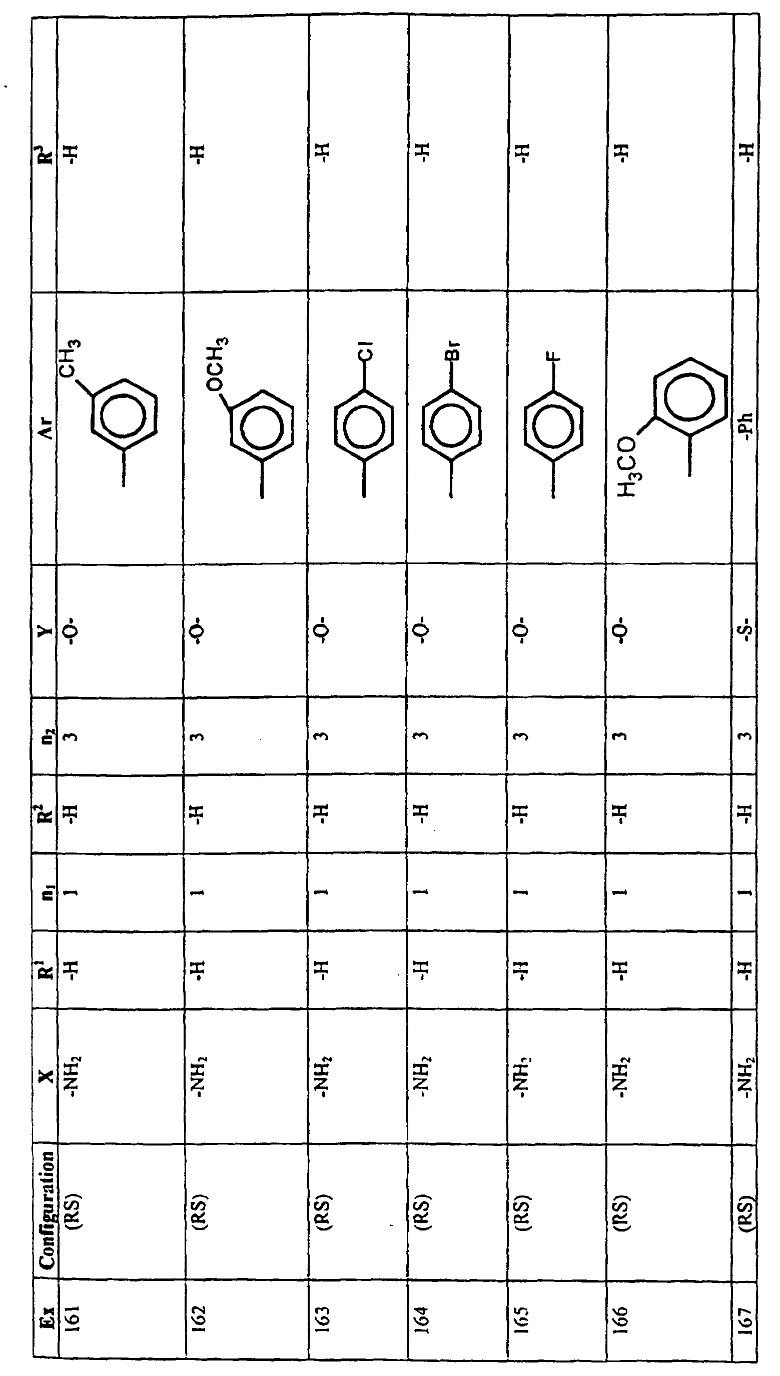

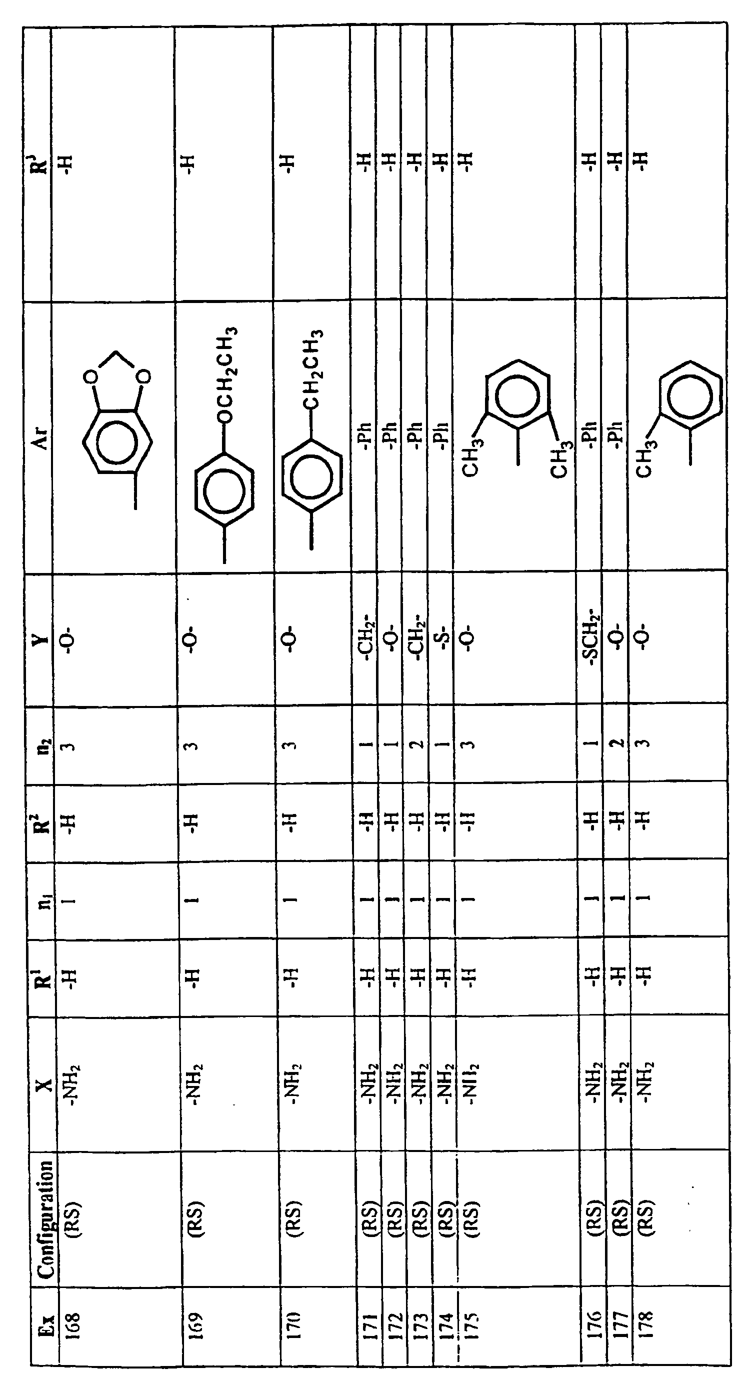

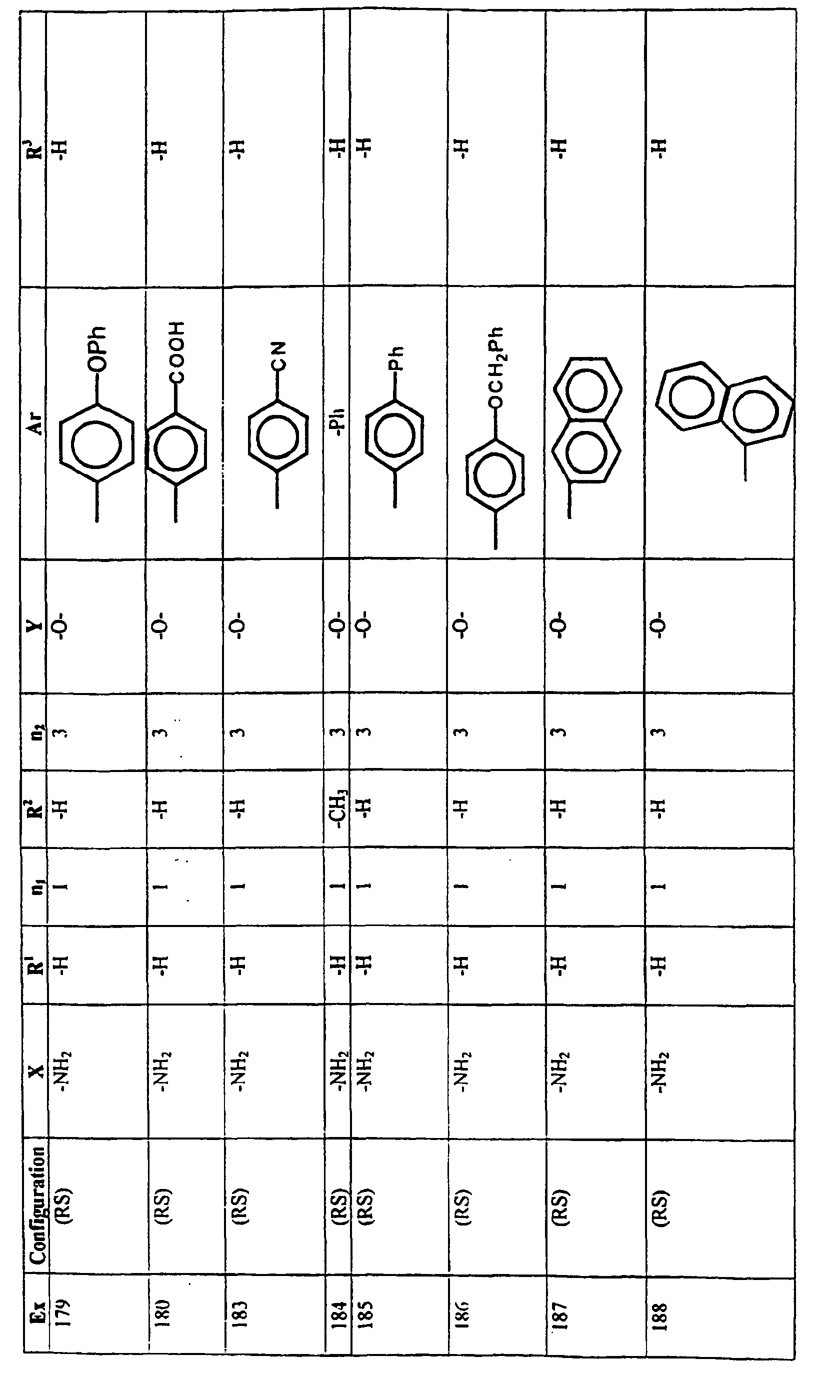

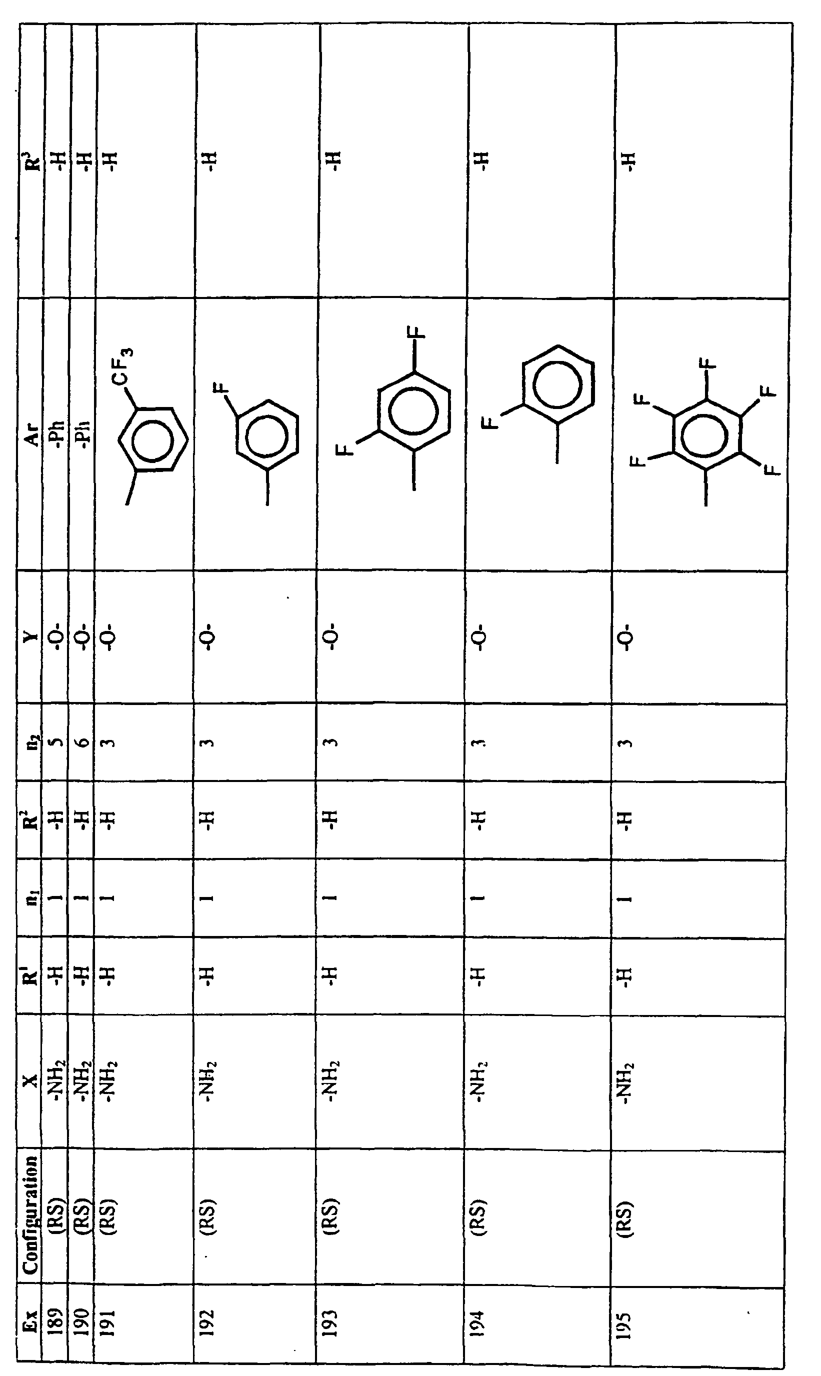

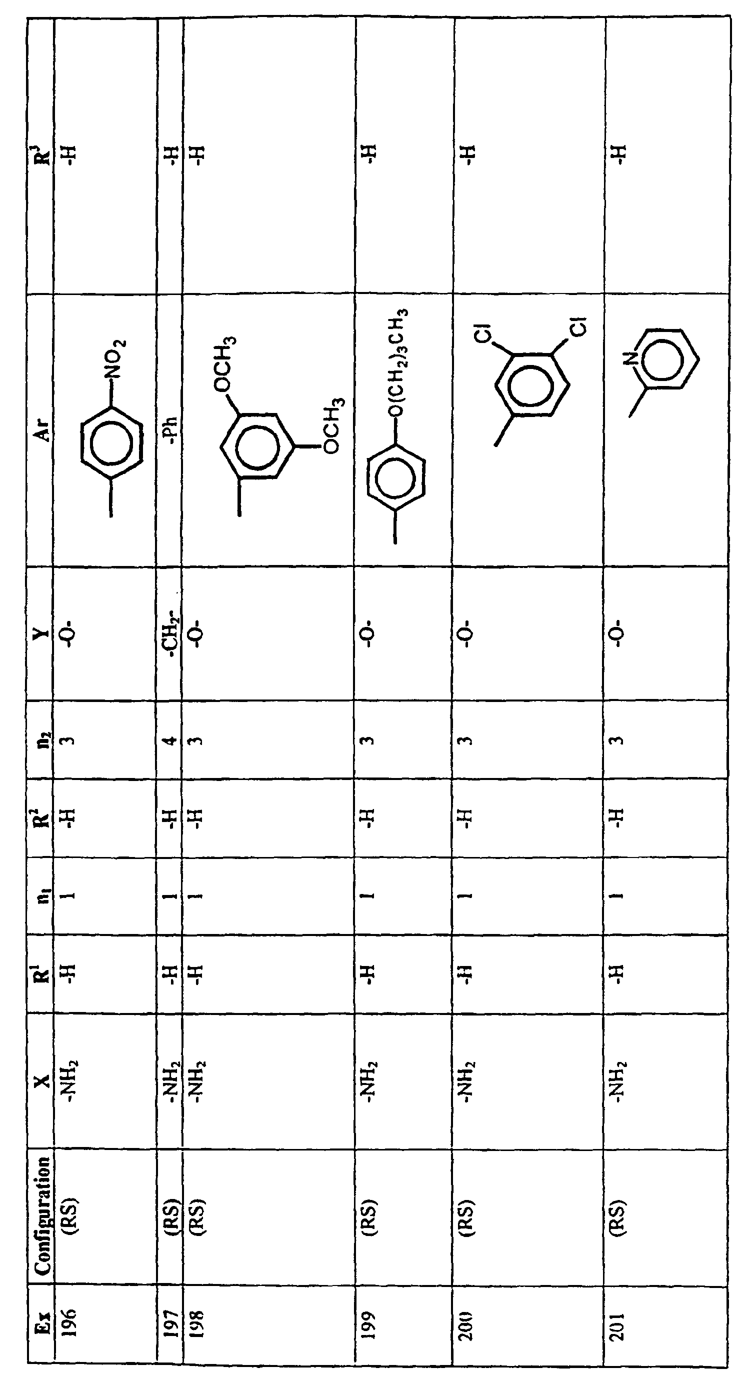

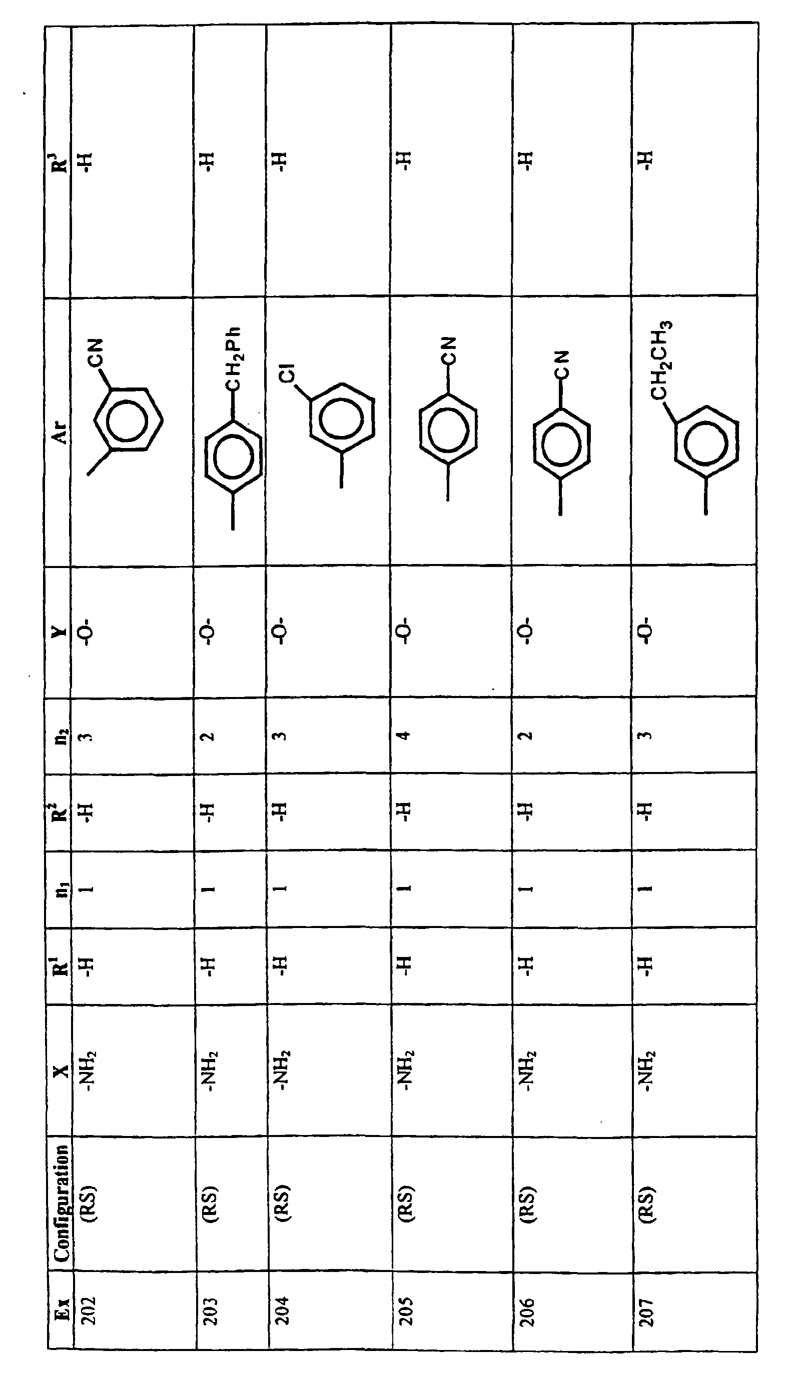

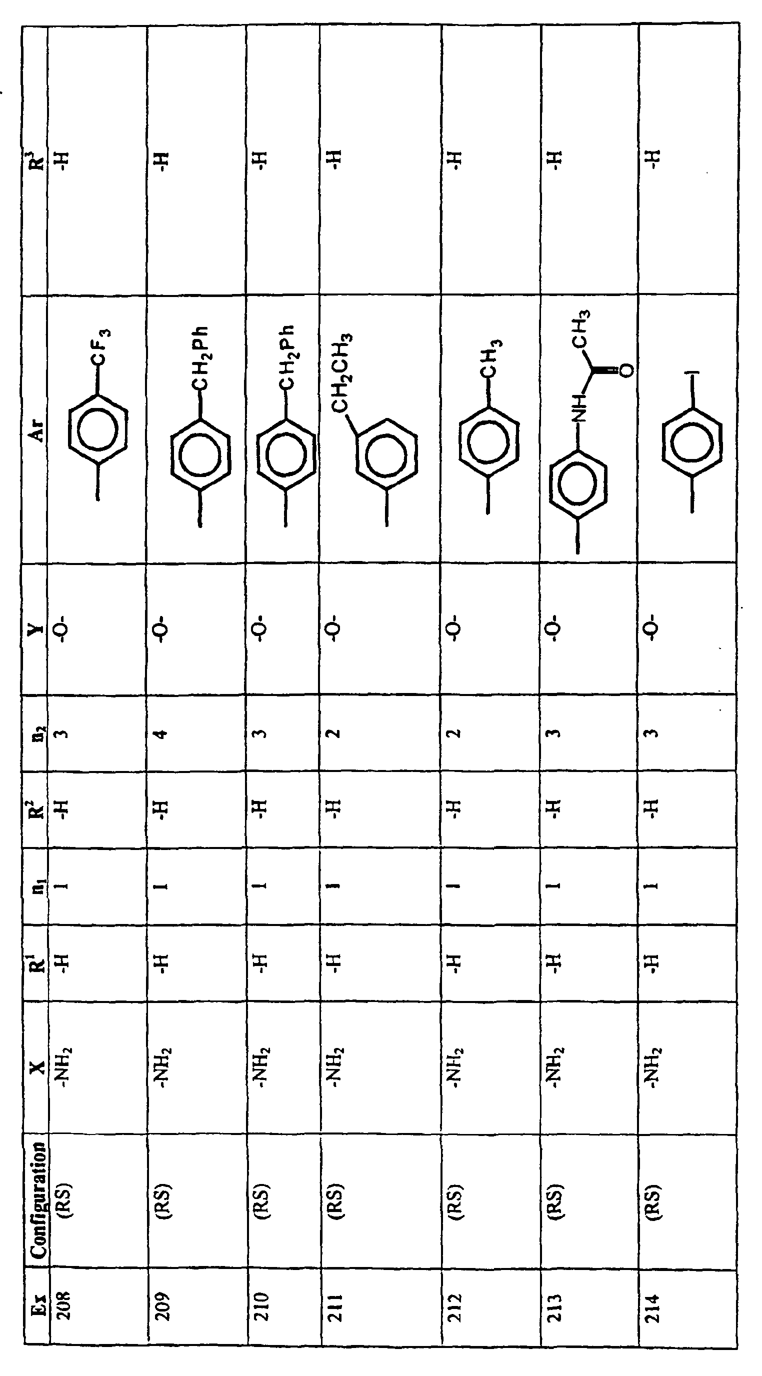

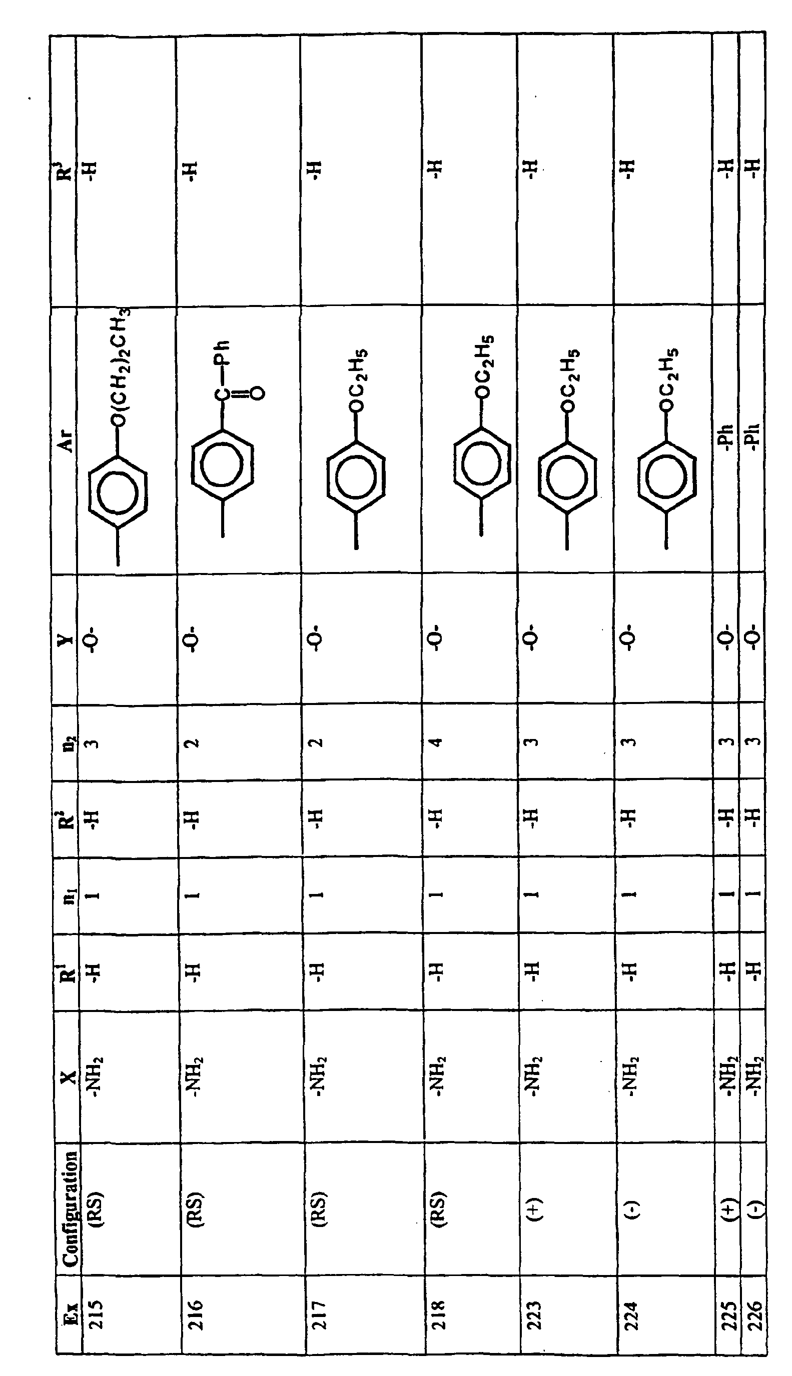

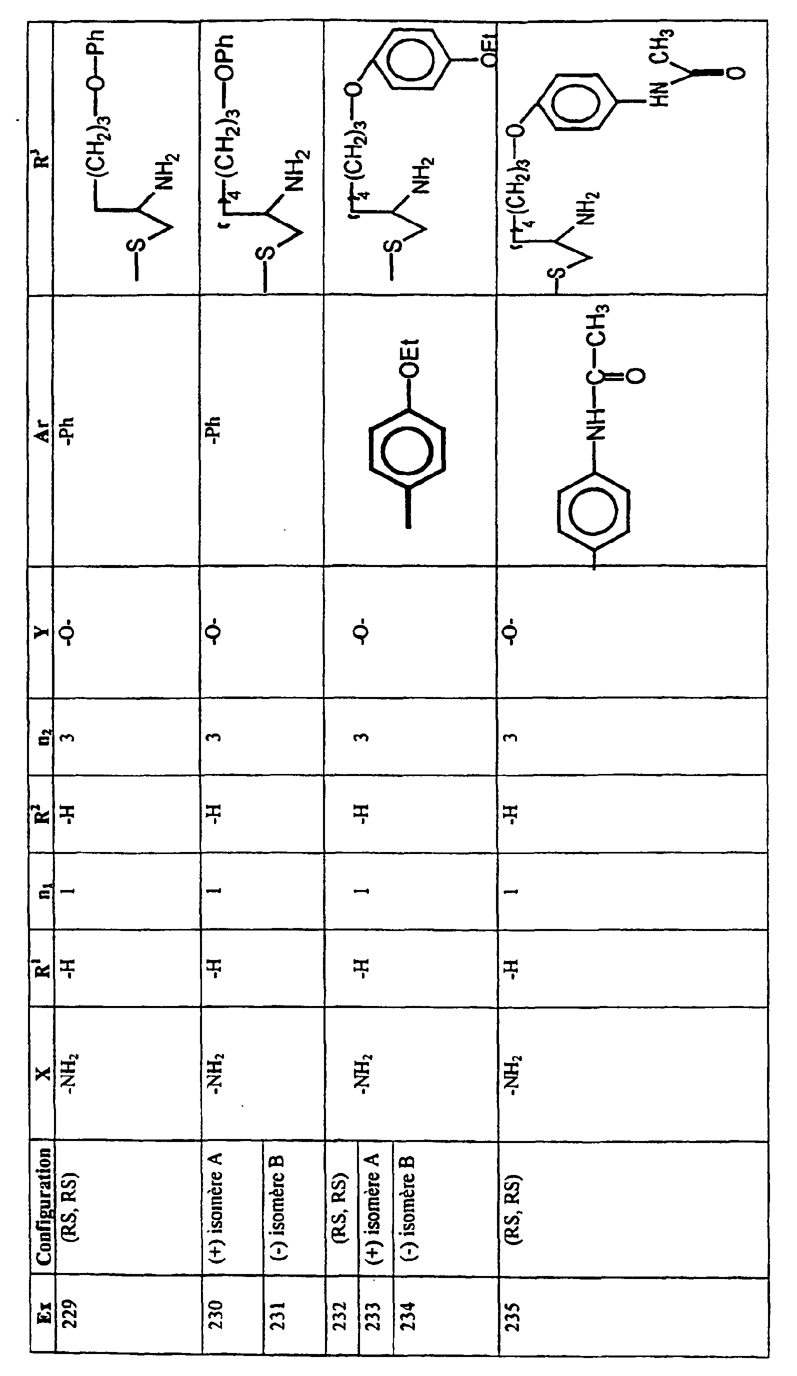

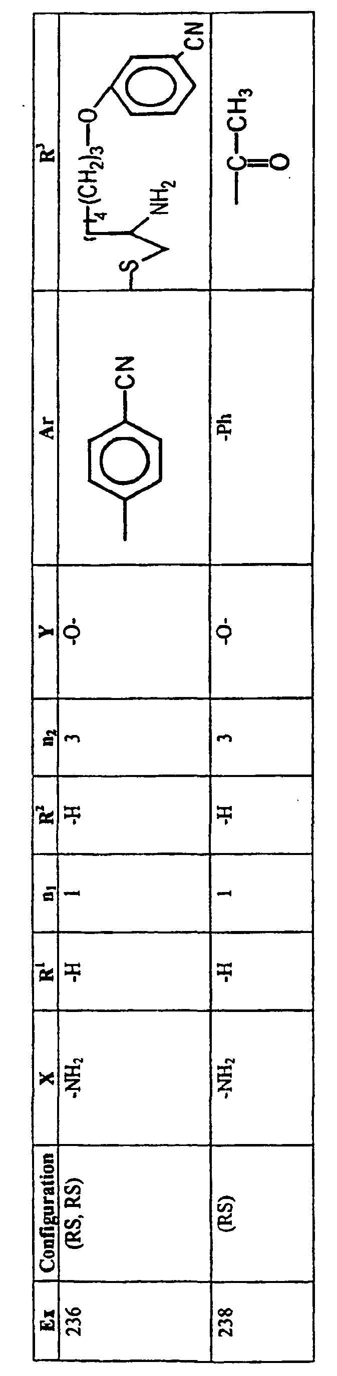

Dans les exemples donnés ci-après, les abréviations suivantes ont été utilisées pour les données RMN : s = singulet, d = doublet, t = triplet, q = quadruplet, m = multiplet.

### Exemple 1

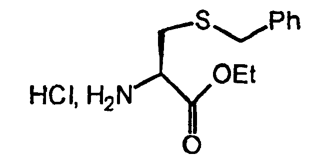

A une suspension de 5 g (23,6 mmol) de (S)-S benzyl cystéine dans 50 ml d'EtOH refroidie à 0°C, on ajoute 4,7 g de chlorure de thionyle.

On chauffe ensuite au reflux pendant 3 heures, on concentre le milieu à l'évaporateur rotatif et on reprend le résidu avec 50 ml Et₂O.

On filtre, on lave le précipité avec de l'Et₂O et on sèche sous vide. On obtient 6,37 g de solide blanc (98 %).

La RMN ¹H à 200 MH_{z} est en accord avec la structure.

### Exemple 2

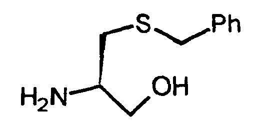

A une suspension de 5,6 g (20,25 mmol) de produit de l'exemple 1, on ajoute à 0°C, une solution de 2,45 g (24,2 mmol) de NEt₃ dans 75 ml d'Et₂O. On agite pendant 2 heures à 0°C, on filtre et on évapore le filtrat à sec. On obtient 2,7 g (11,3 mmol) d'amino-ester libre que l'on reprend avec 7 ml d'Et₂O anhydre et que l'on ajoute à 0°C sur une suspension de 0,52 g (13,7 mmol) de LAH dans 36 ml d'Et₂O anhydre. On agite une nuit à température ambiante puis on hydrolyse successivement par 0,5 ml d'eau, 0,5 ml de soude à 15 % et 1,5 ml d'eau.

On agite 2 heures à température ambiante, on filtre, on rince le précipité à l'Et₂O et on concentre le filtrat sous vide. On obtient ainsi 1,64 g (8,3 mmol) d'aminoalcool huileux.

Les exemples 3 à 6 sont préparés selon la même suite réactionnelle.

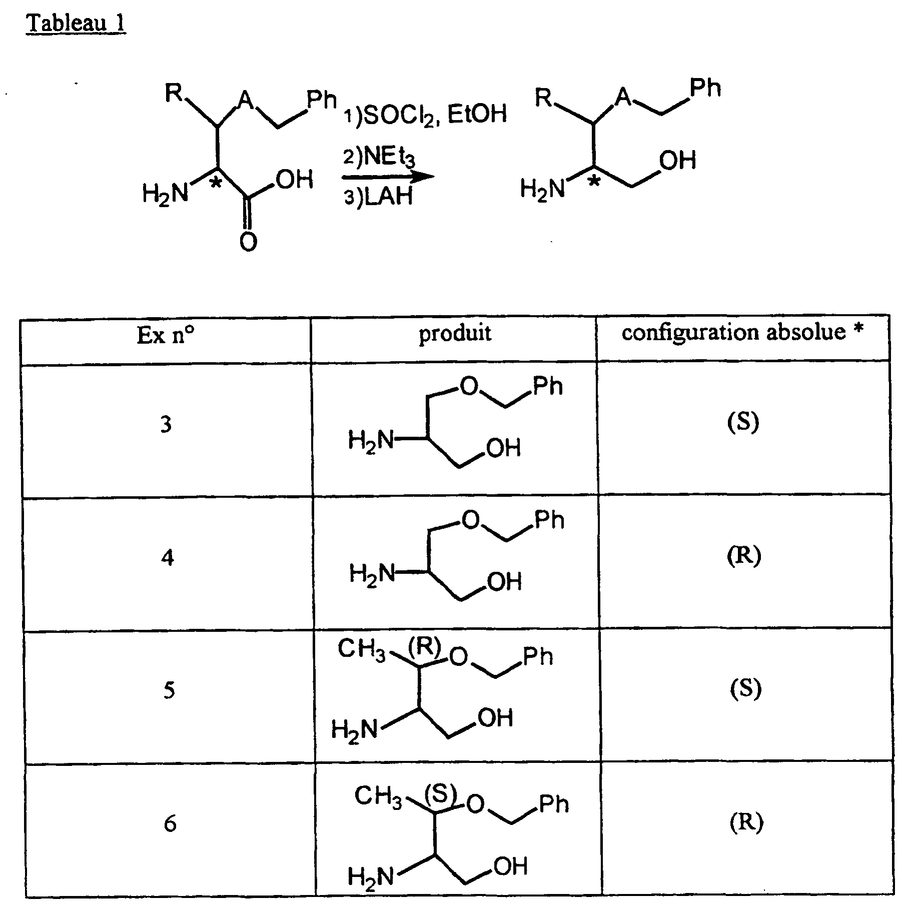

### Exemple 7

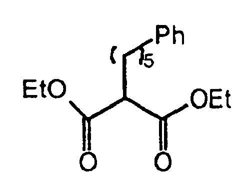

Une solution d'éthylate de sodium préparée à partir de 12 g (300 mmol) de sodium dans 306 ml d'EtOH est ajoutée à un mélange de 141 g (881,15 mmol) de malonate de diéthyle et de 38,4 g (210 mmol) de 5-phényl 1-chloropentane. On porte au reflux pendant 4 heures.

On concentre sous vide, on reprend le résidu avec de l'eau et on extrait avec de l'Et₂O.

La phase éthérée est lavée 3 fois avec de l'eau, séchée sur MgSO₄, filtrée puis concentrée. L'excès de diéthylmalonate est éliminé par distillation sous vide.

On obtient 57,2 g (Rendement 89 %) d'huile jaune.

### Exemple 8

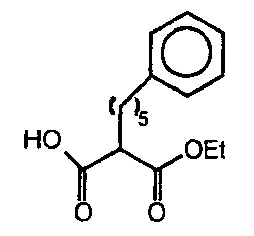

A une solution de 12,25 g (40 mmol) de diester de l'exemple 7 dans 21 ml d'EtOH, on ajoute à 0°C une solution de 2,7 g (40,9 mmol) de potasse dans 58 ml d'EtOH.

On agite pendant une nuit à 0°C.

Le milieu est ensuite concentré. Le résidu est repris avec 100 ml d'eau et lavé à l'Et₂O (2 fois 30 ml). La phase aqueuse est refroidie puis acidifiée par une solution d'acide chlorhydrique concentré. La phase aqueuse est extraite avec de l'éther (2 fois 40 ml). Les phases éthérées sont réunies, séchées sur MgSO₄, filtrées et concentrées. On obtient ainsi 9,8 g (Rendement 88 %) d'huile jaune très visqueuse.

### Exemple 9

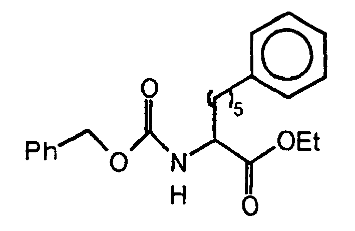

A une solution de 9,8 g du mono-acide de l'exemple 8 dans 36 ml de toluène, on ajoute goutte à goutte 10,34 g (37,53 mmol) de DPPA puis 3,77 g (37,32 mmol) de NEt₃. On chauffe à 80°C pendant 1 heure. On revient à température ambiante et on ajoute 4,75 g (43,98 mmol) d'alcool benzylique et on chauffe à 80°C pendant une nuit. La phase toluènique est successivement lavée à l'eau (1 fois 10 ml), par une solution aqueuse saturée d'hydrogénocarbonate de sodium (1 fois 10 ml) et à l'eau (1 fois 5 ml). On sèche la phase organique sur MgSO₄, on la filtre et on la concentre sous vide.

On obtient ainsi 13,1 g de produit brut.

Celui-ci est purifié par chromatographie éclair sur silice avec le mélange éther éthylique - éther de pétrole (3/7) comme éluant. 8,1 g de carbamate sont obtenus (21 mmol ; Rendement = 61 %).

### Exemple 10

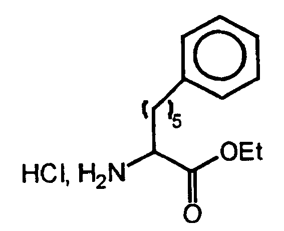

On met en solution 8,1 g (21 mmol) du carbamate de l'exemple 9 dans 60 ml d'EtOH. On ajoute ensuite 200 mg de Pd/C à 10 % puis on hydrogène à une pression d'environ 1 bar pendant une nuit à température ambiante. La suspension est filtrée sur célite puis évaporée à sec. Le résidu huileux est repris par une solution aqueuse d'HCl concentré. La phase aqueuse acide est lavée à l'Et₂O (2 fois 20 ml). La phase aqueuse est évaporée à sec et le résidu est séché sous vide sur P₂O₅ jusqu'à masse constante. On obtient ainsi 5,4 g (Rendement 90 %) de solide blanc.

### Exemple 11

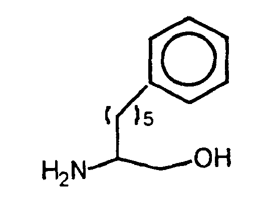

Le produit de l'exemple 10 est réduit en aminoalcool 11 selon le procédé décrit à l'exemple 2. Rendement = 84 %.

### Exemple 12

L'exemple 12 est préparé selon la même suite réactionnelle que celle décrite pour l'exemple 11.

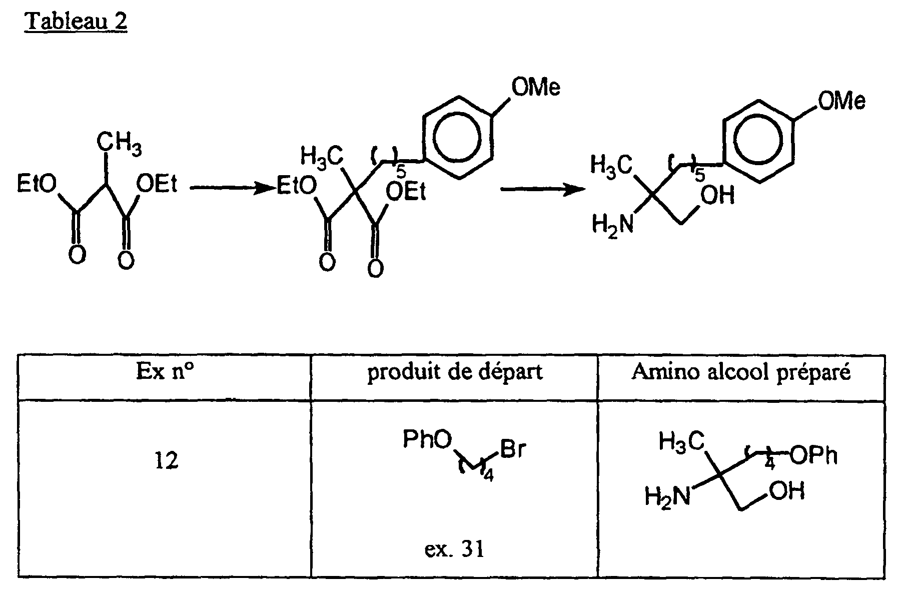

### Exemple 13

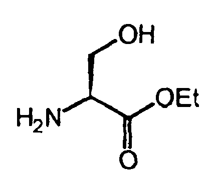

Une solution de 16,9 g (100 mmol) de chlorhydrate de sérinate d'éthyle obtenu selon le procédé décrit à l'exemple 1 mais en partant de la sérine, dans 150 ml de chloroforme est refroidie à 0-5°C. On ajoute 500 ml d'Et₂O et 10,12g (100 mmol) de triéthylamine.

On agite pendant 2 heures à 0°C. On filtre et évapore à sec.

On obtient 11,98 g d'huile incolore (90 %).

### Exemple 14

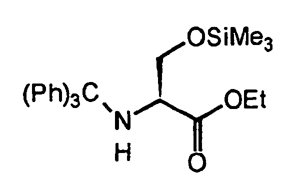

Les 11,98 g (90 mmol) du produit de l'exemple 13 sont mis en solution dans 155 ml de CH₂Cl₂. On ajoute sous atmosphère inerte 22,78 g (209,68 mmol) de chlorure de triméthylsilyle.

On porte au reflux pendant 20 minutes puis revient à température ambiante. On ajoute alors 21,2 g (209,90 mmol) de triéthylamine dans 60 ml de CH₂Cl₂ puis on porte au reflux pendant 45 minutes.

On refroidit ensuite à 0°C et on ajoute une solution de 5,4 ml (135 mmol) de méthanol anhydre dans 22 ml de CH₂Cl₂. On laisse remonter le milieu à température ambiante puis on ajoute successivement 9,1 g (90 mmol) de NEt₃ et 25 g (90 mmol) de chlorure de trityle et on agite pendant une nuit à température ambiante.

On concentre sous vide, on reprend avec 200 ml d'Et₂O. On lave à l'eau (une fois 30 ml).

On sèche sur MgSO₄, on filtre et on concentre sous vide.

On obtient 38,8 g du composé désiré.

### Exemple 15

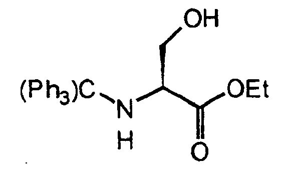

A une solution de 38,8 g de produit de l'exemple 14 dans 53 ml de THF, on ajoute à température ambiante 50 ml d'une solution molaire de fluorure de tétrabutylammonium (NBu₄F) dans le THF. On agite pendant 10 minutes à température ambiante.

On ajoute ensuite 500 ml dEt₂O et on lave la phase organique successivement avec une solution aqueuse saturée d'hydrogénocarbonate de sodium (2 fois 60 ml) puis une solution aqueuse saturée de thiosulfate de sodium (2 fois 60 ml). La phase organique est séchée sur MgSO₄, filtrée et concentrée sous vide. Le résidu huileux obtenu est purifié par chromatographie éclair en utilisant le mélange éther de pétrole/Et₂O (1/1) puis Et₂O comme éluants.

On obtient 29,31 g (78 mmol) du composé désiré.

### Exemple 16

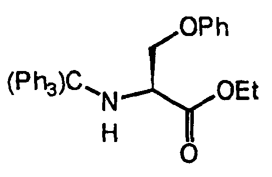

A une solution de 19,85 g (52,59 mmol) d'aminoester de l'exemple 15 dans 300 ml de toluène, on ajoute successivement 14,75 g (56,23 mmol) de triphénylphosphine et 7,2 g (76,5 mmol) de phénol. Le milieu réactionnel est agité vivement durant 5 minutes, puis on ajoute 11,37 g (56,2 mmol) d'azodicarboxylate de diisopropyle.

On agite une nuit à température ambiante, on filtre le milieu réactionnel et on évapore à sec. Le résidu huileux est purifié par chromatographie éclair en utilisant le mélange éther-éther de pétrole (5/95) comme éluant. On obtient ainsi 15,43 g (34 mmol) du composé désiré.

### Exemple 17

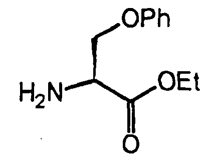

7,6 g (16,7 mmol) d'aminoester de l'exemple 16 sont agités vivement durant 5 heures à température ambiante en présence de 104 ml d'acide formique. Le milieu réactionnel est ensuite évaporé à sec, et l'on obtient un solide blanc que l'on reprend avec 100 ml d'eau. La phase aqueuse est lavée avec Et₂O (3 fois 20 ml) puis est basifiée à l'aide d'hydrogénocarbonate de sodium. La phase aqueuse basique est ensuite extraite avec de l'acétate d'éthyle (3 fois 20 ml). La phase organique est séchée sur MgSO₄, filtrée et concentrée sous vide. On obtient 2,1 g (10,1 mmol) du composé désiré.

### Exemple 18

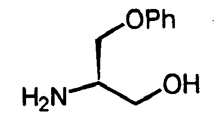

Les 2,1 g (10,1 mmol) d'aminoester de l'exemple 17 sont repris avec 4 ml d'Et₂O anhydre et ajoutés à 0°C sur une suspension de 0,46 g (12,1 mmol) de LAH dans 30 ml d'Et₂O anhydre. On agite une nuit à température ambiante puis on hydrolyse successivement par 0,3 ml d'eau, 0,3 ml de soude à 15 % et 0,9 ml d'eau. On agite 2 heures à température ambiante, on filtre, on rince le précipité à l'Et₂O et on concentre le filtrat sous vide. On obtient ainsi 1,35 g (8,1 mmol) d'aminoalcool huileux.

### Exemple 19

L'exemple 19 est préparé selon la même suite réactionnelle que celle décrite pour l'exemple 18.

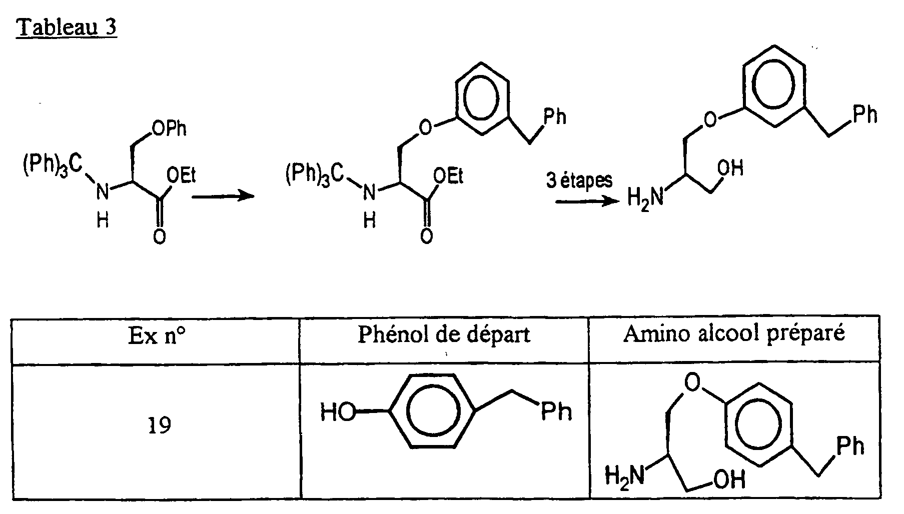

### Exemple 20

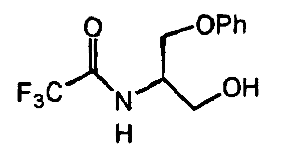

A une solution de 2,65 g (15,85 mmol) de l'aminoalcool 18 dans 10 ml de pyridine, on ajoute à 0°C, 2,6 g (15,85 mmol) de trifluoroacétylimidazole. Après 30 minutes d'agitation à 0°C, on acidifie le milieu réactionnel avec de l'acide phosphonique 10 % aqueux, et on extrait la phase aqueuse avec de l'Et₂O (2 fois 15 ml). Les phases éthérées réunies sont lavées avec H₃PO₄ 10 % (1 fois 10 ml) puis séchées sur MgSO₄. On filtre et on concentre sous vide. Le produit brut est purifiée par chromatographie éclair en utilisant le mélange Et₂O/éther de pétrole (1/1) comme éluant. On obtient ainsi 2,31 g (8,77 mmol) de produit attendu.

### Exemple 21

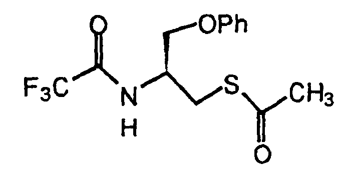

A 3,45 g (13,15 mmol) de triphénylphosphine en solution dans 32 ml de THF, on ajoute à 0°C 2,3 g (13,2 mmol) de DEAD. On agite 30 minutes à 0°C (apparition d'un précipité blanc). On ajoute alors à cette température une solution de 1 g (13,14 mmol) d'acide thioacétique et de 2,31 g (8,77 mmol) d'alcool obtenu à l'exemple 20 dans 16 ml de THF. On agite 2 heures à 0°C puis on laisse le milieu réactionnel revenir à température ambiante. Après une nuit d'agitation, on évapore à sec. Le résidu est repris par 100 ml d'Et₂O et la phase organique est ensuite lavée par une solution aqueuse saturée de NaHCO₃ (3 fois 15 ml). On sèche la phase éthérée sur MgSO₄, on filtre et on concentre sous vide. Le résidu est repris par 60 ml d'un mélange AcOEt - éther de pétrole (1/1) et agité 1 heure à 0°C. On filtre et on évapore le filtrat sous vide. On obtient 4,92 g de produit brut qui est purifié par chromatographie éclair en utilisant le mélange éther-éther de pétrole (2/8) comme éluant. On récupère ainsi 1,6 g (4,95 mmol) de produit attendu.

### Exemple 22

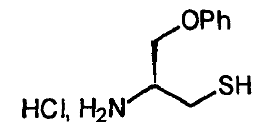

A 1,6 g (4,95 mmol) de produit obtenu à l'exemple 21, on ajoute sous argon 35 ml d'une solution de soude 2N (eau-éthanol 1/1). On porte le milieu réactionnel 2 heures à 50°C, on revient à température ambiante et on élimine l'EtOH sous vide. On refroidit le milieu réactionnel à 5°C et on acidifie sous argon à l'aide d'HCl 6N. La phase aqueuse acide est lavée avec Et₂O (2 fois 10 ml) puis concentrée sous vide. Le résidu solide est extrait par du chloroforme au reflux. Après filtration, les phases chloroforme sont évaporées à sec. On obtient un solide blanc qui est purifié par trituration dans 10 ml d'Et₂O anhydre. Après filtration, le solide est séché au dessicateur sur P₂O₅.

On obtient 0,73 g (3,3 mmol) de chlorhydrate d'aminothiol.
RMN ¹H (200 MHz, D₂O) : 7,35 à 7,15 (m, 2H) ; 7,0 à 6,8 (m, 3H) ; 4,60 (s, 4H) ; 4,3 à 4,0 (m, 2H) ; 3,75 à 3,55 (m, 1H) ; 3,0 à 2,7 (m, 2H).
F = 150°C

Les exemples 23 à 30 sont préparés à partir des aminoalcools correspondants selon la même suite réactionnelle que celle décrite pour l'exemple 22 :

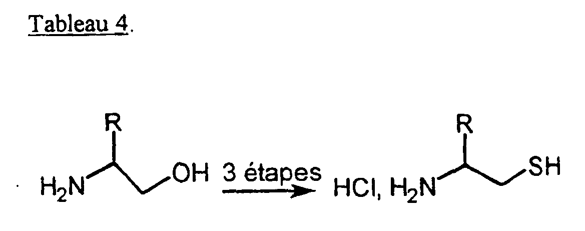

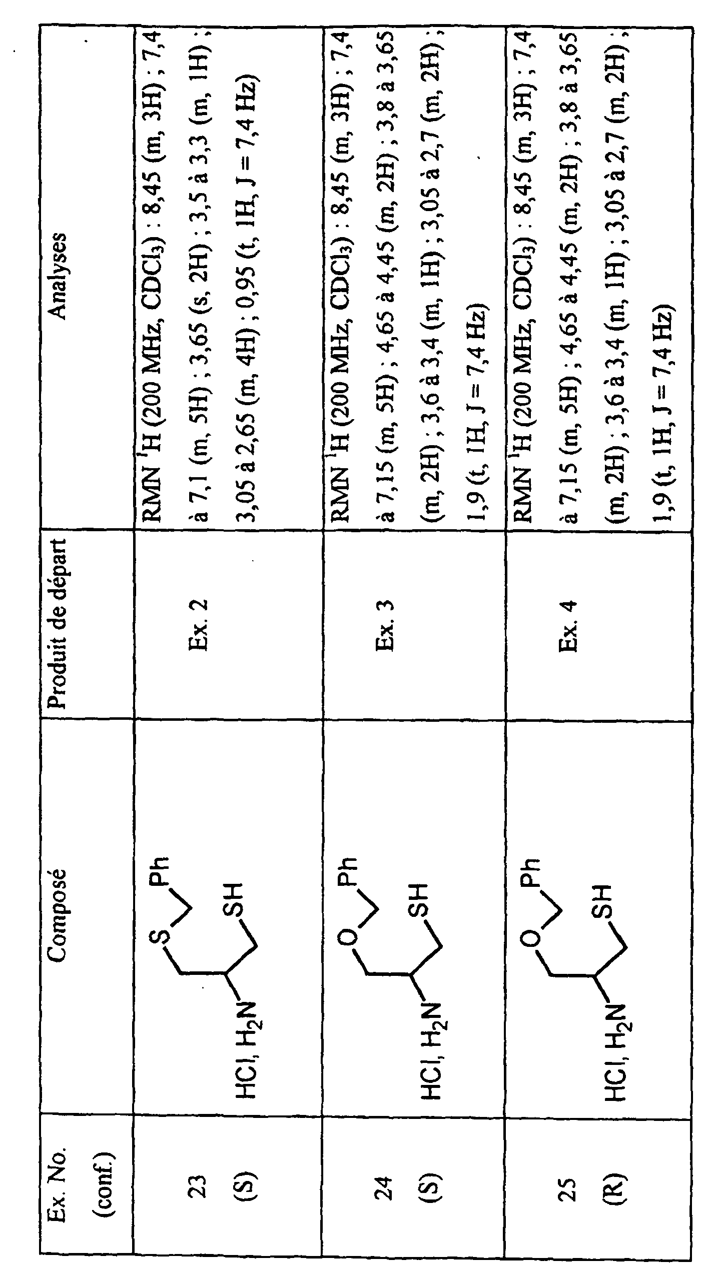

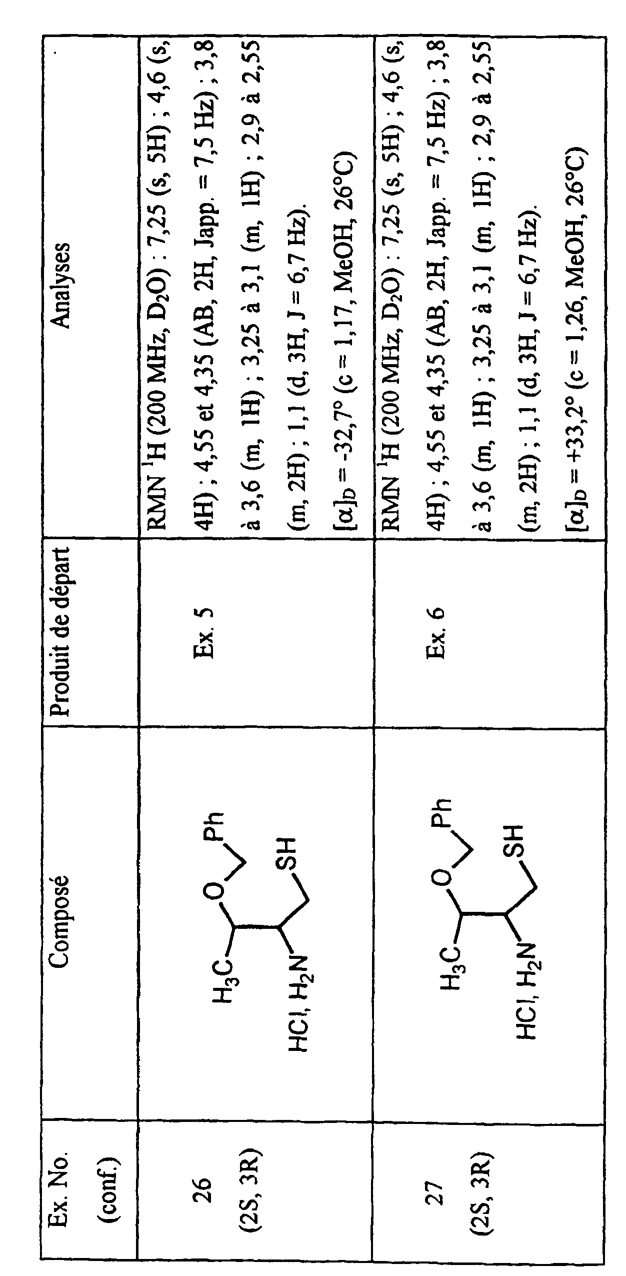

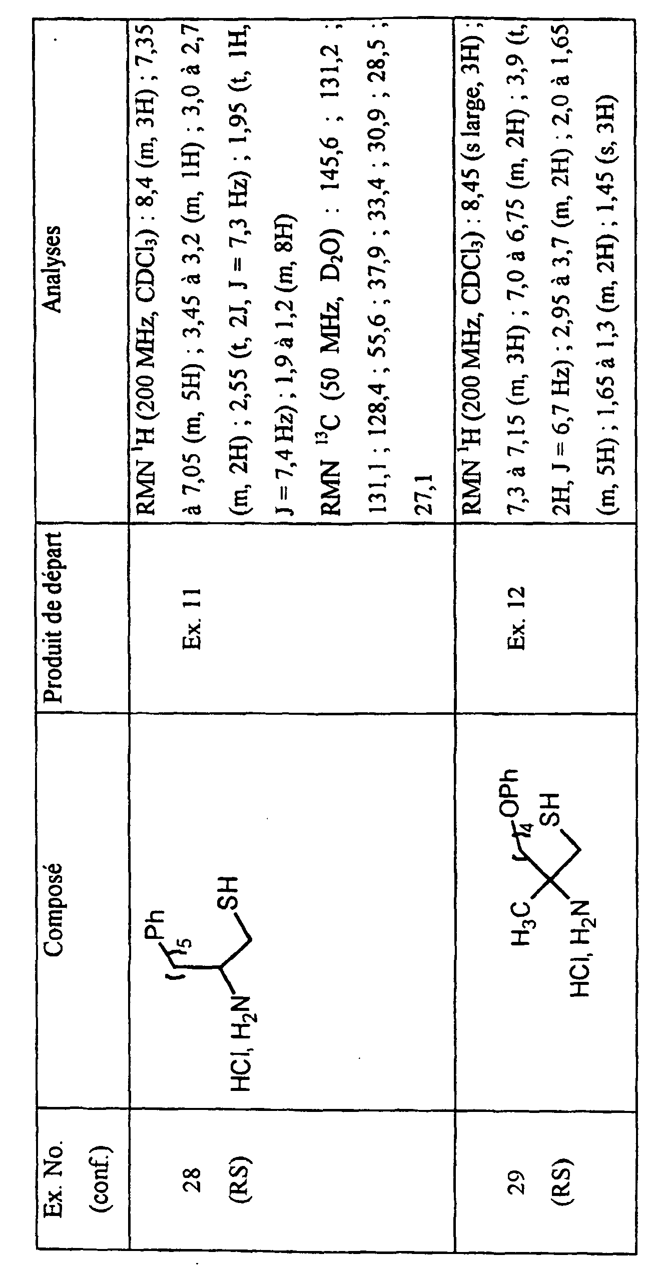

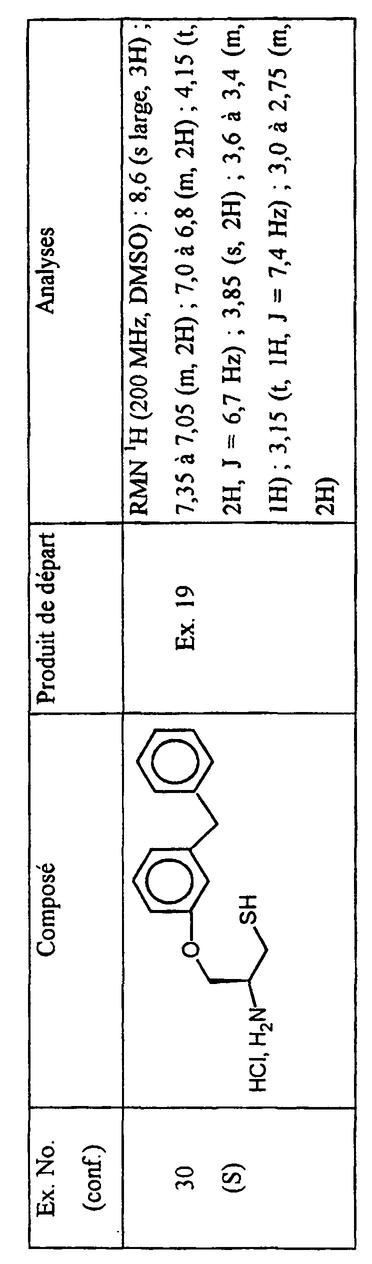

### Exemple 31 (Méthode a)

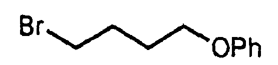

Dans un ballon, on introduit 17,41 g (185,25 mmol) de phénol, 14,5 ml de THF et 62 ml de NaOH 9N.

On ajoute goutte à goutte, 40 g (185,25 mmol) de 1,4-dibromobutane.

On agite et on porte au reflux pendant 45 minutes.

Après refroidissement, on dilue avec 50 ml d'eau puis on extrait avec 100 ml d'Et₂O. La phase organique est lavée avec 30 ml d'eau, séchée sur MgSO₄, filtrée et concentrée.

Le résidu huileux est distillé à la pompe à palette.

On récupère la fraction distillant à 80 - 105°C sous 1 mm de Hg.

On obtient 15,96 g (37 %) d'huile incolore.

### Exemple 32 (Méthode b)

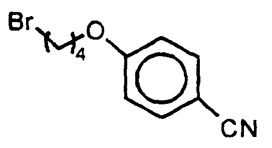

Dans un ballon, on introduit successivement 5 g (41,97 mmol) de 4-cyanophénol, 45,3 g (209,87 mmol) de 1,4-dibrobutane, 28,96 g (209,85 mmol) de carbonate de potassium en poudre et 50 ml de DMF anhydre.

On agite à température ambiante pendant 20 heures.

On filtre et on reprend le filtrat avec 300 ml d'acétate d'éthyle.

La phase organique est lavée avec une solution aqueuse saturée de NaCl (3 fois 100 ml), séchée sur Mg SO₄ filtrée et concentrée. On obtient 54,4 g d'huile que l'on distille sous le vide d'une pompe à palettes. On récupère la fraction distillant à 100-110°C sous 1 mm de Hg.

On obtient 9,6 g (90 %) d'huile incolore.

Les exemples 33 à 81 sont préparés selon une des méthodes (a ou b) précédemment décrite.

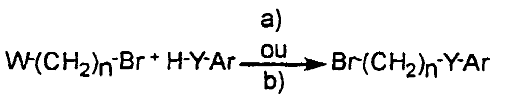

W = Cl, Br ; n varie de 3 à 11 ; Y = O, S ; Ar est tel que défini dans la formule (I)

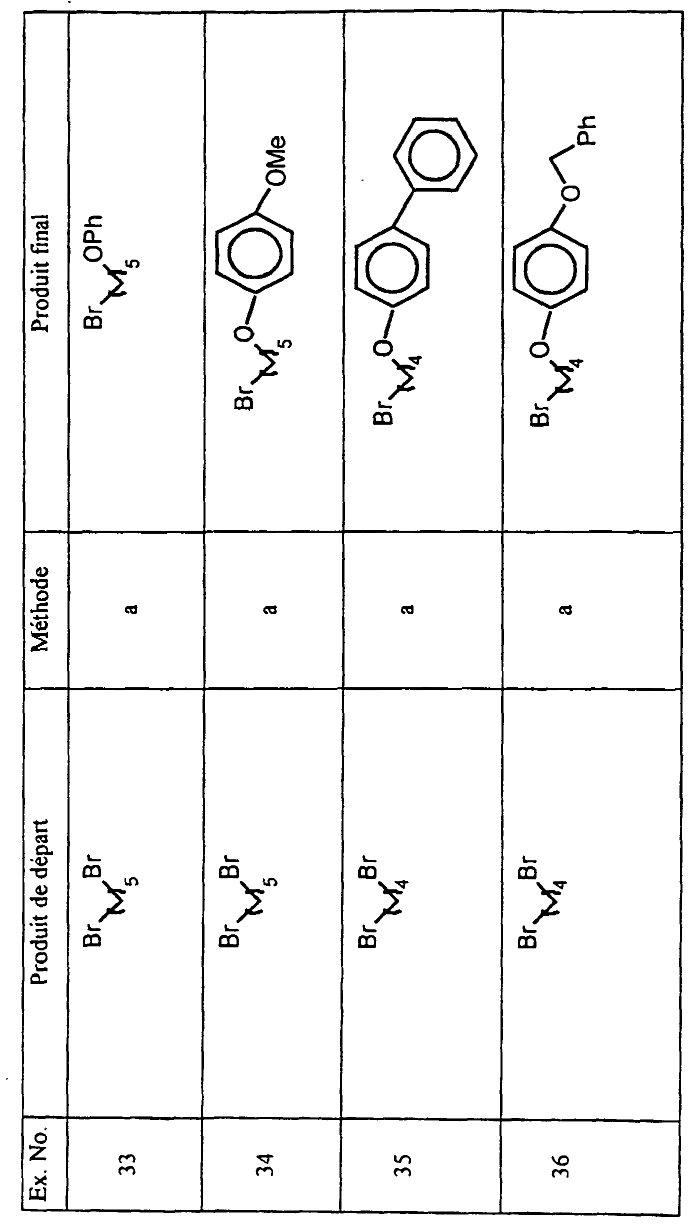

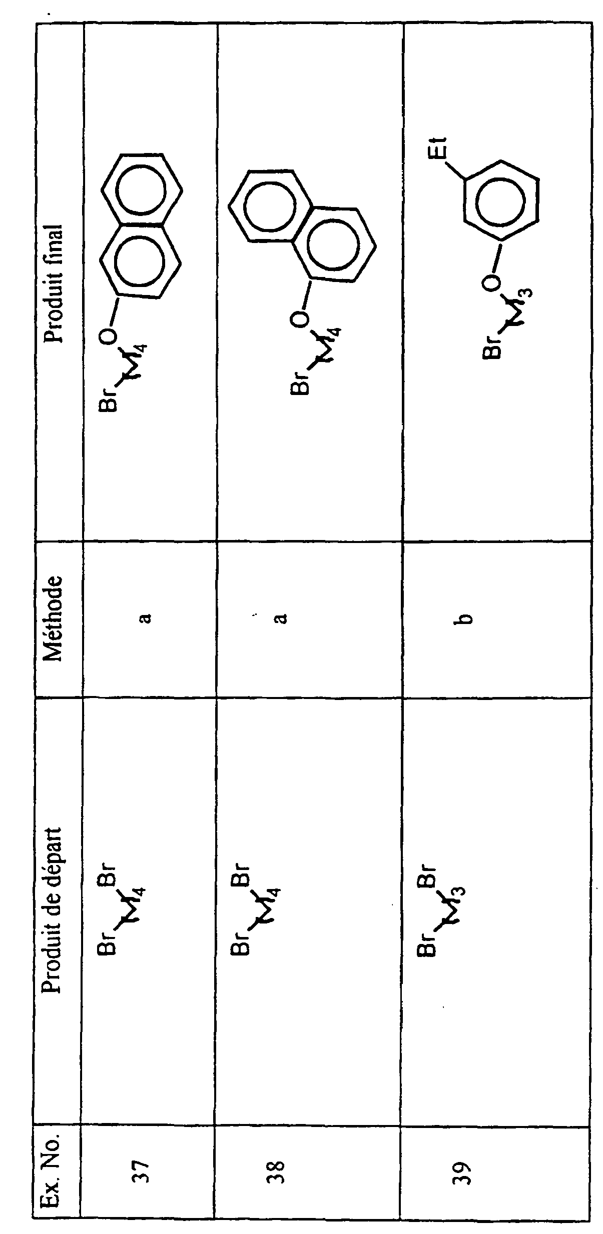

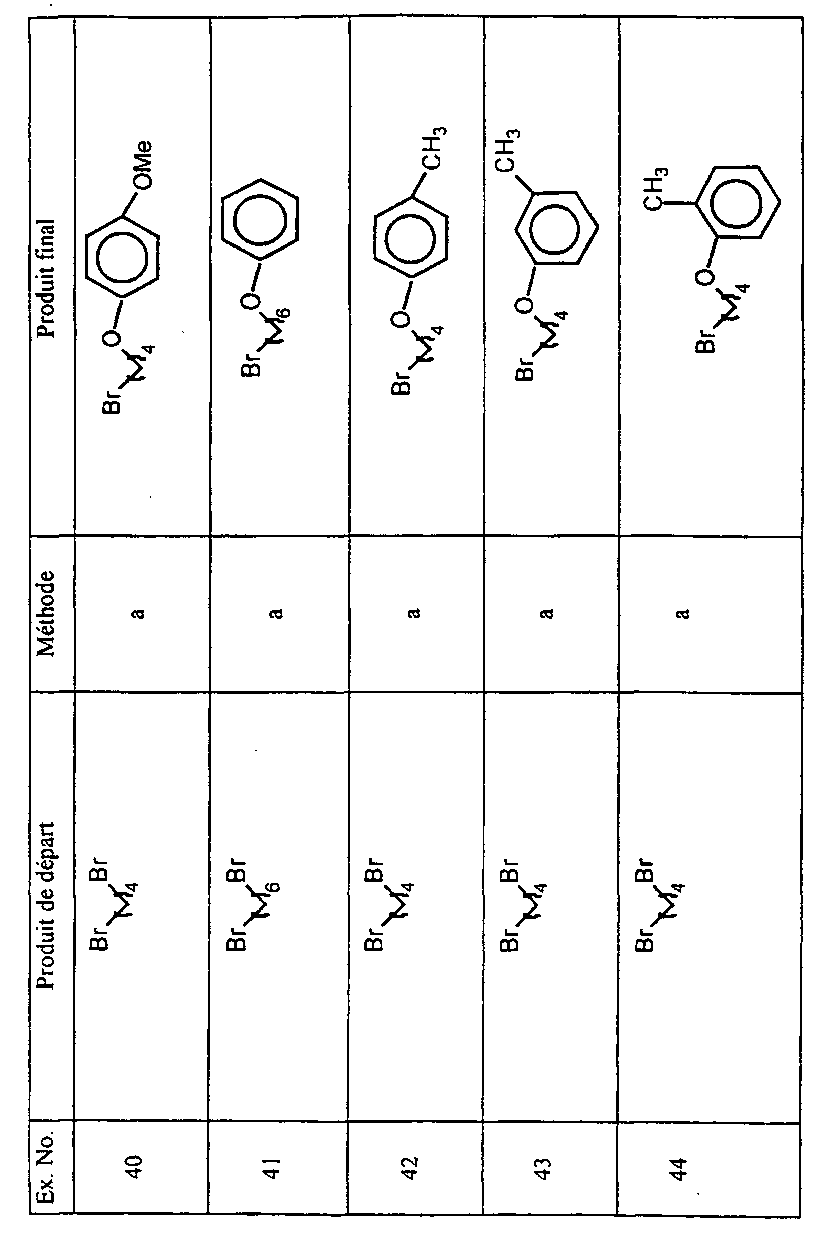

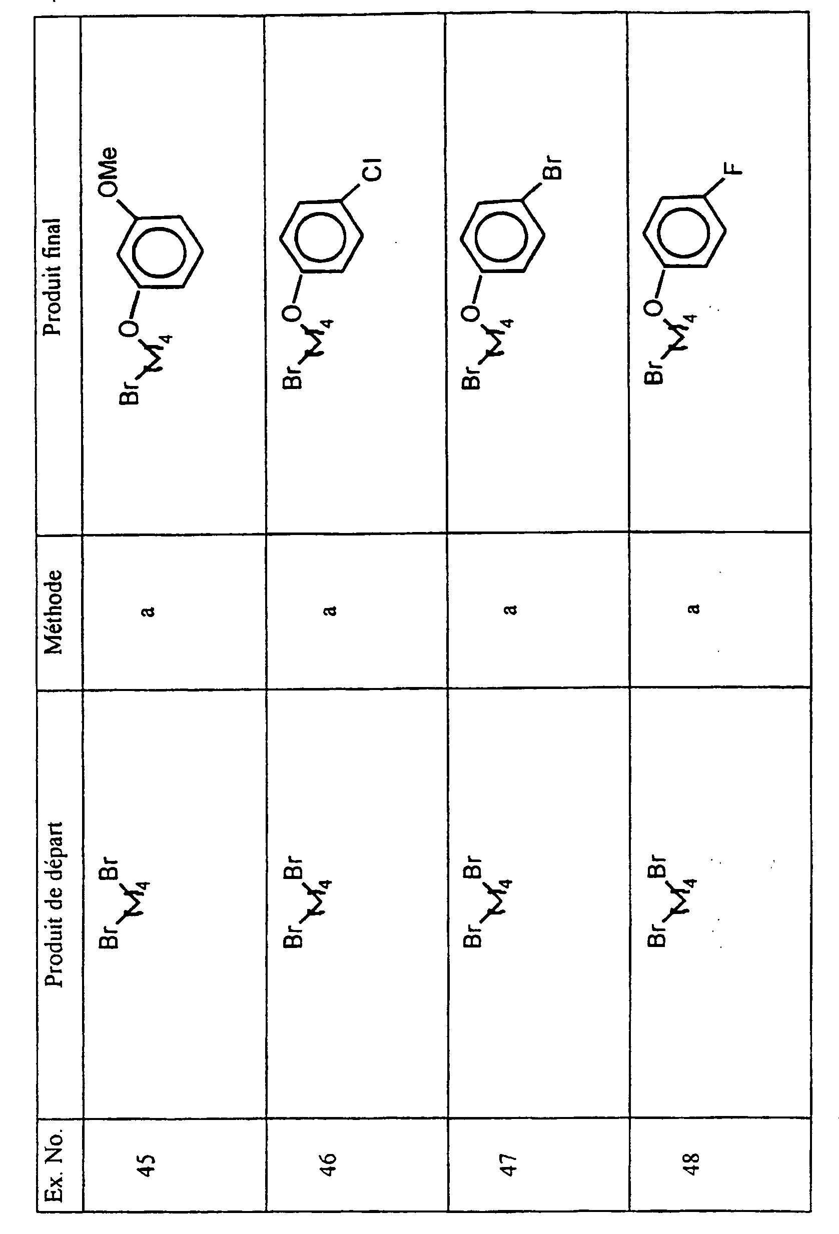

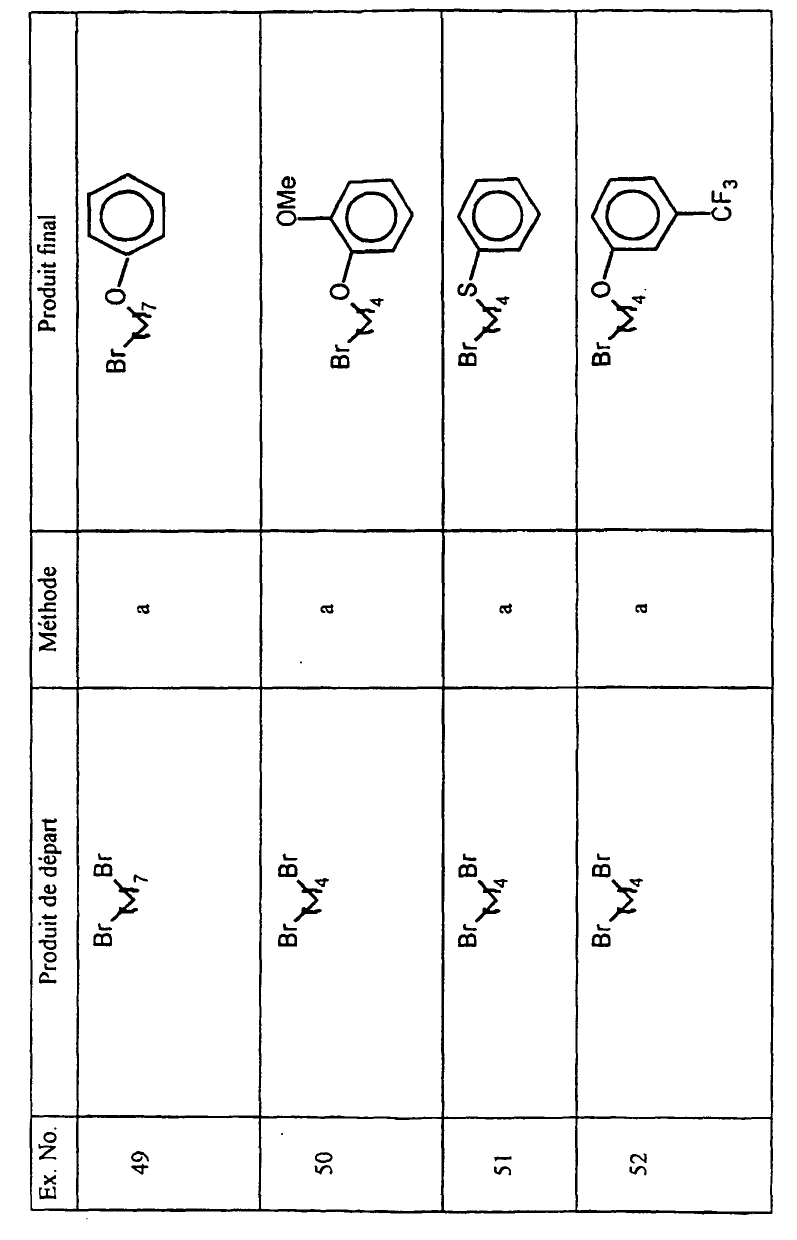

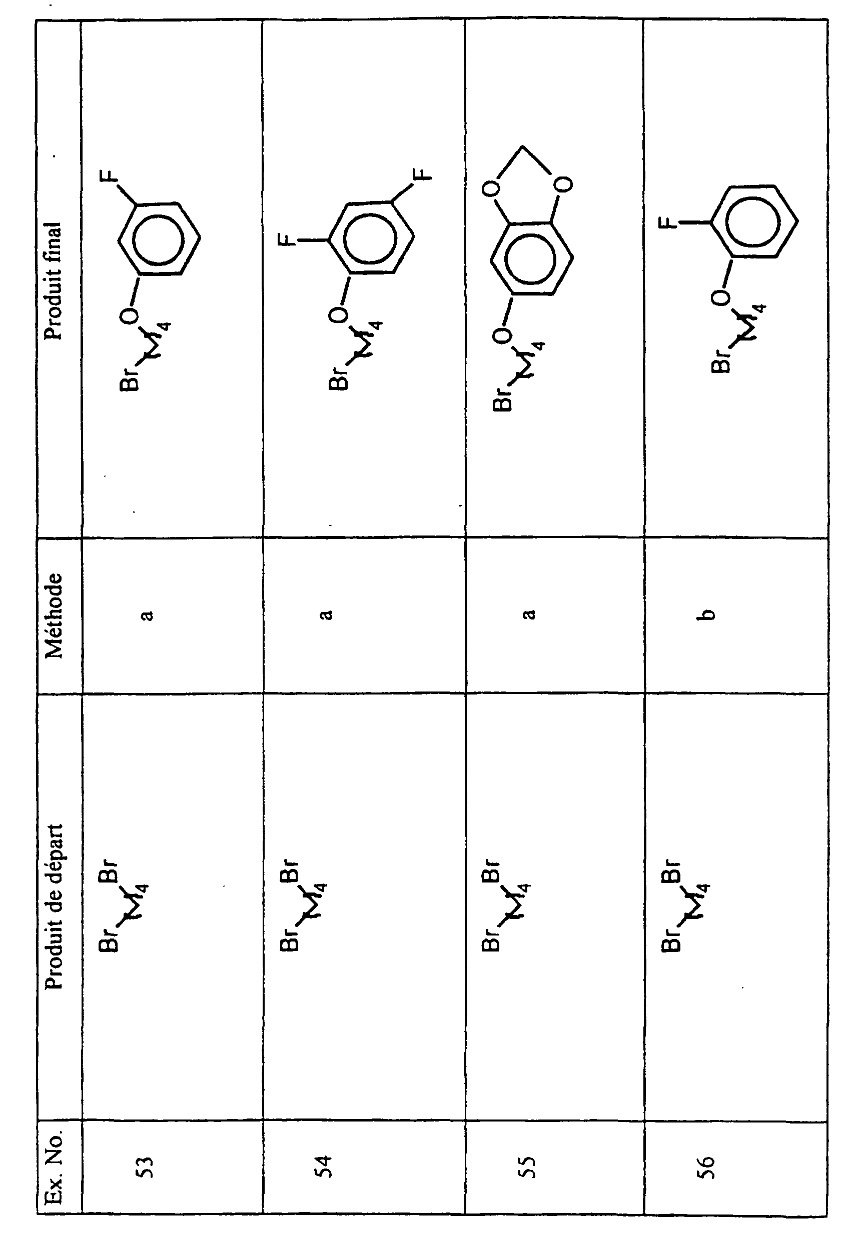

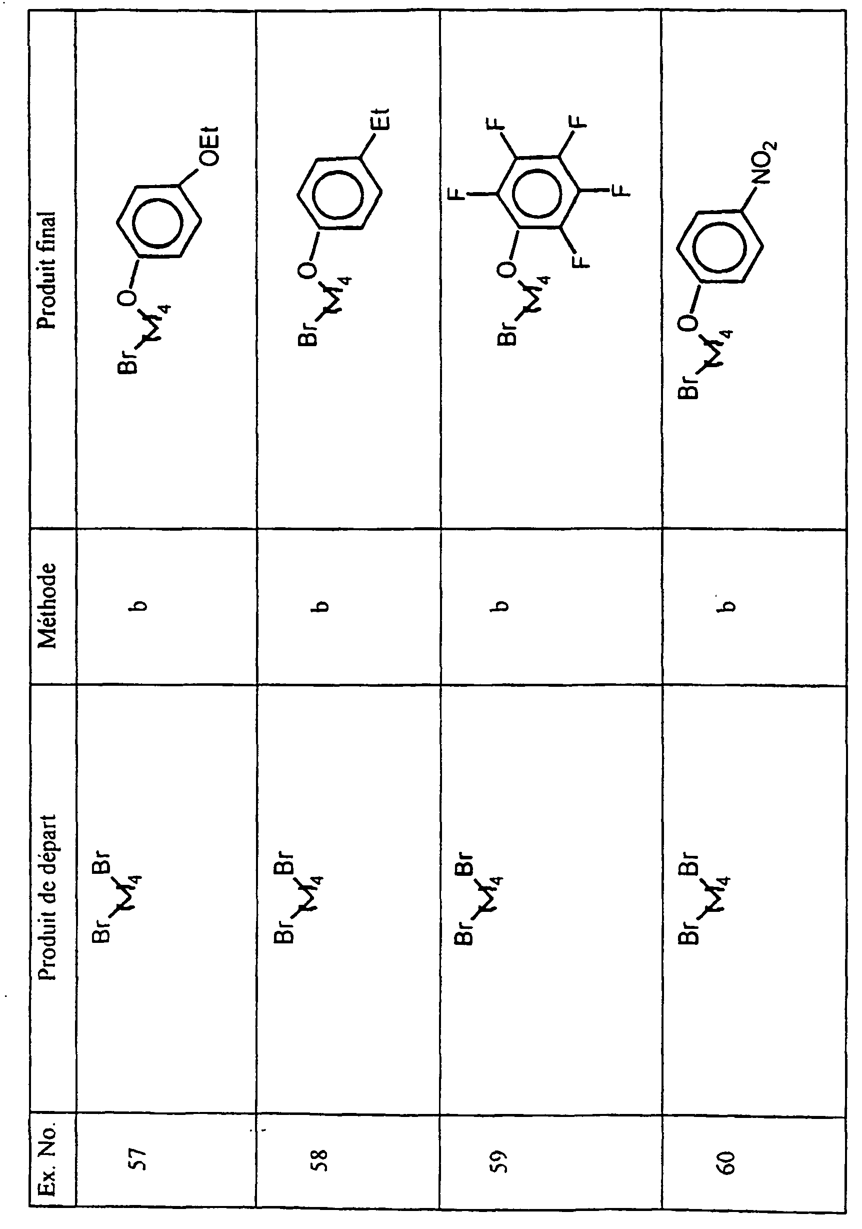

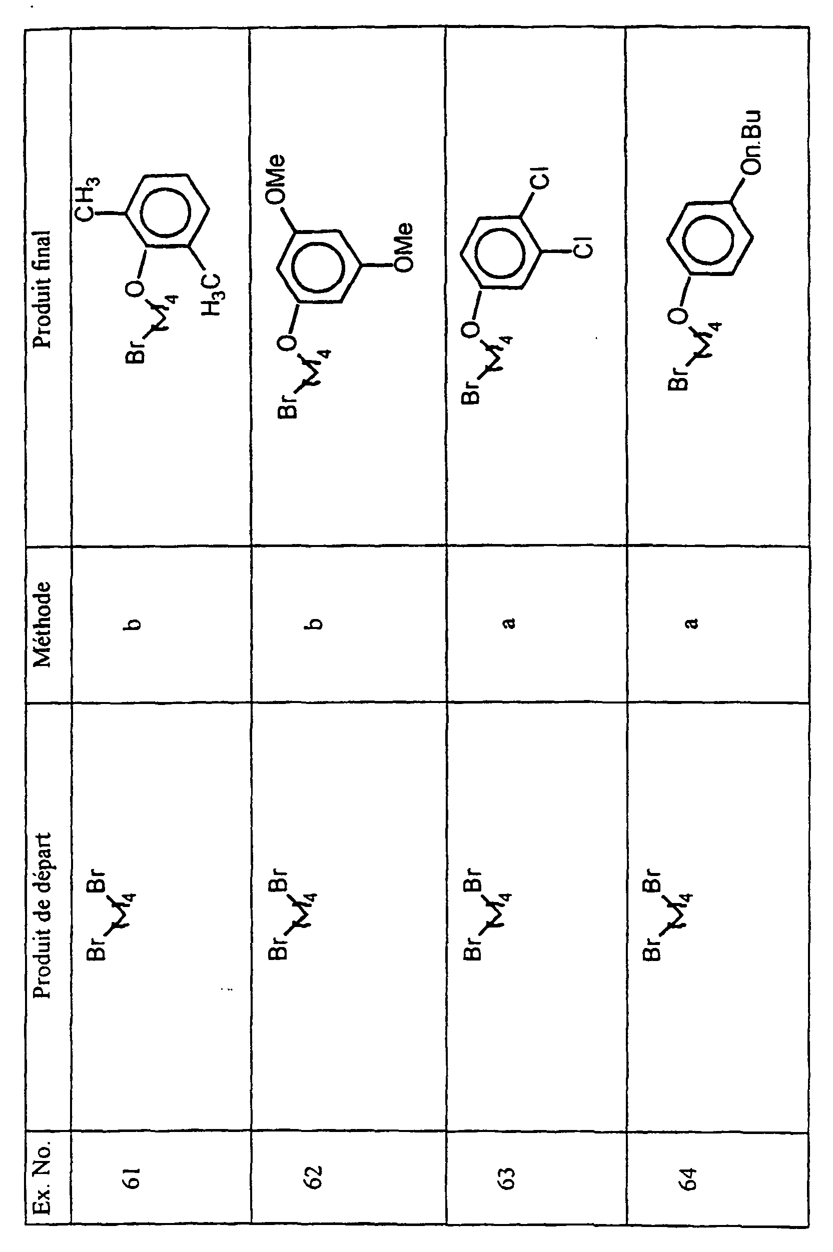

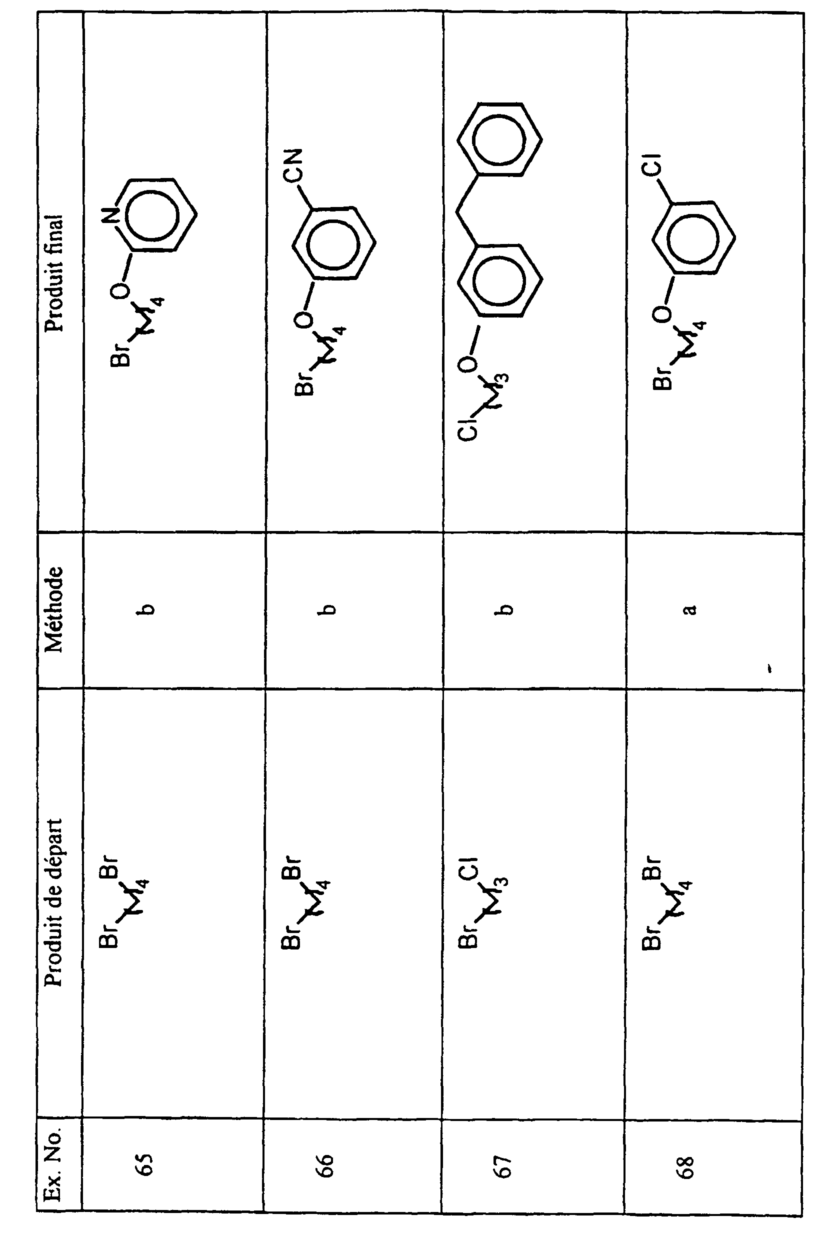

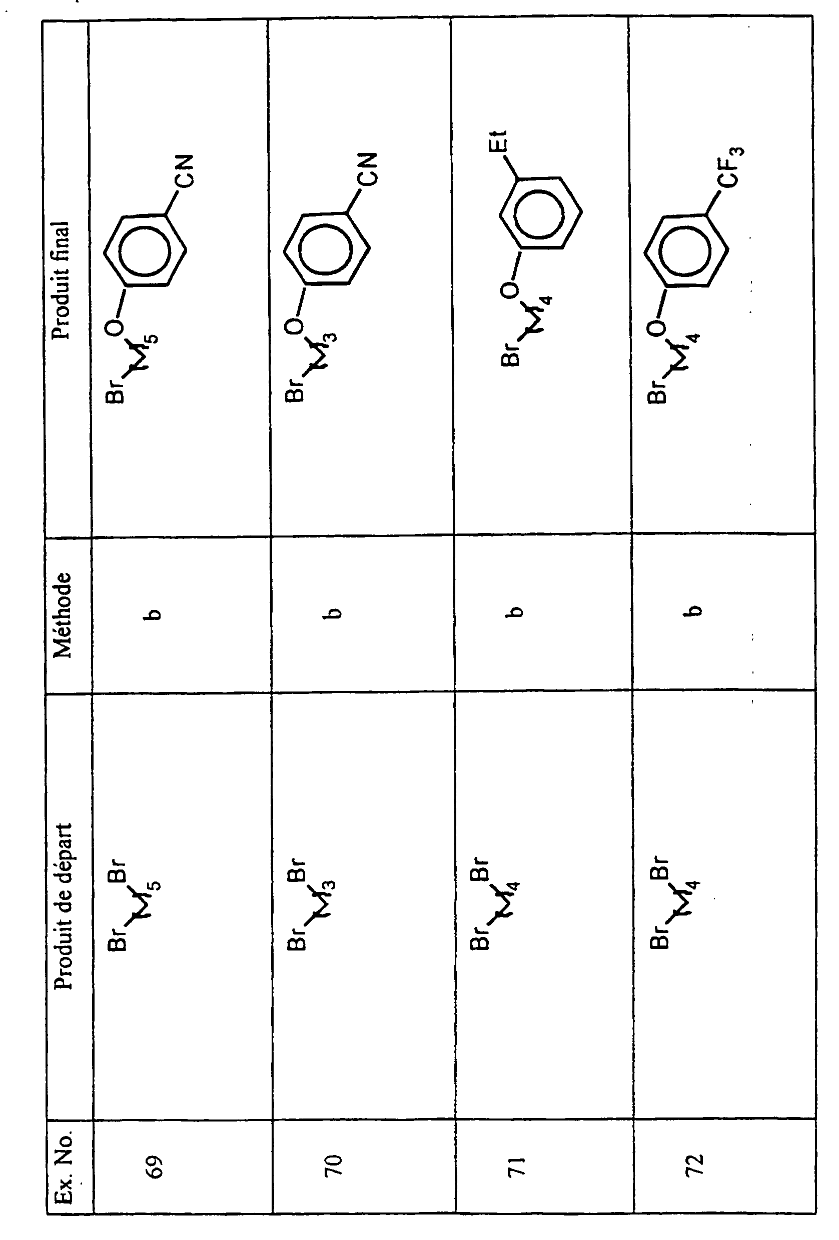

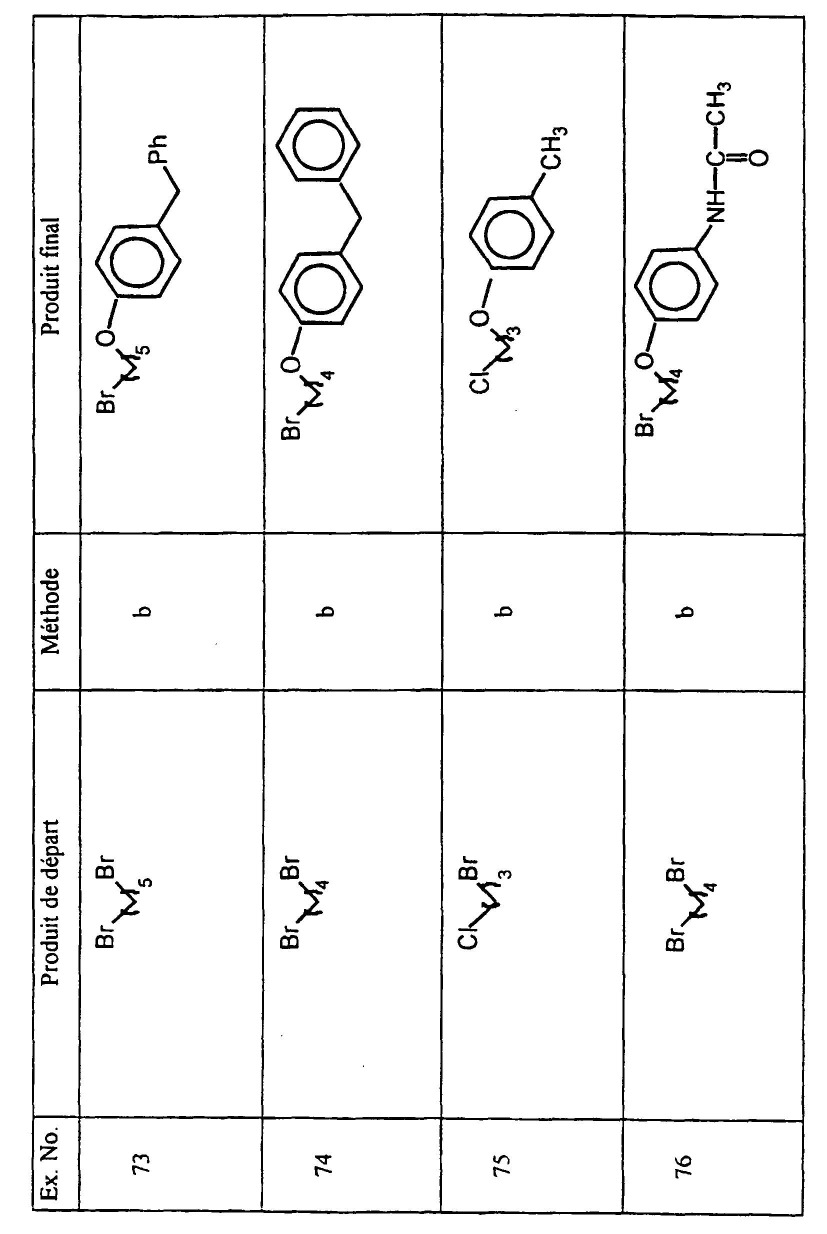

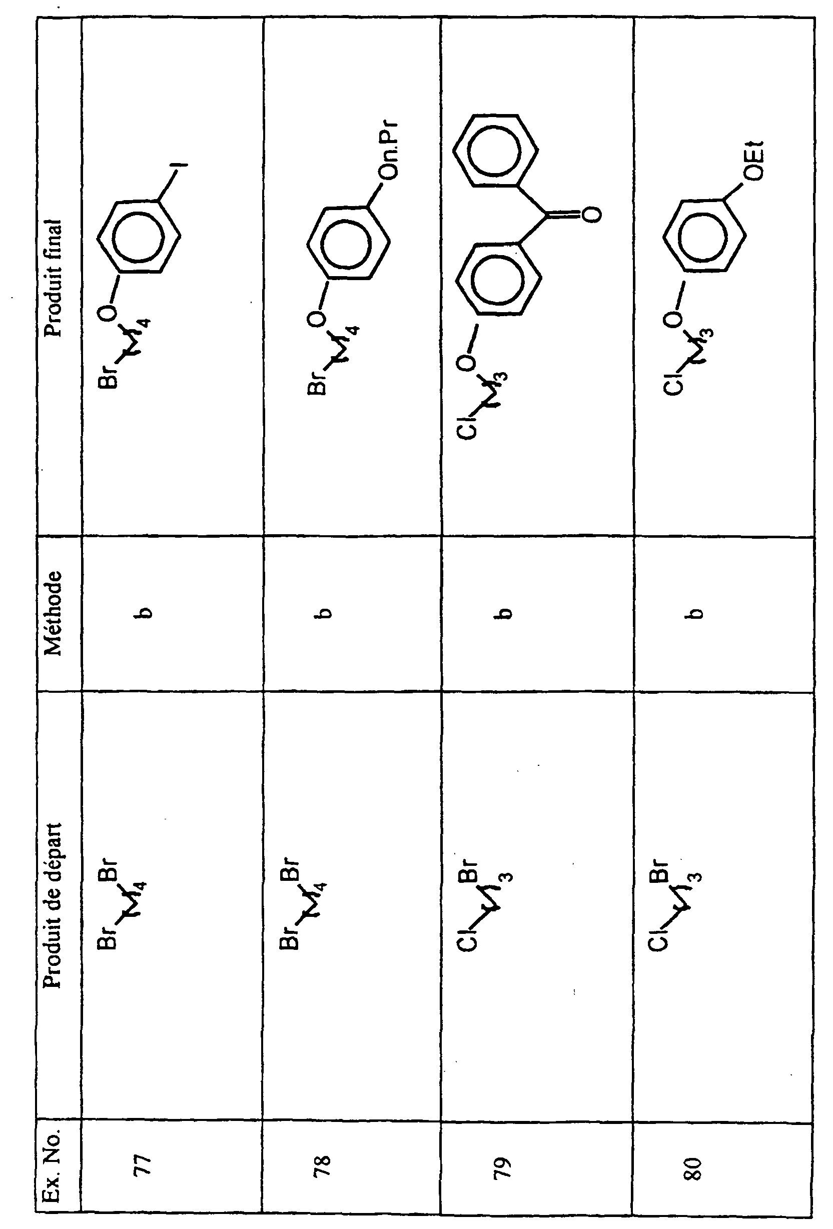

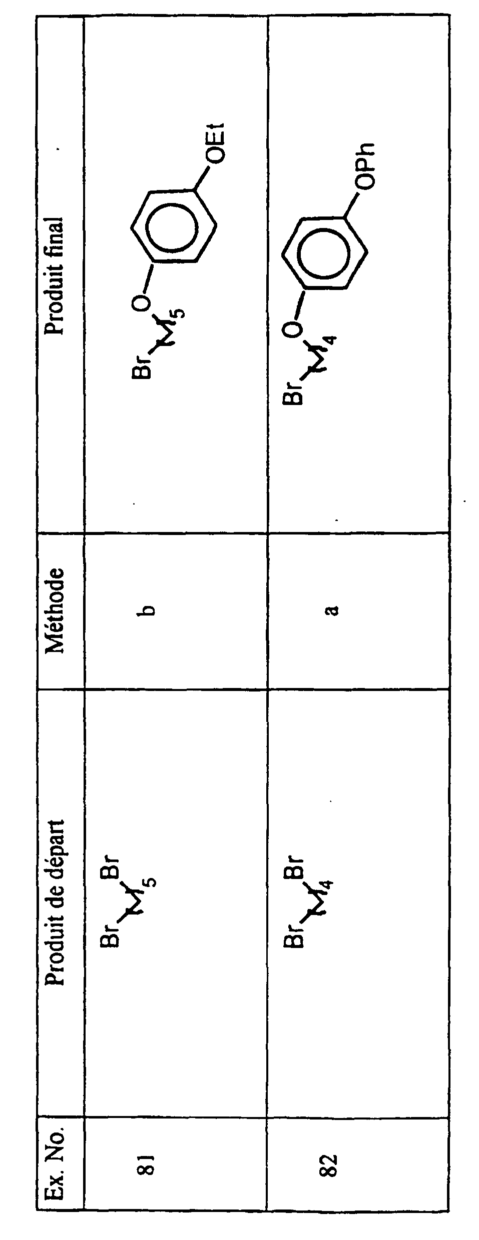

### Exemple 83

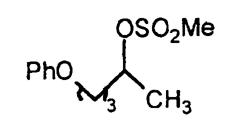

A une solution de 4-phénoxy 2-butanol 4,57 g (25,35 mmol) dans 30 ml de CH₂Cl₂, on ajoute à 0°C 3,07 g (30,29 mmol) de triéthylamine. On additionne goutte à goutte 3,47 g (30,29 mmol) de chlorure de mésyle et on agite 4 heures à température ambiante.

La phase organique est lavée par 10 ml de HCl 1, 2N, séchée sur MgSO₄, filtrée puis concentrée sous vide. On obtient 6,5 g (Rendement 99 %) de résidu huileux.

### Exemple 84

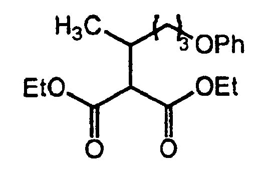

On ajoute successivement dans un ballon sous atmosphère inerte 12,1 g (75,48 mmol) de malonate de diéthyle, 11,46 g (75,48 mmol) de fluorure de césium et 100 ml de DMF anhydre. On agite pendant 1 heure à température ambiante. On ajoute une solution de 6,5 g (25,16 mmol) du mésylate préparé à l'exemple 83 dans 25 ml de DMF anhydre. On chauffe à 60°C pendant 24 heures.

On dilue avec 100 ml d'acétate d'éthyle. On lave avec de l'eau (3 fois avec 30 ml), avec une solution aqueuse saturée de Na-HCO₃. La phase organique est séchée sur MgSO₄, filtrée et concentrée. On obtient 21,4 g de résidu que l'on distille à la pompe à palettes afin d'éliminer l'excès de DMF et de malonate de diéthyle. Le résidu (6,4 g) est chromatographié sur silice (éluant : éther - éther de pétrole 10/90). On obtient 2,82 g (Rendement 35 %) du malonate attendu.

### Exemple 85

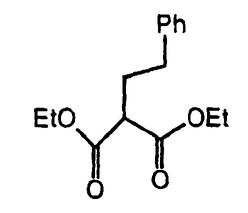

Le produit de l'exemple 85 est préparé à partir de diéthylmalonate et du 2-bromo-éthylphényle selon le même procédé que celui décrit pour l'exemple 7.
Rendement = 85 %, huile jaune.

### Exemple 86

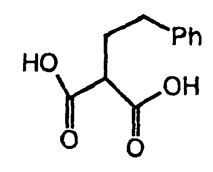

6,02 g (22,78 mmol) du diester malonique de l'exemple 84 sont dilués avec 26 ml d'eau. On ajoute 2,25 g (56,2 mmol) de pastilles de soude.

On agite et on porte au reflux pendant 1 h 30.

On dilue avec de l'eau et on lave avec de l'Et₂O (1 fois 15 ml).

La phase aqueuse est refroidie et acidifiée par une solution aqueuse d'acide chlorhydrique concentré jusqu'à pH = 1.

On extrait à l'Et₂O (2 fois 25 ml). Les phases éthérées sont réunies, séchées sur Mg SO₄ filtrées et concentrées.

On obtient 4,1 g (86 %) de solide blanc.

### Exemple 87

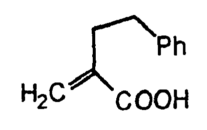

A une solution de 4,1 g (19,7 mmol) du diacide malonique de l'exemple 86 dans 20 ml d'AcOEt, on ajoute 1,44 g (19,7 mmol) de diéthylamine puis 0,99 g (33 mmol) de paraformaldéhyde.

On porte au reflux pendant 30 minutes.

La solution est ensuite refroidie à l'aide d'un bain de glace et d'eau, diluée avec 10 ml d'eau puis acidifiée avec une solution d'HCl 3N jusqu'à pH = 1. La phase aqueuse est éliminée. La phase organique est lavée avec de l'eau (une fois 10 ml), séchée sur Mg SO₄, filtrée et concentrée.

On obtient 3,23 g (93 %) de solide blanc.

### Exemple 88

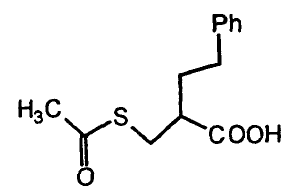

L'acide acrylique de l'exemple 87 est chauffé à 70°C pendant une nuit avec 2,23 g (29,34 mmol) d'acide thioacétique.

La solution est diluée avec 10 ml d'Et₂O puis concentrée sous vide.

On obtient ainsi 4,6 g (99 %) d'acide désiré.

Les exemples 89 à 145 sont préparés selon la même suite réactionnelle que celle décrite pour l'exemple 88 :

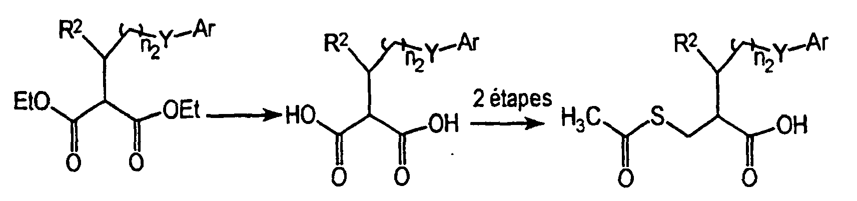

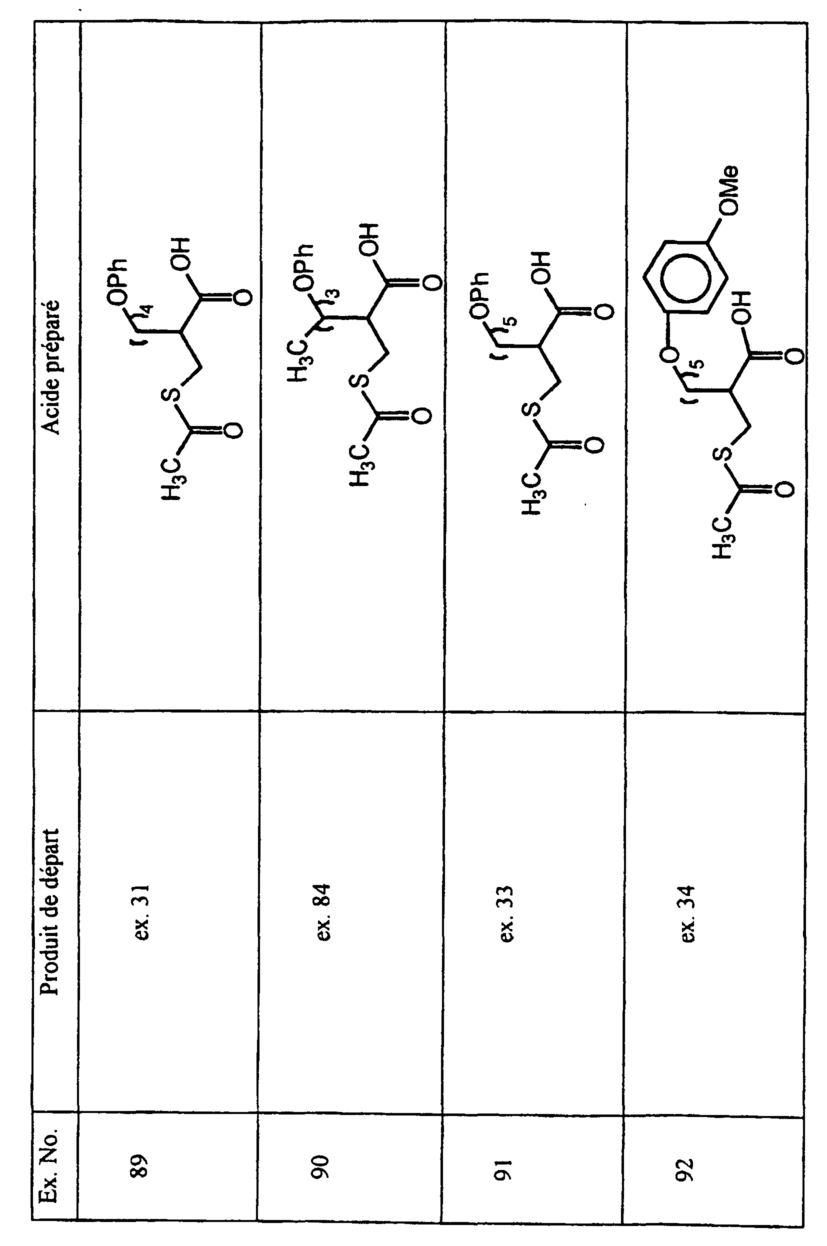

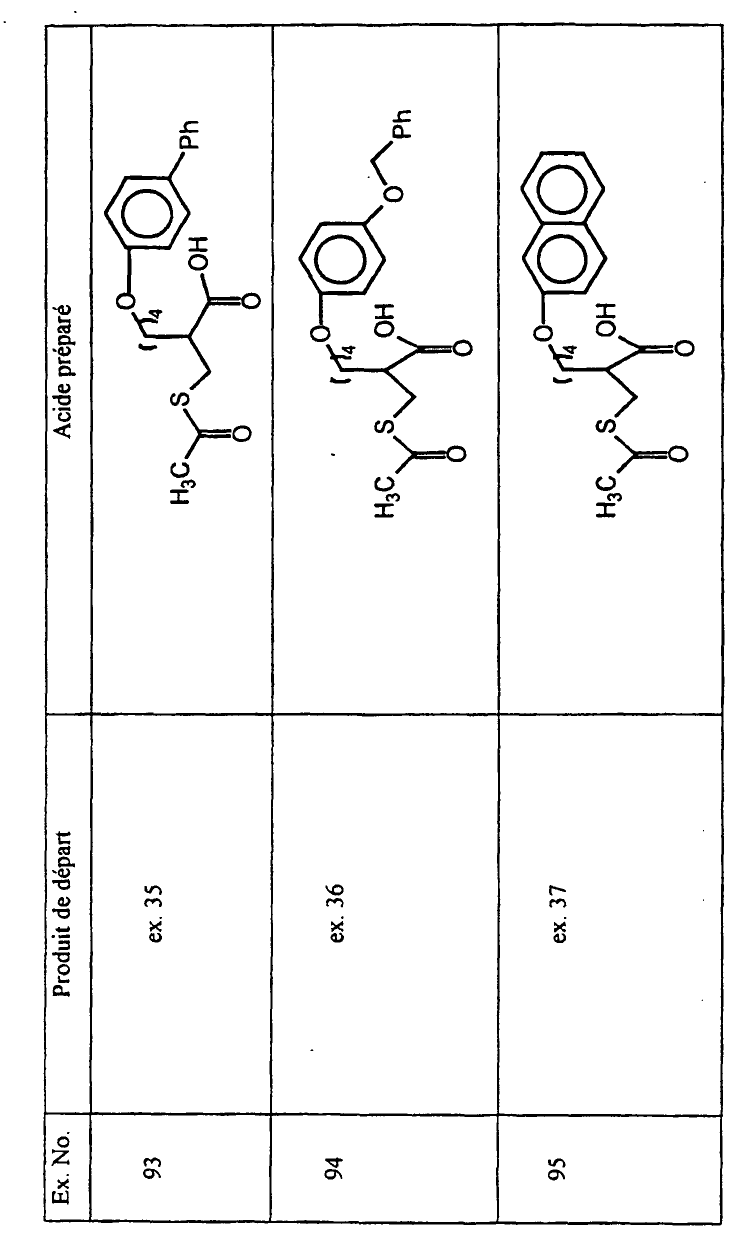

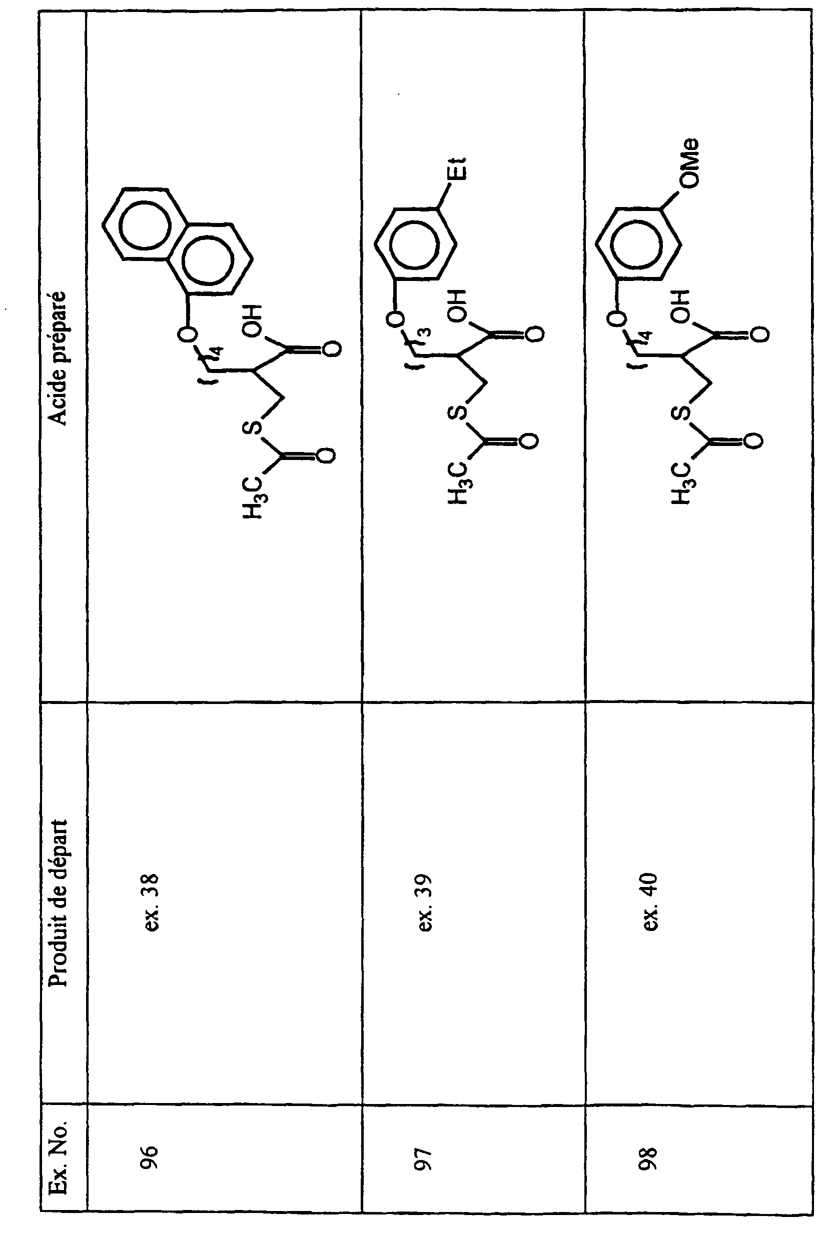

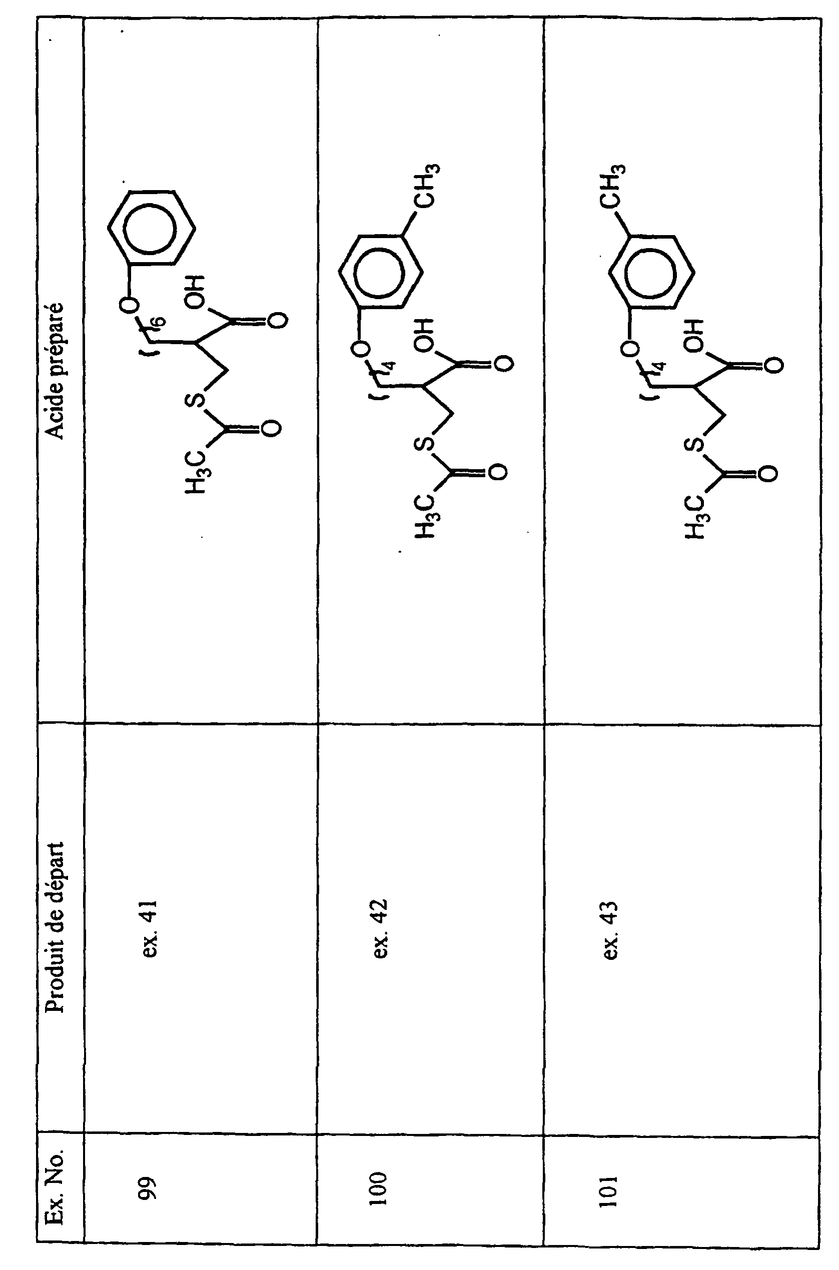

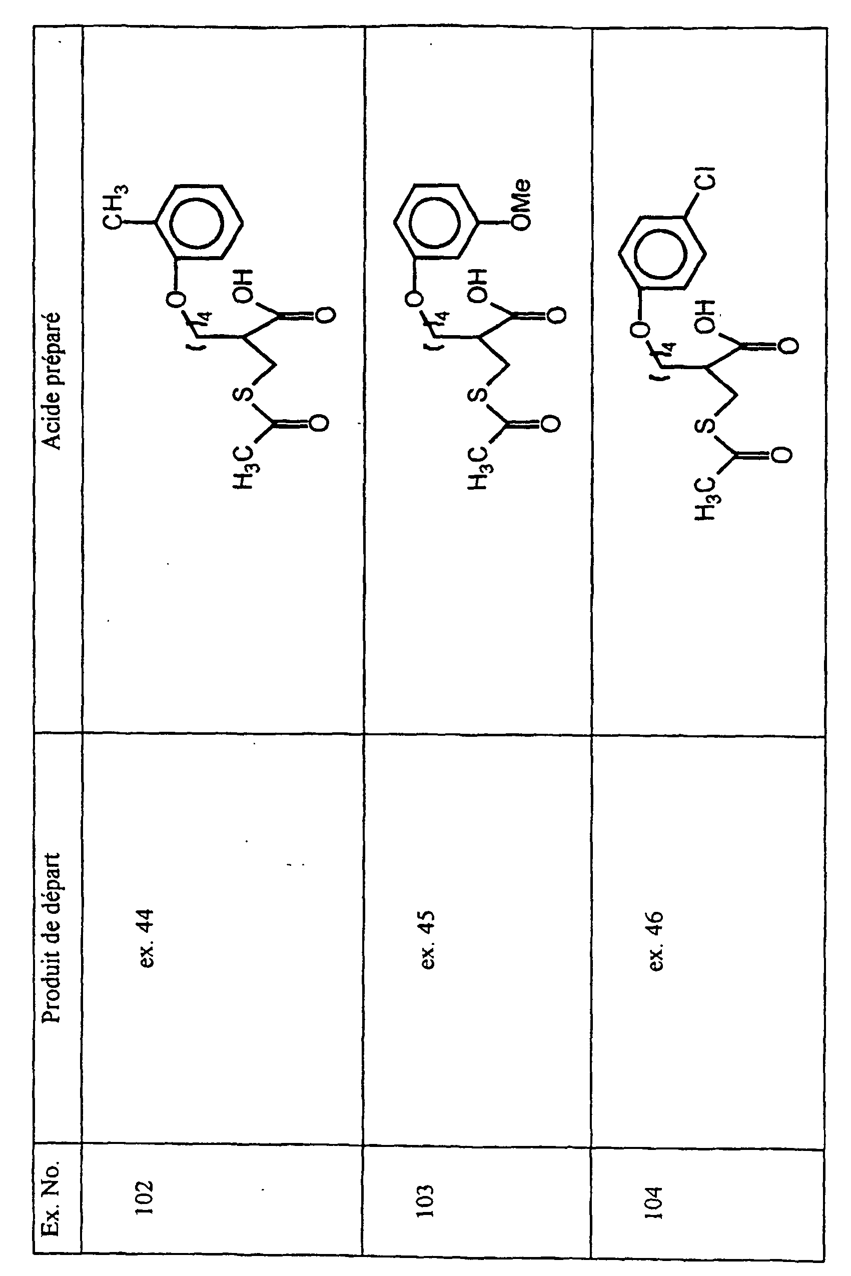

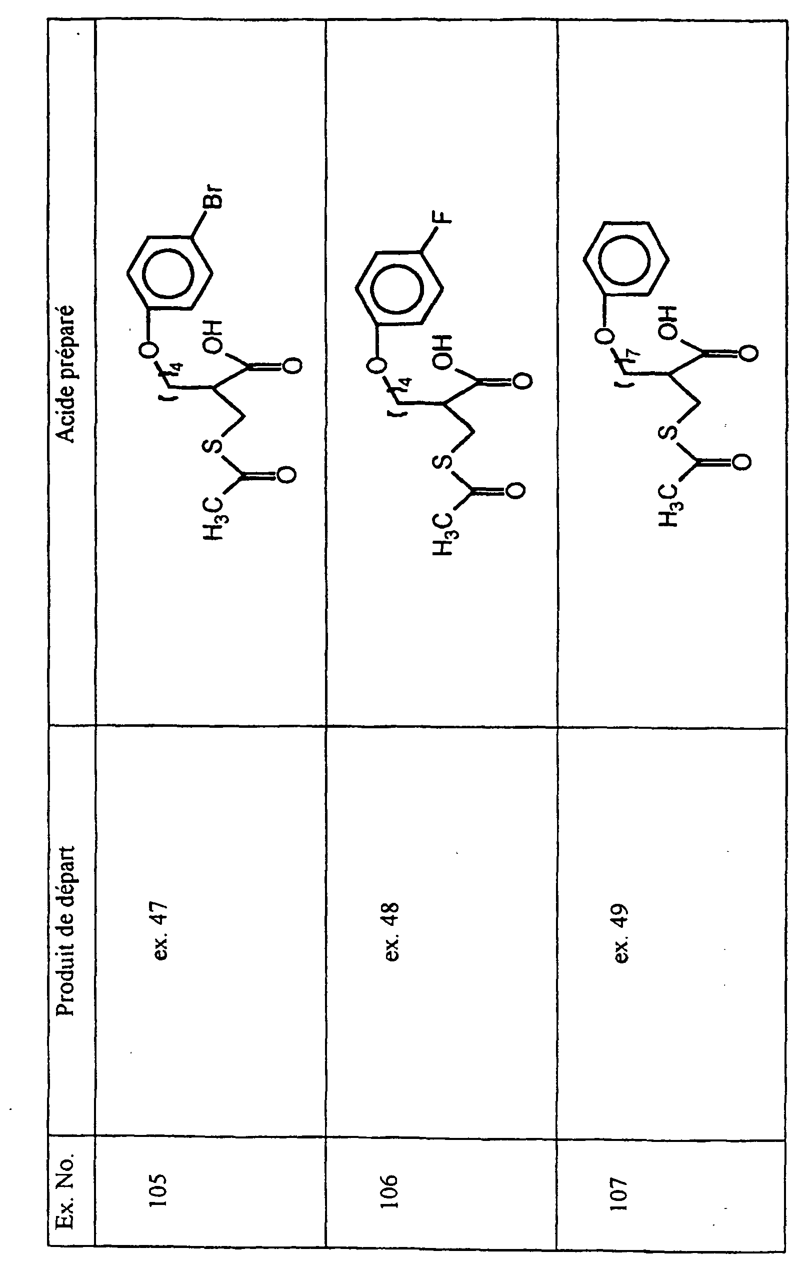

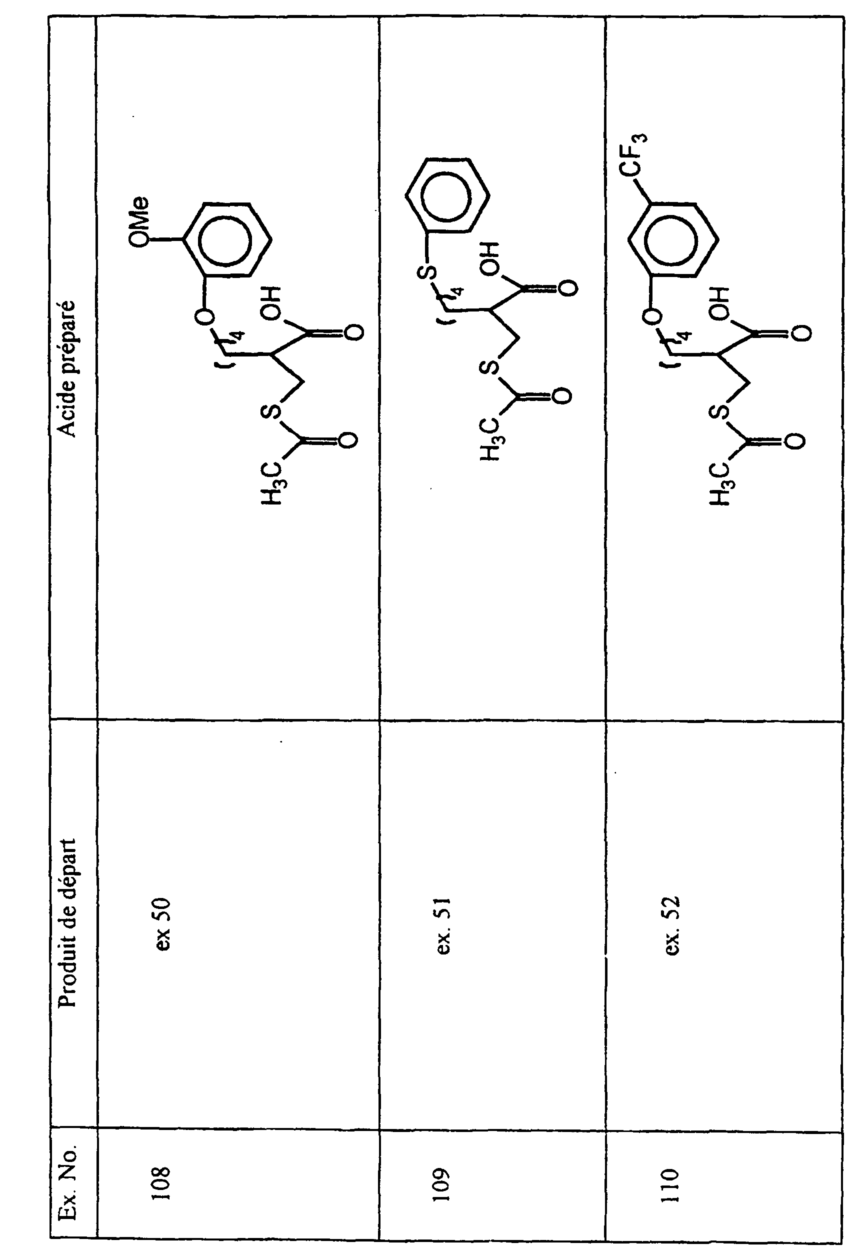

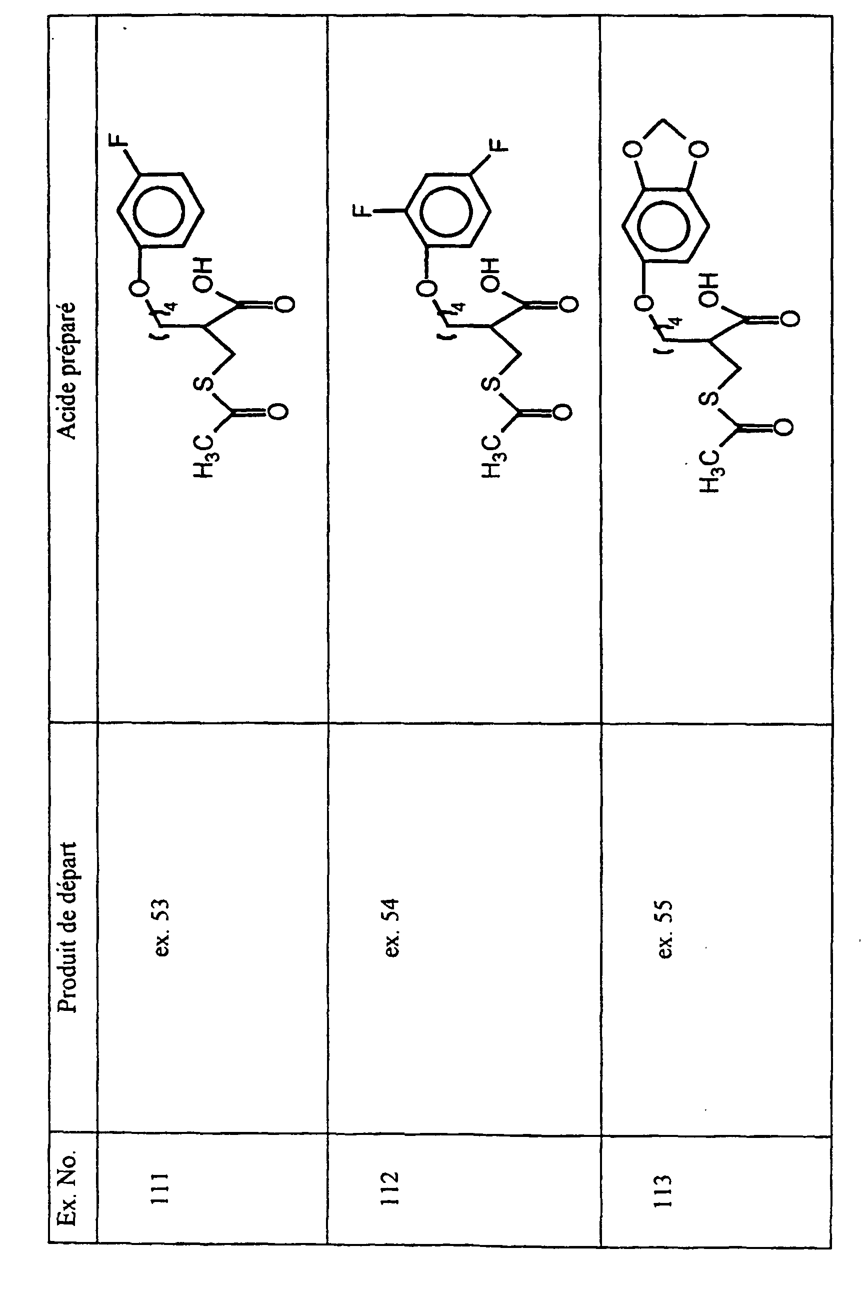

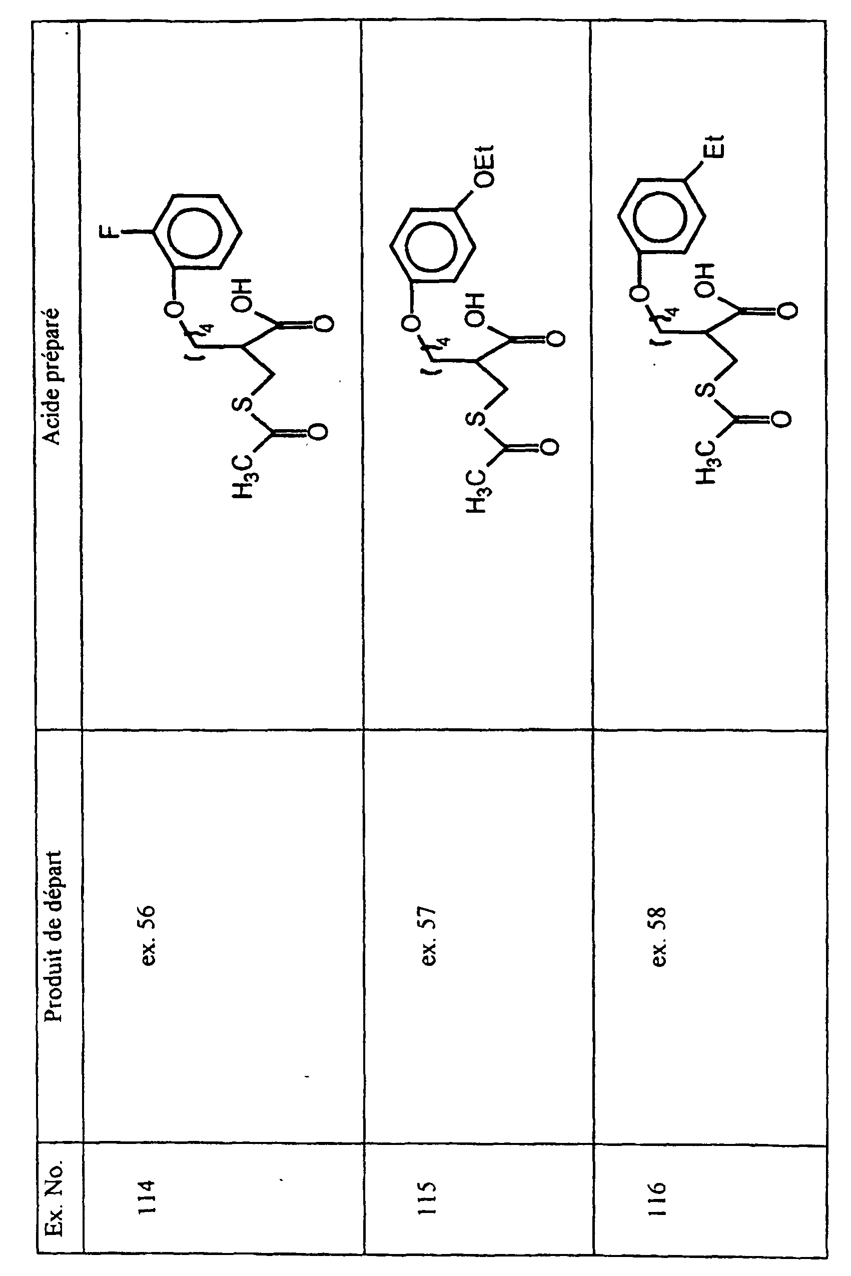

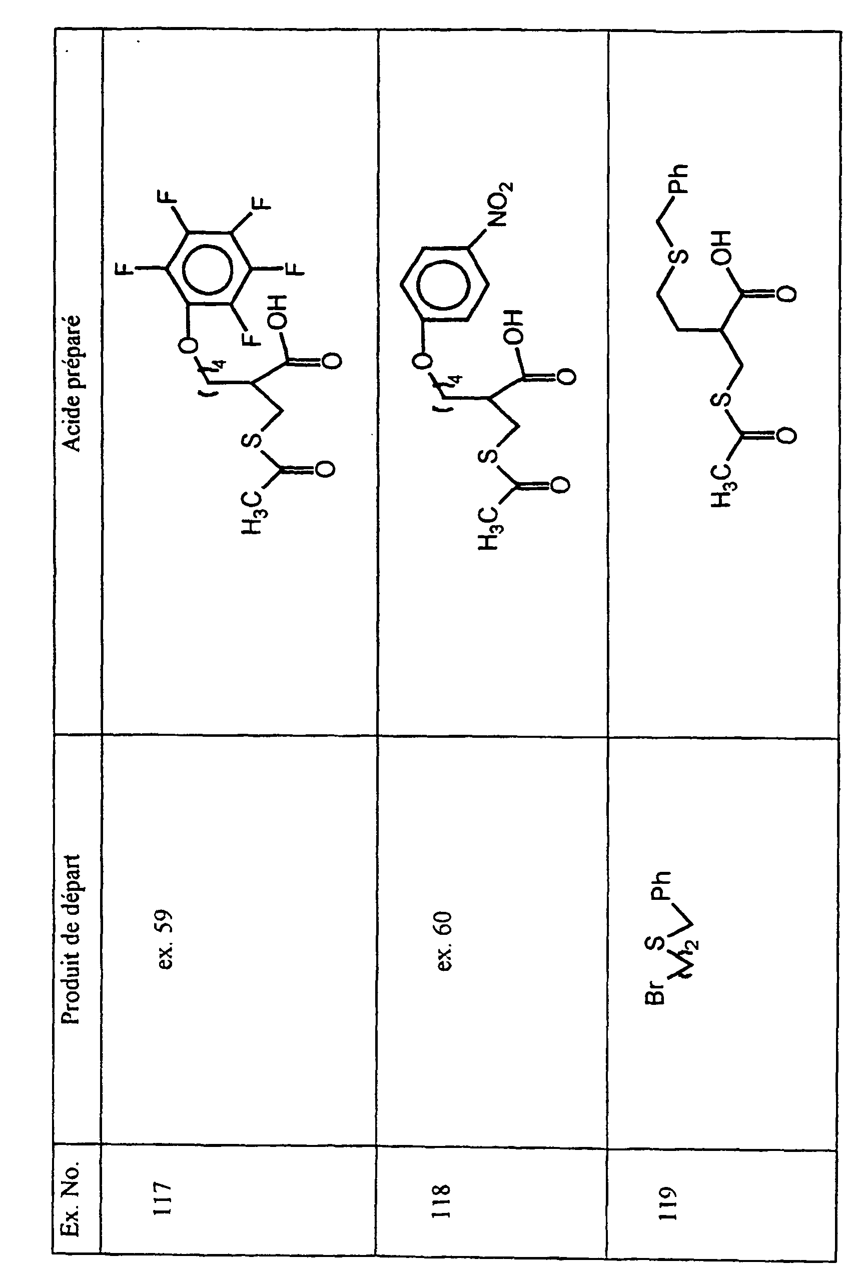

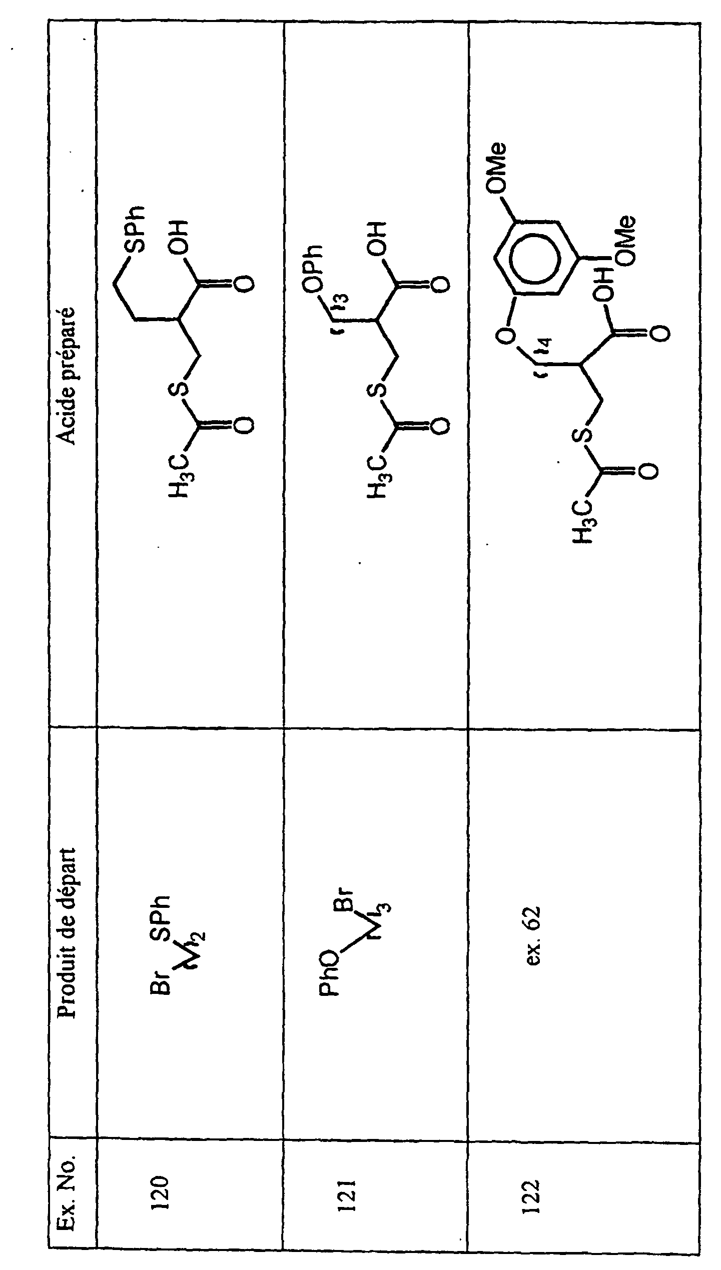

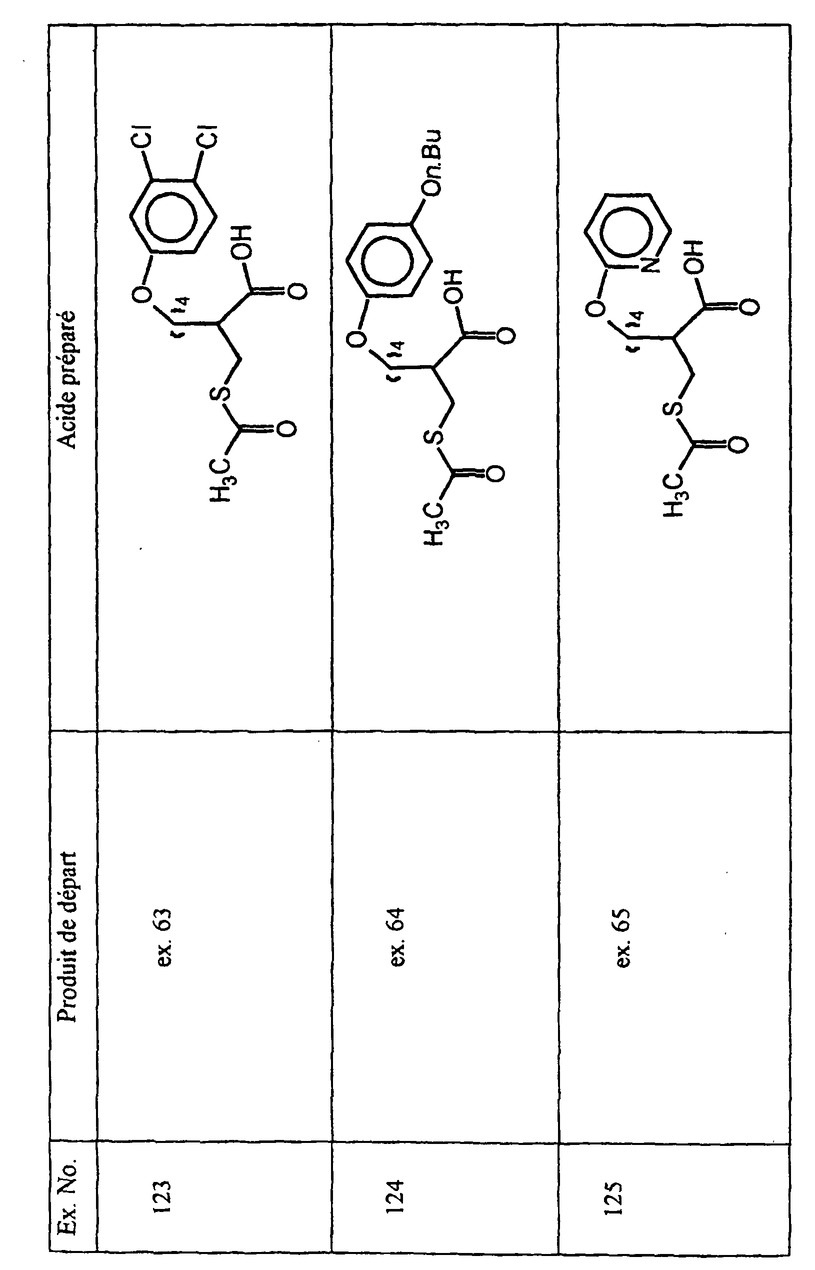

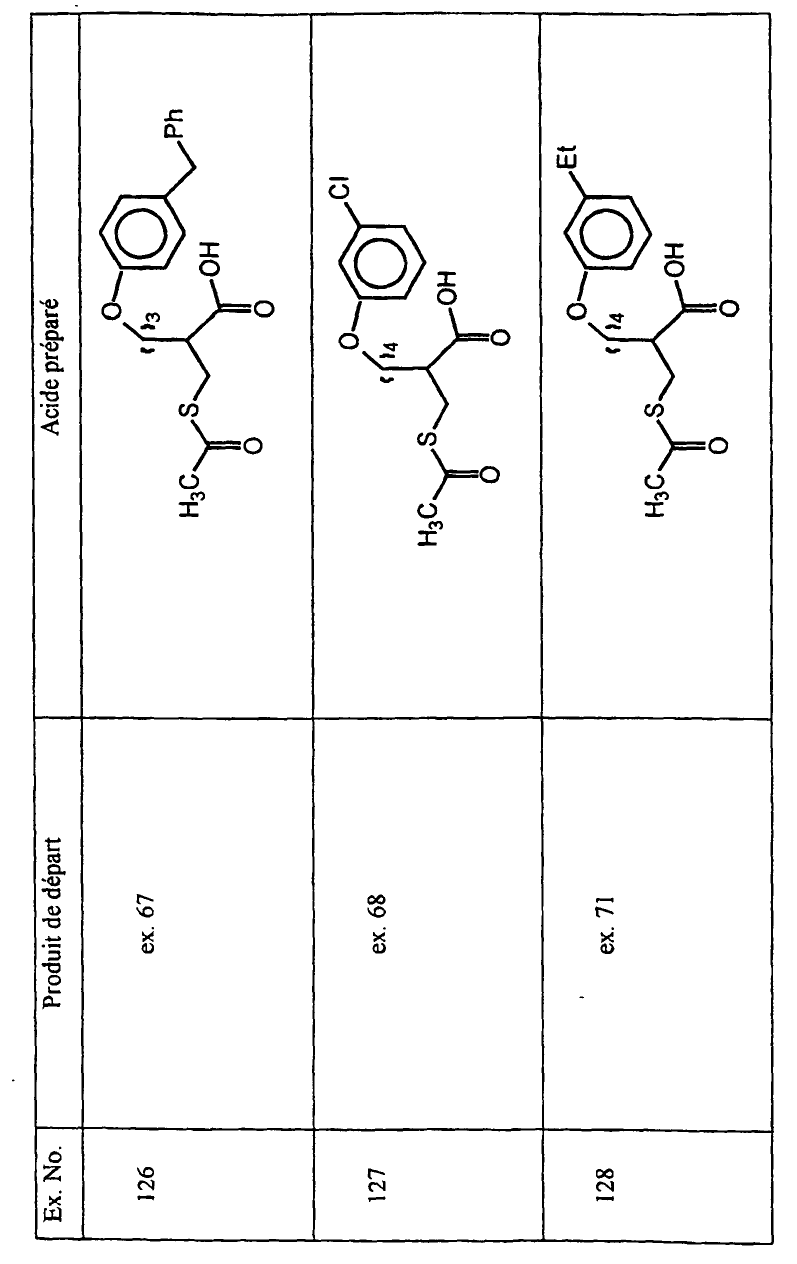

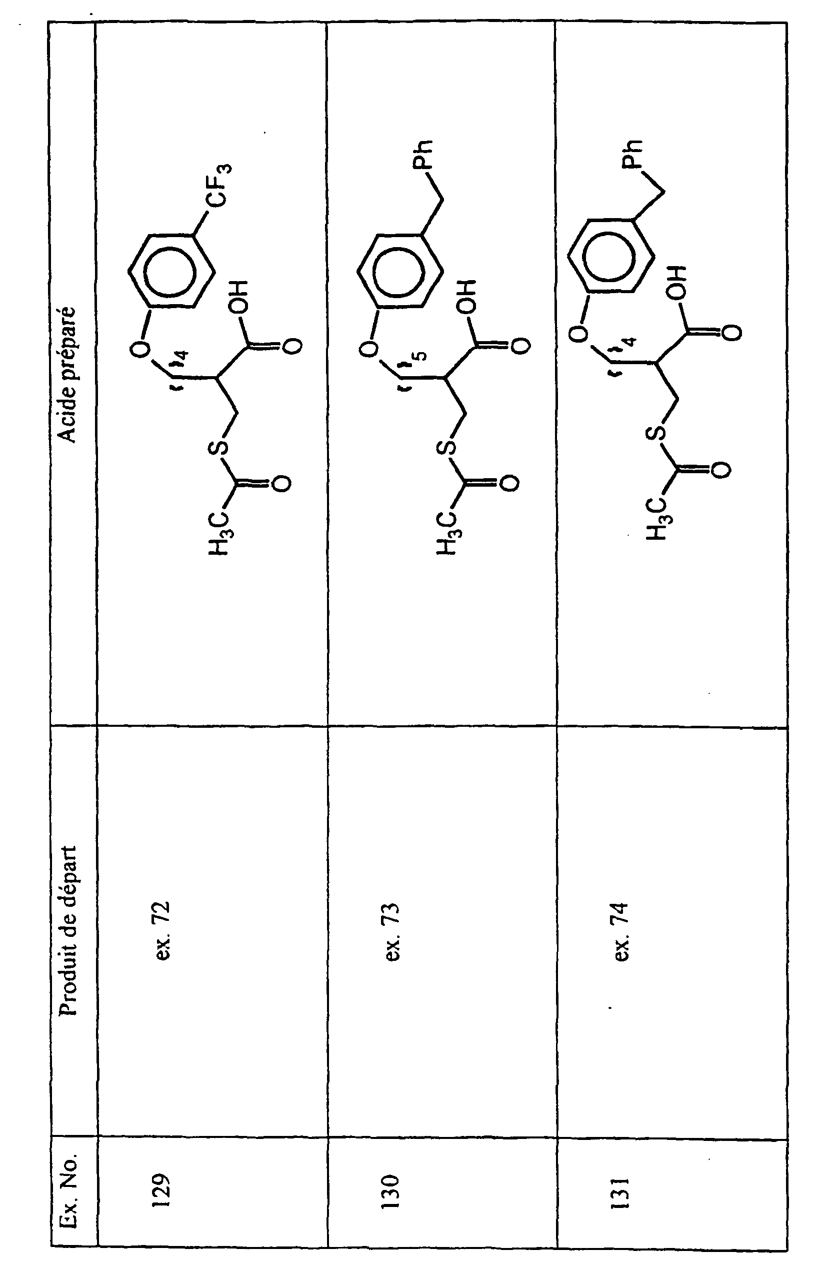

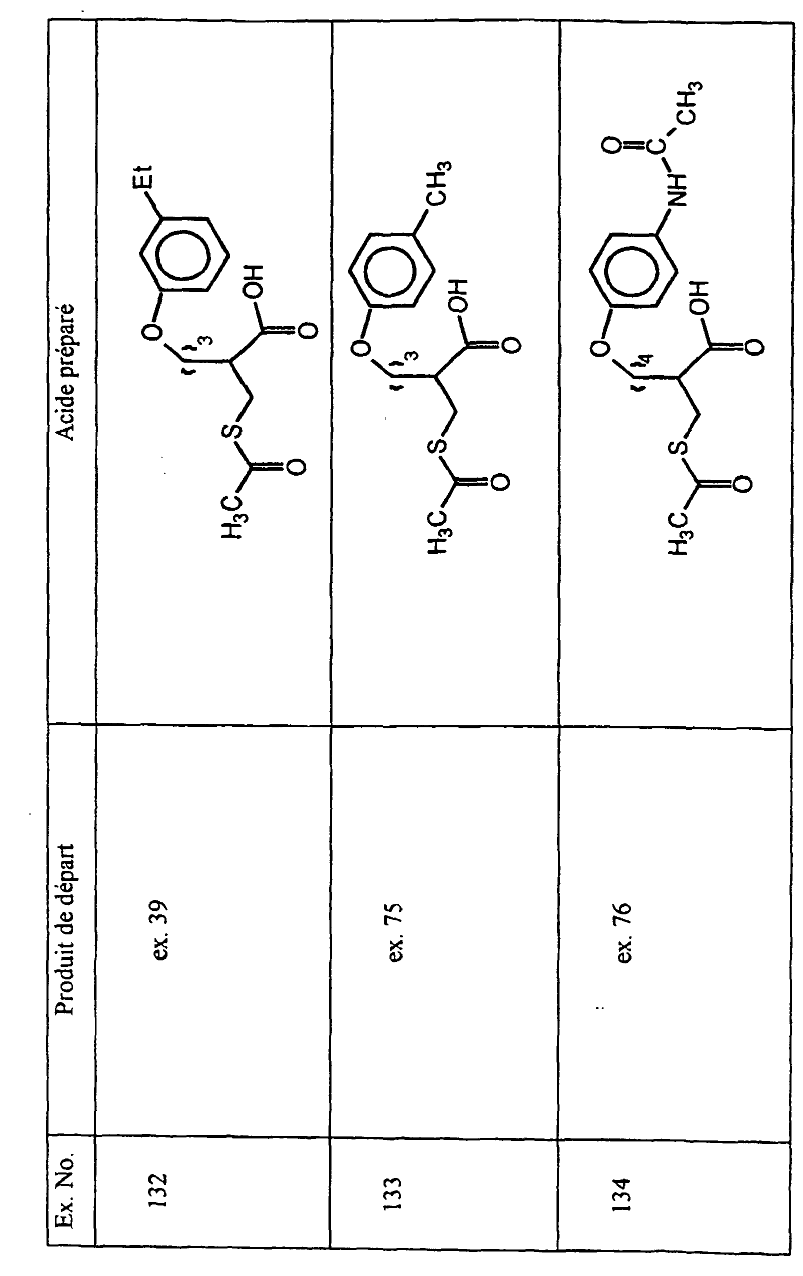

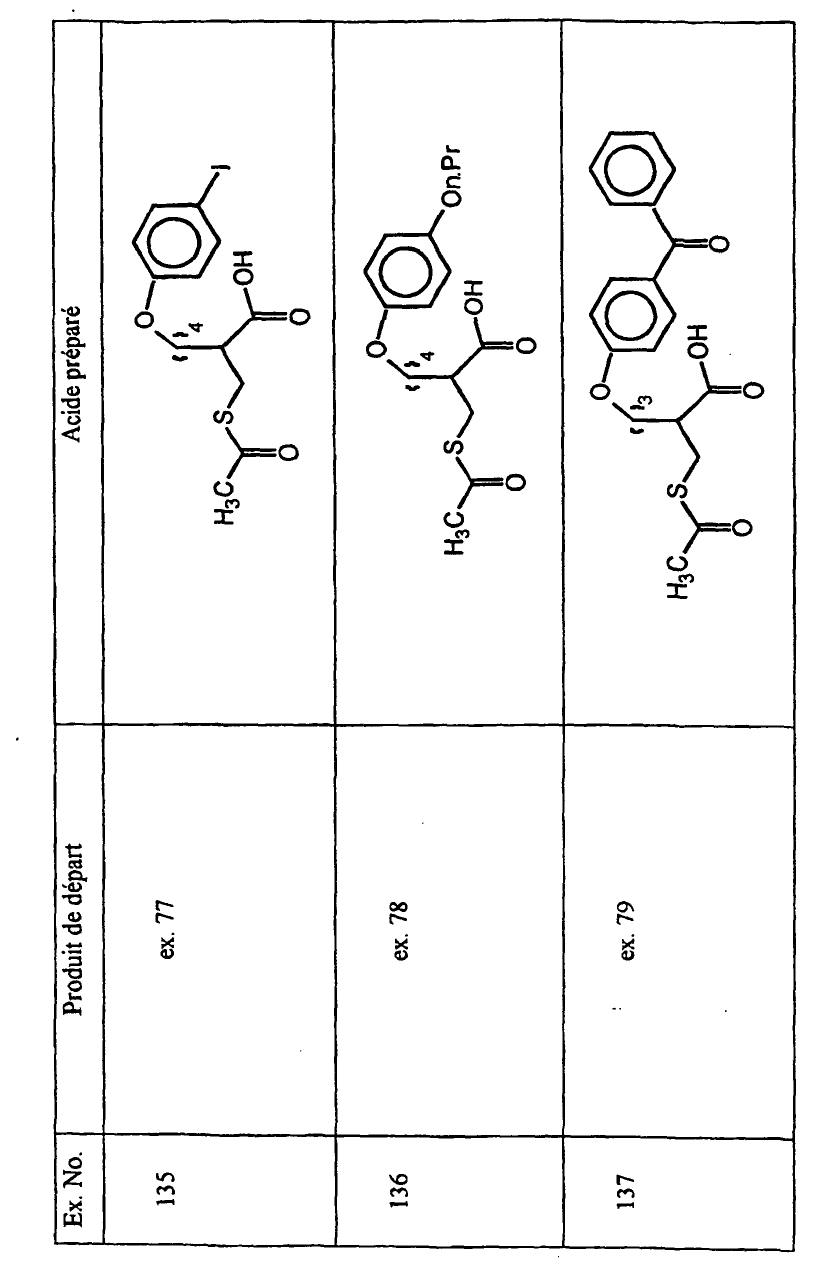

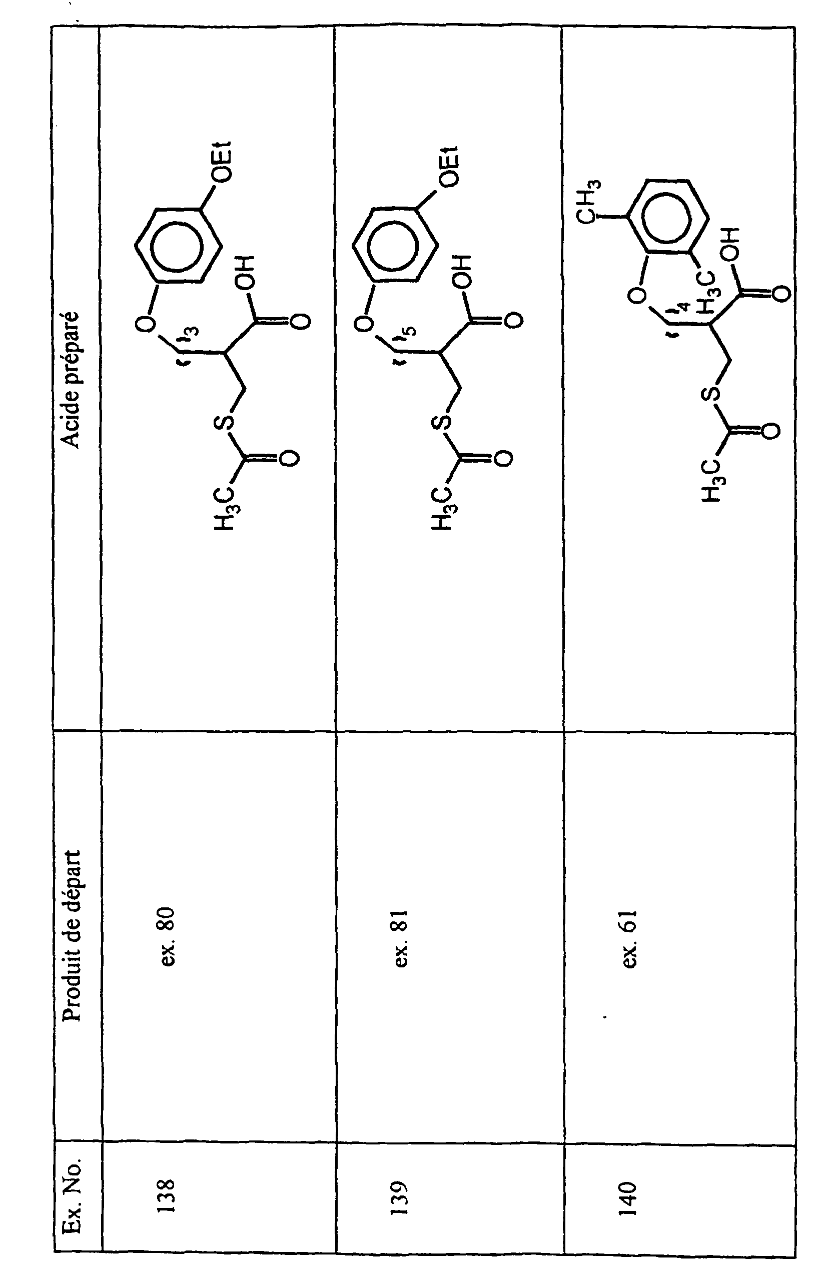

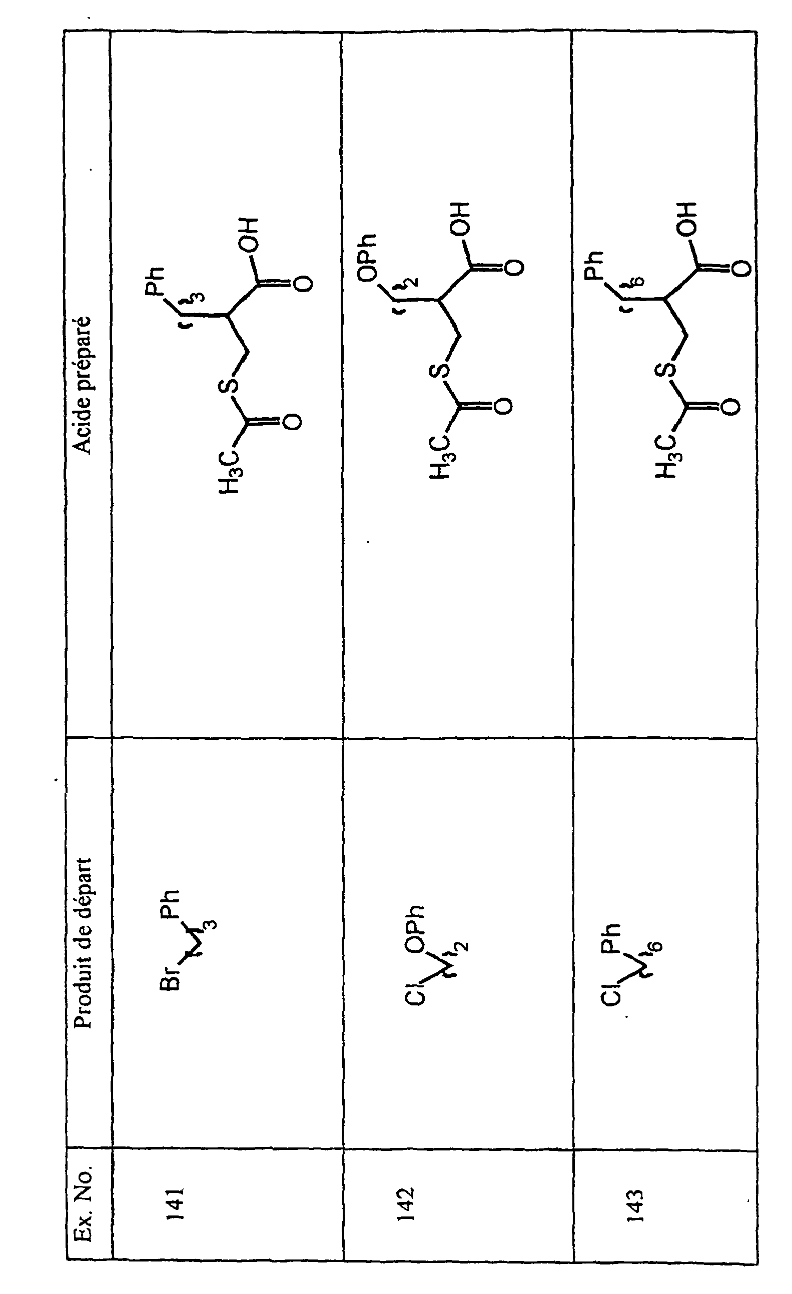

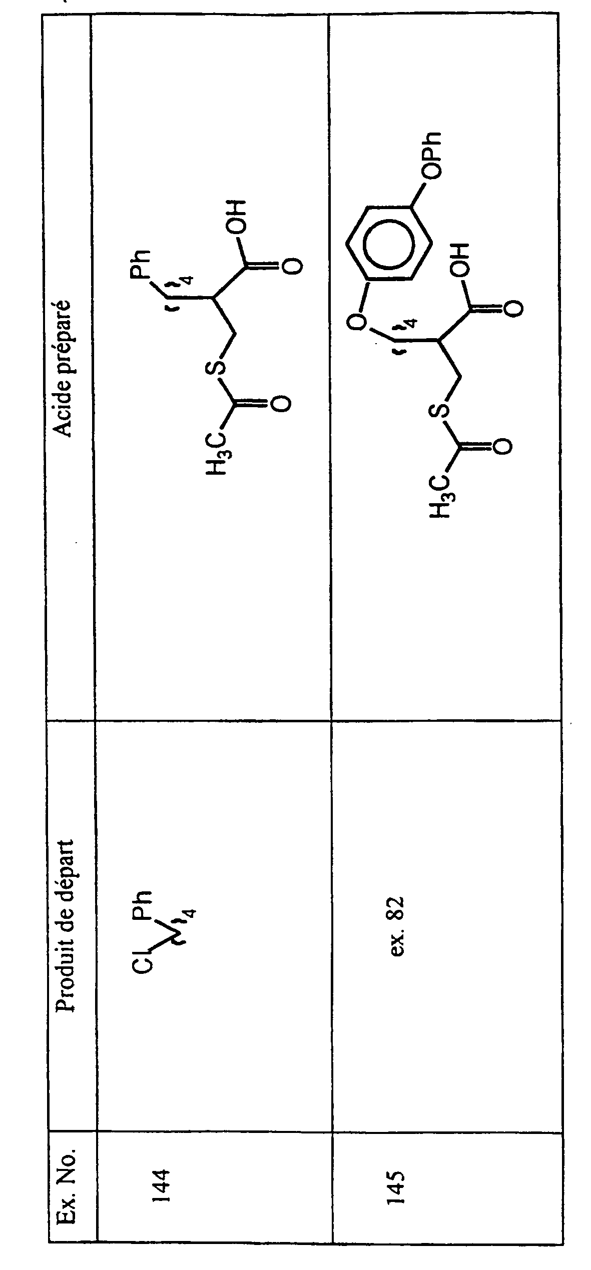

### Exemple 146

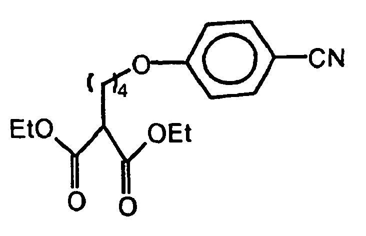

Le diester malonique de l'exemple 146 est préparé à partir de diéthylmalonate et du dérivé bromé de l'exemple 32 selon le même procédé que celui décrit pour l'exemple 7.
Rendement : 81 %, solide jaune.

### Exemple 147

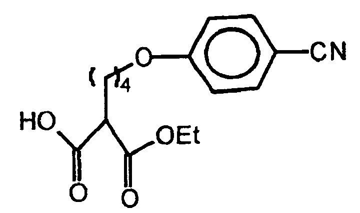

Le diester de l'exemple 146 est monosaponifié selon le même procédé que celui décrit pour l'exemple 8.
Rendement : 90 %, huile visqueuse jaune.

### Exemple 148

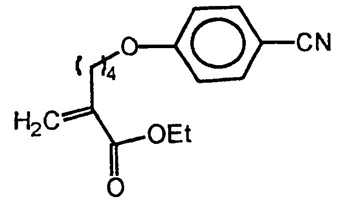

On introduit dans un ballon 8,45 g (27,70 mmol) d'acide de l'exemple 147 en solution dans 50 ml de CH₂Cl₂ que l'on refroidit à l'aide d'un bain de glace-eau.

On ajoute 2,02 g (27,67 mmol) de diéthylamine et 2,8 ml d'une solution aqueuse à 37 % de formaldéhyde.

On laisse la température remonter à l'ambiante puis on agite pendant une nuit.

La phase organique est lavée par une solution d'HCl N (2 fois 20 ml), séchée sur MgSO₄, filtrée et concentrée.

On obtient 7,26 (76 %) de solide jaune.

### Exemple 149

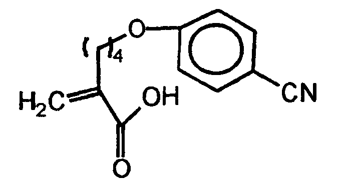

7,2 g (26,37 mmol) de l'ester de l'exemple 148 sont dilués avec 29 ml d'acétone et 9 ml d'eau. On refroidit à environ 5°C et ajoute 32 ml de NaOH N. On laisse revenir à température ambiante puis on porte au reflux pendant 6 heures.

On concentre sous vide et on lave la phase aqueuse résiduelle à l'éther éthylique (2 fois 20 ml).

La phase aqueuse est refroidie puis acidifiée jusqu'à pH = 1 avec une solution d'HCl 3N. Le précipité obtenu est filtré, lavé avec de l'eau (1 fois 20 ml) et séché sous vide sur P₂O₅ jusqu'à une masse constante.

On obtient 4,05 g (63 %) de solide blanc.

### Exemple 150

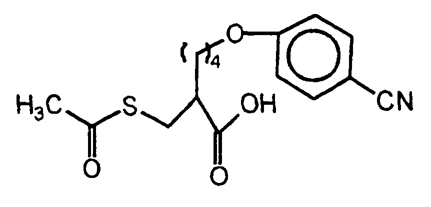

On chauffe à 70°C pendant une nuit 4,05 g (16,59 mmol) de l'acide de l'exemple 149 et 2,02 g (26,55 mmol) d'acide thioacétique.

On dilue avec 20 ml d'éther éthylique puis on concentre sous vide. Le résidu est chromatographié sur silice (éluant 60/40 éther éthylique/éther de pétrole).

On obtient 2,04 g (38 %) du produit désiré.

Les exemples 151 à 153 sont préparés selon la même suite réactionnelle que celle décrite pour l'exemple 150.

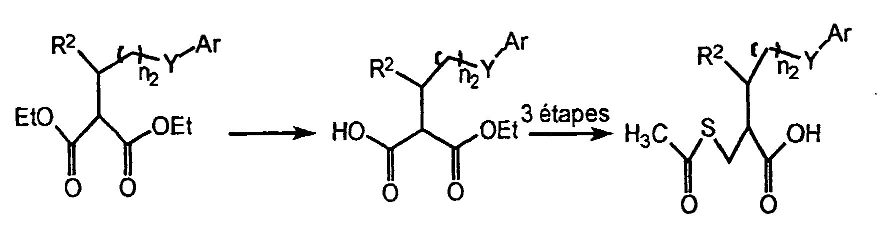

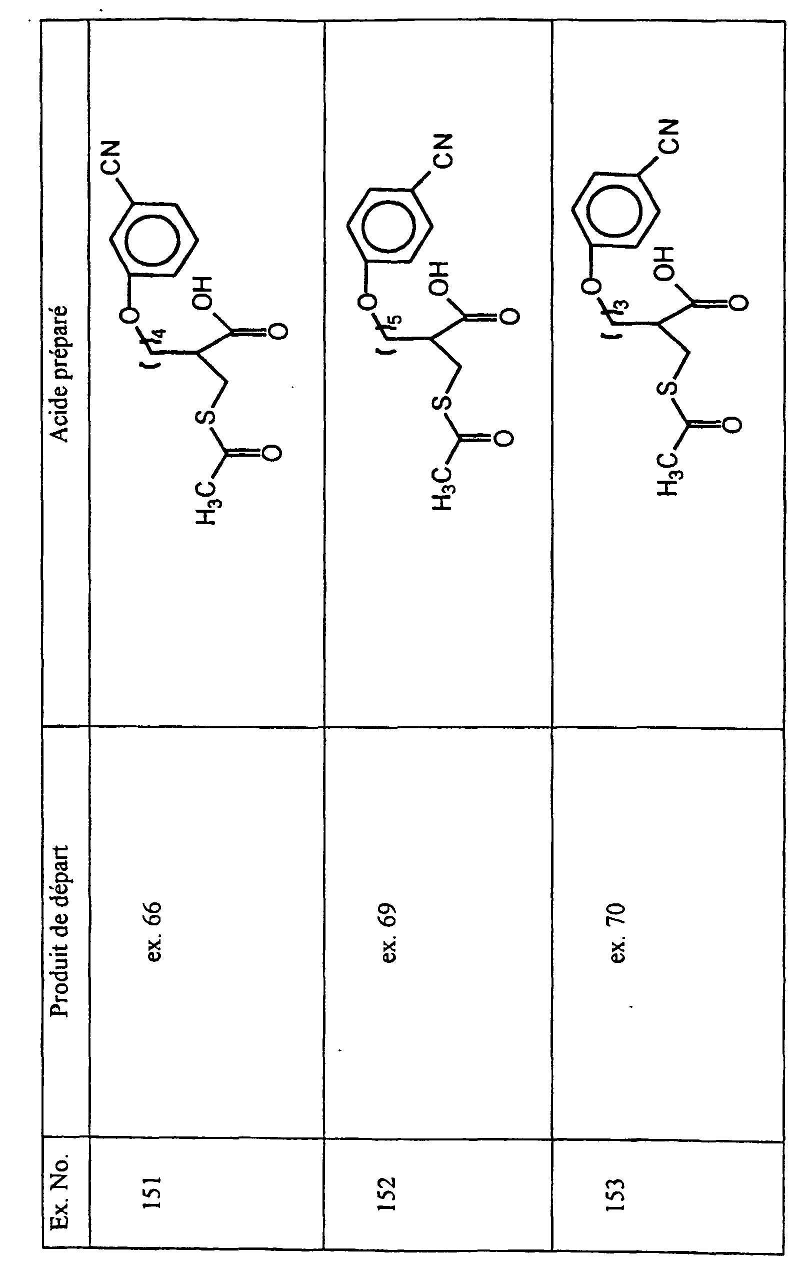

### Exemple 154

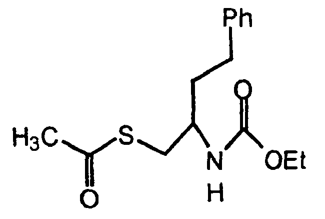

A 2,5 g (10 mmol) de l'acide de l'exemple 88 en solution dans 10 ml de toluène, on ajoute goutte à goutte 2,9 g (10,53 mmol) de DPPA et 1,06 g (10,47 mmol) de triéthylamine. On porte le mélange à 80°C pendant 1 heure. Après retour à température ambiante, on ajoute 0,73 ml (12,5 mmol) d'éthanol et on chauffe à 80°C pendant une nuit.

On dilue avec 20 ml de toluène et on lave successivement avec de l'eau (1 fois 10 ml), une solution aqueuse saturée de NaHCO₃ (1 fois 10 ml) et à l'eau (1 fois 10 ml).

La phase organique est séchée sur MgSO₄, filtrée et concentrée. On obtient 3,1 g de résidu huileux que l'on chromatographie sur silice (éluant : Ether - éther de pétrole 3/7). On obtient 1,5 g (Rendement 50 %) d'un solide blanc.

### Exemple 155

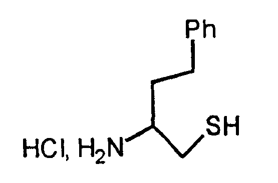

A 0,92 g (3,1 mmol) du produit de l'exemple 154 sous atmosphère inerte, on ajoute 3 ml d'une solution aqueuse de soude 10N et 0,3 ml d'EtOH.

On porte au reflux pendant 1 heure 30 minutes.

On refroidit à 0°C et acidifie goutte à goutte jusqu'à pH = 1 avec une solution d'HCl 1N. On lave à l'éther (2 fois 10 ml) et on évapore à sec la phase aqueuse. Le résidu solide est séché sous vide sur P₂O₅ puis extrait par du CHCl₃ au reflux (2 fois 20 ml).

Les phases organiques sont réunies puis concentrées sous vide. Le résidu est ensuite trituré avec 10 ml d'éther anhydre. On filtre, on lave le précipité à l'éther (10 ml). Après séchage sous vide, on obtient 0,37 g (Rendement : 50 %) du produit désiré.
RMN ¹H (200 MHz, D₂O) : 7,4 à 7,0 (m, 5H) ; 4,65 (s, 4H) ; 3,4 à 3,15 (m, 1H) ; 3,0 à 2,5 (m, 4H) ; 2,05 à 1,7 (m, 2H).

Les exemples 156 à 180 sont préparés selon le même procédé que celui décrit pour l'exemple 155.

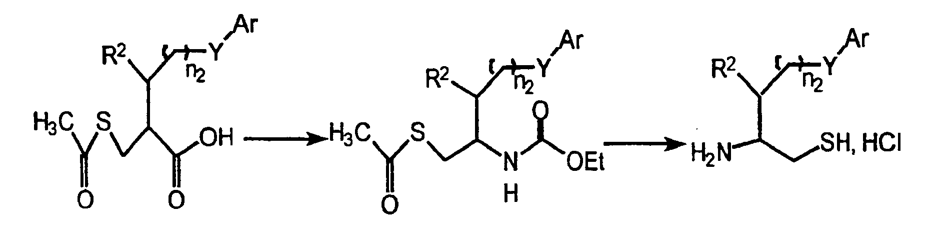

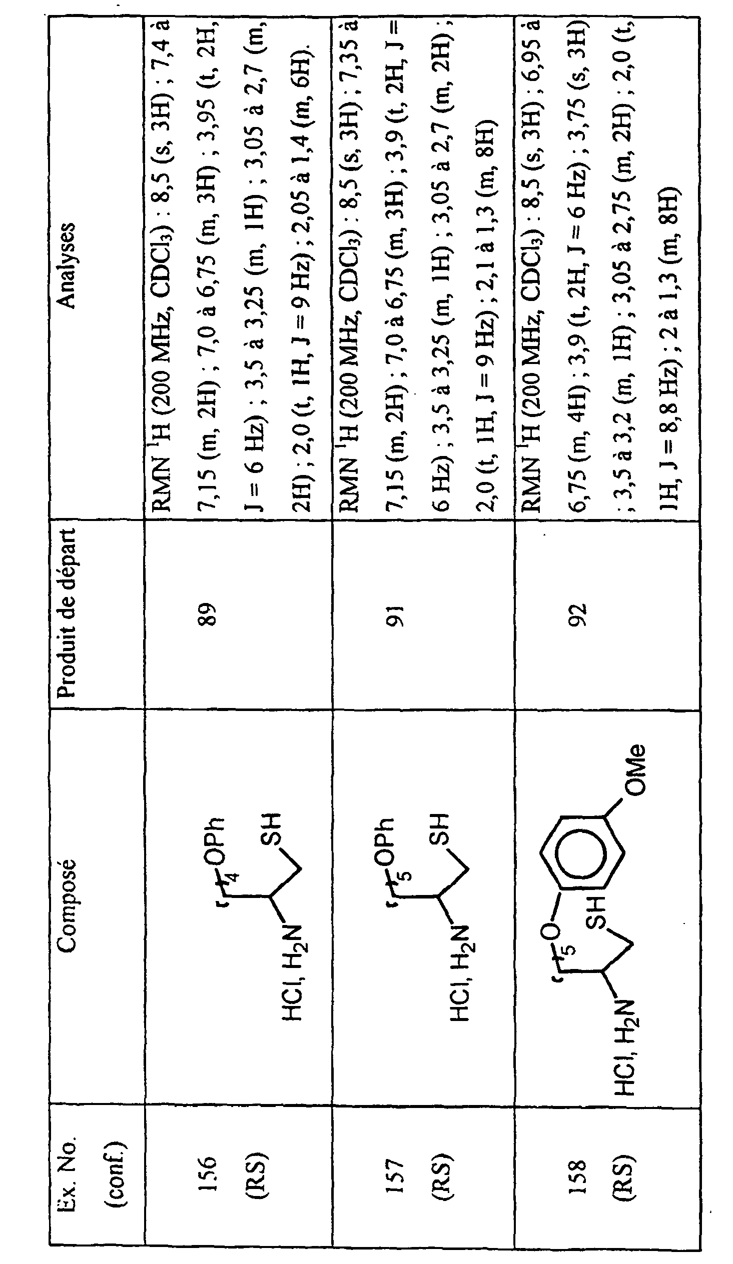

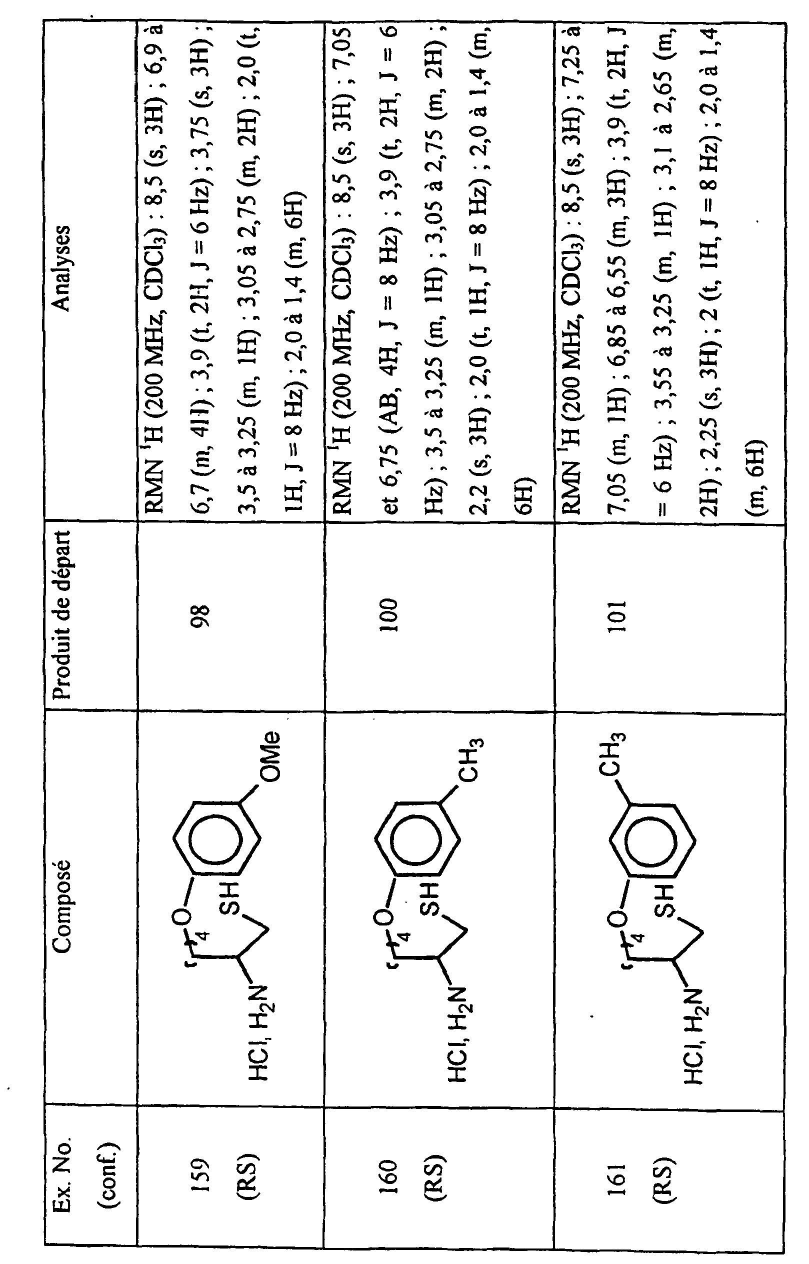

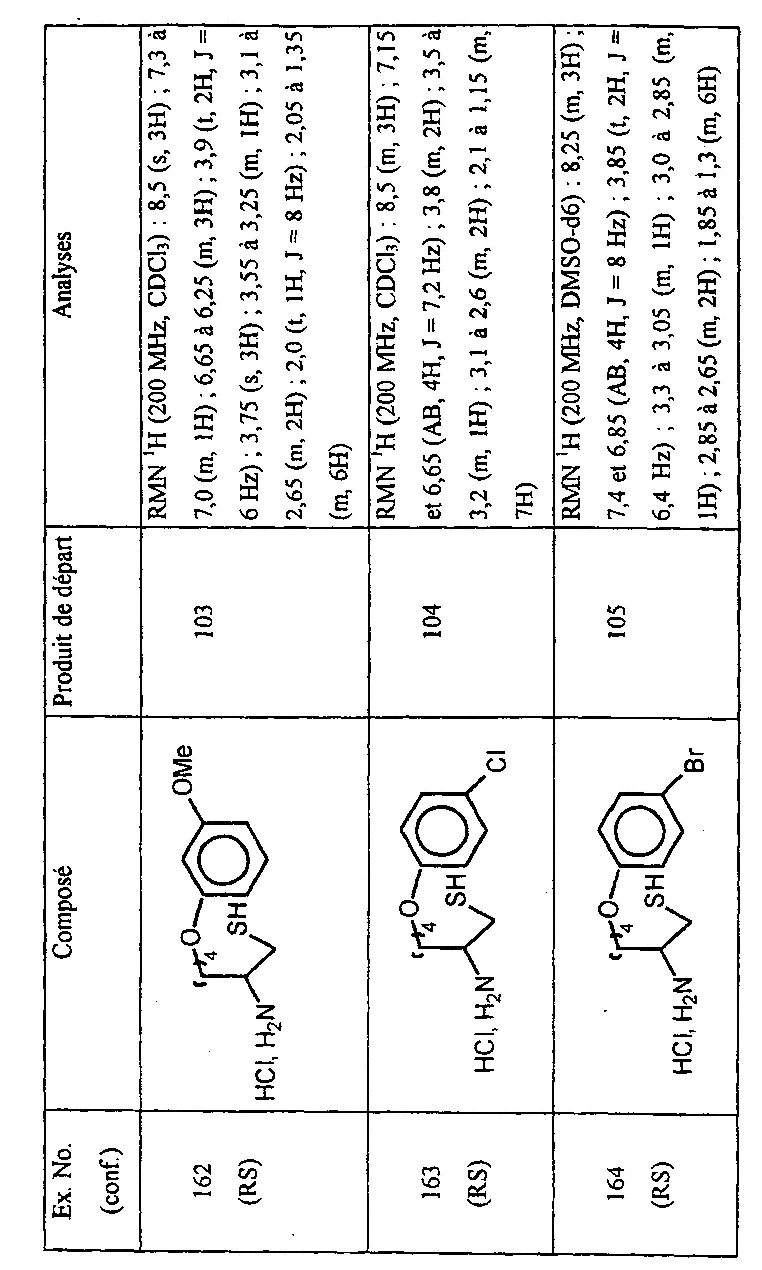

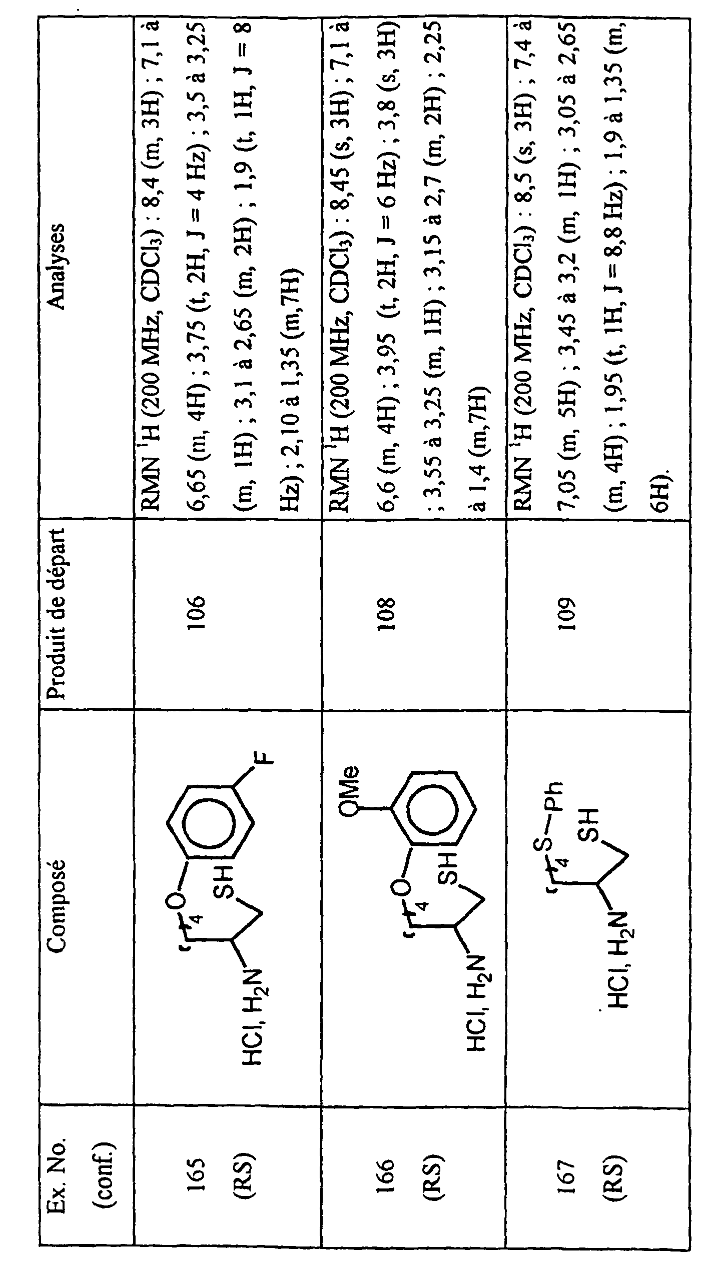

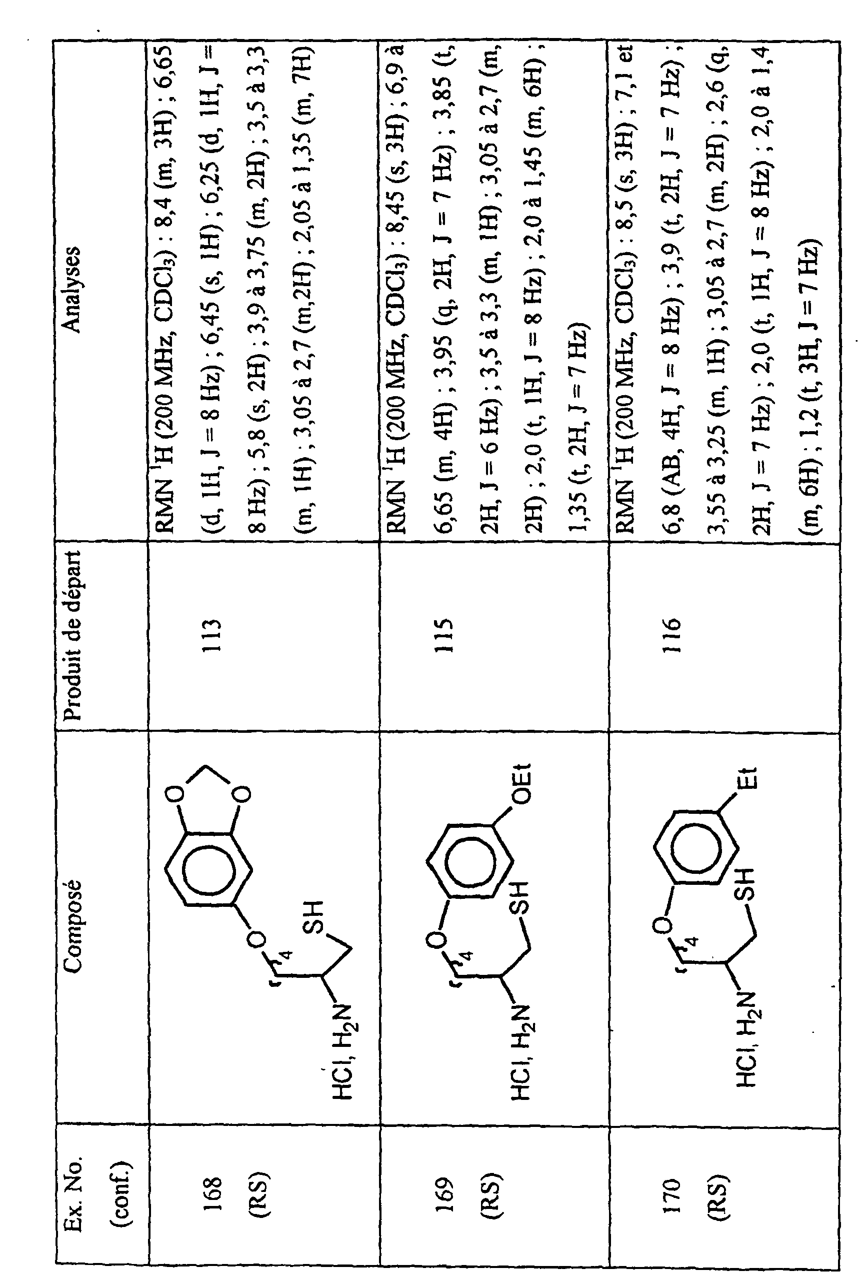

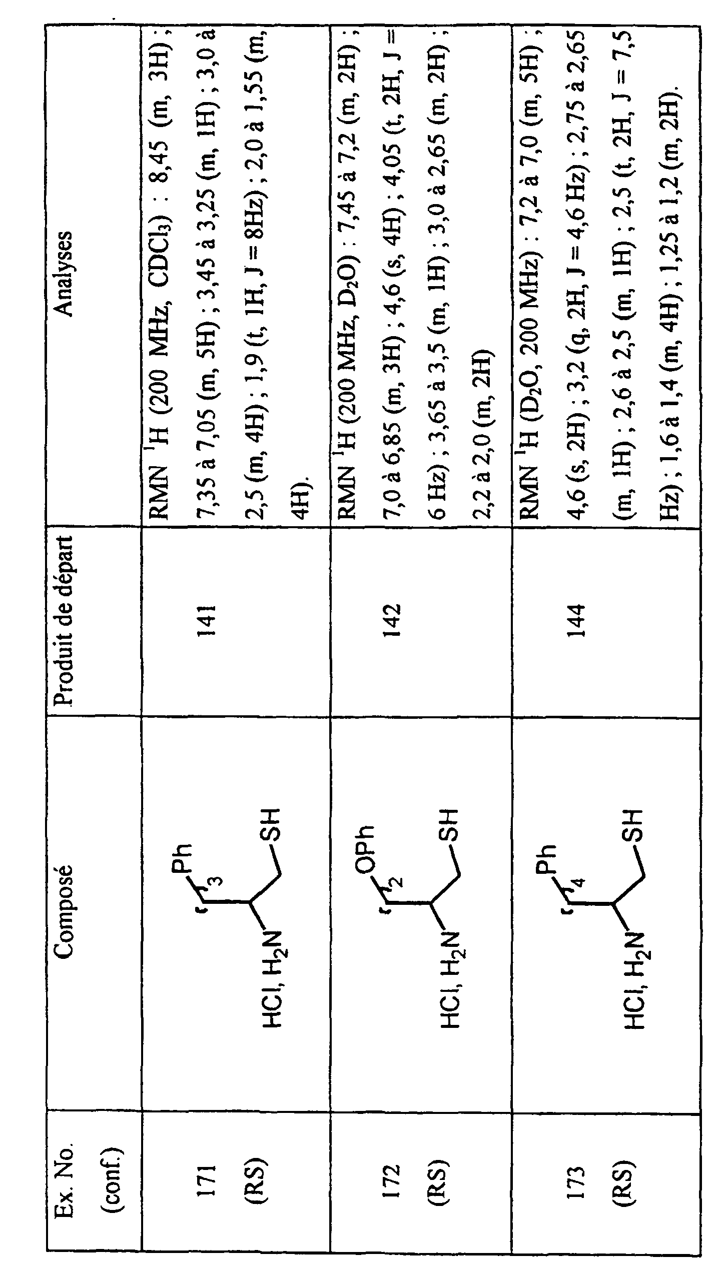

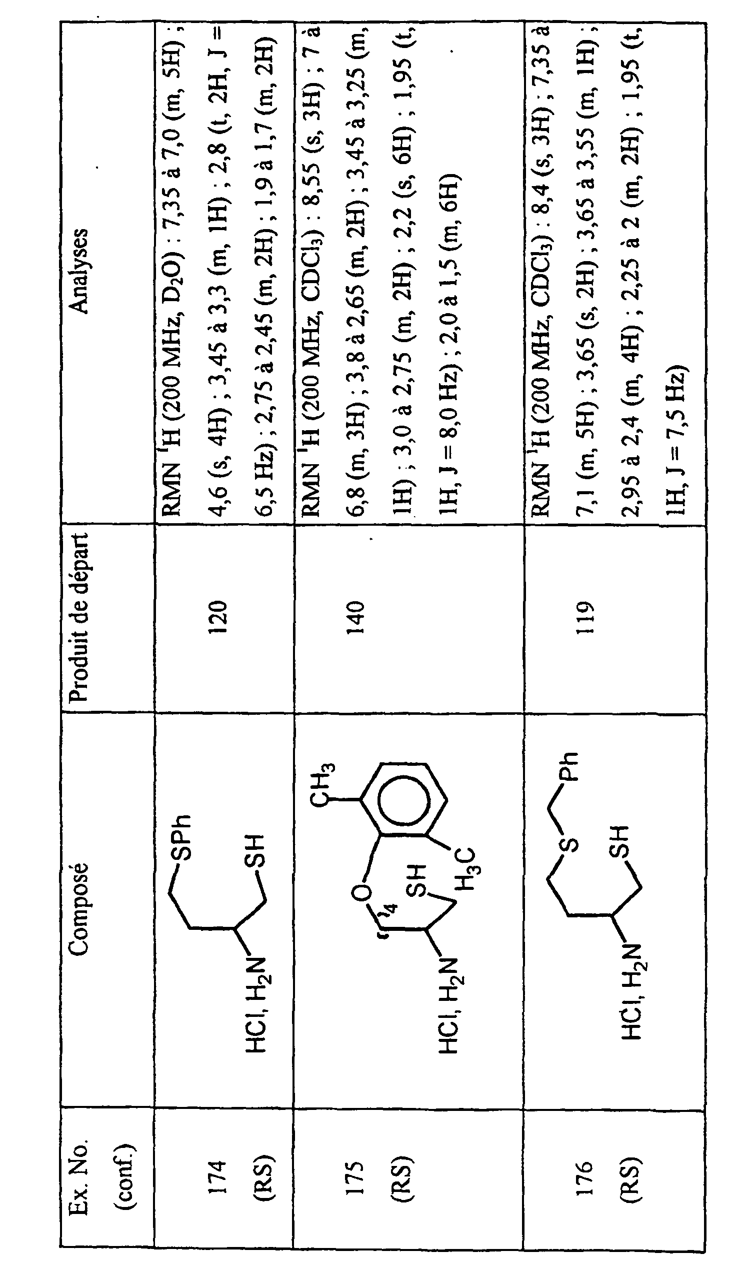

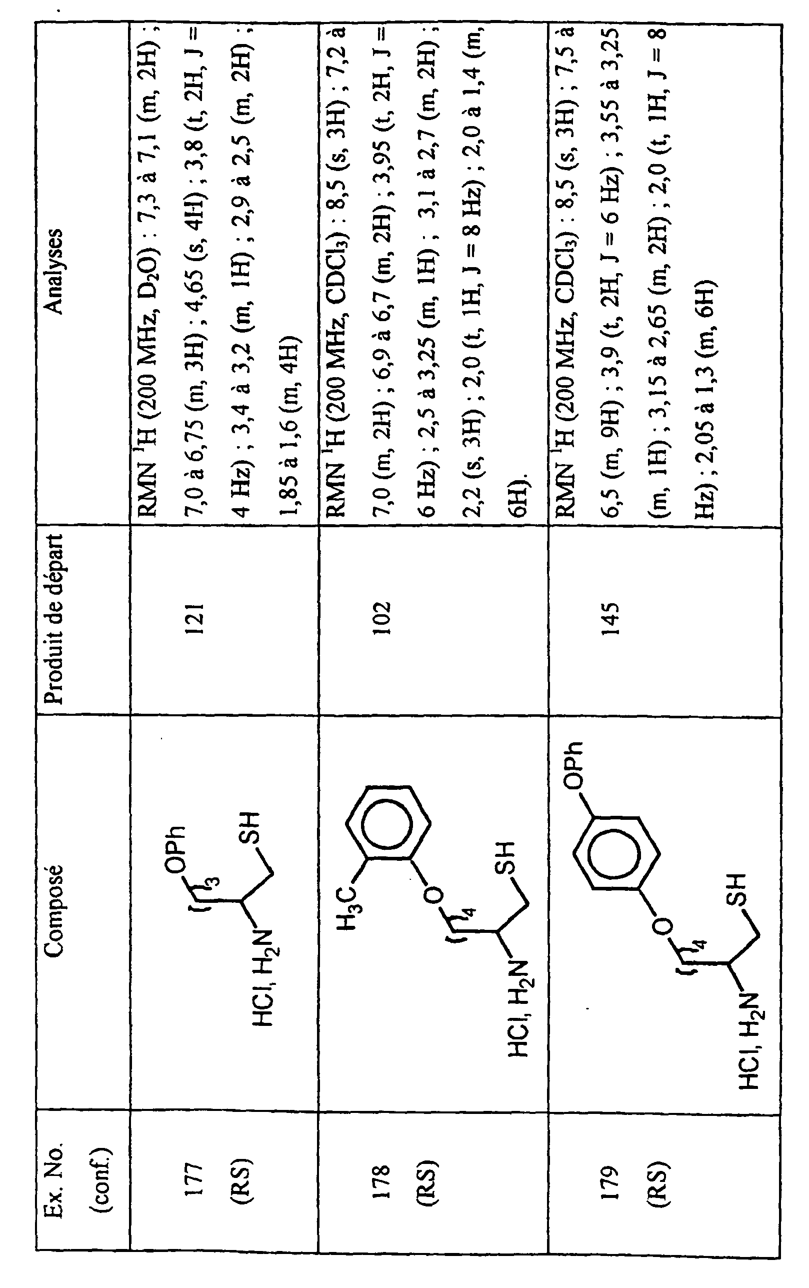

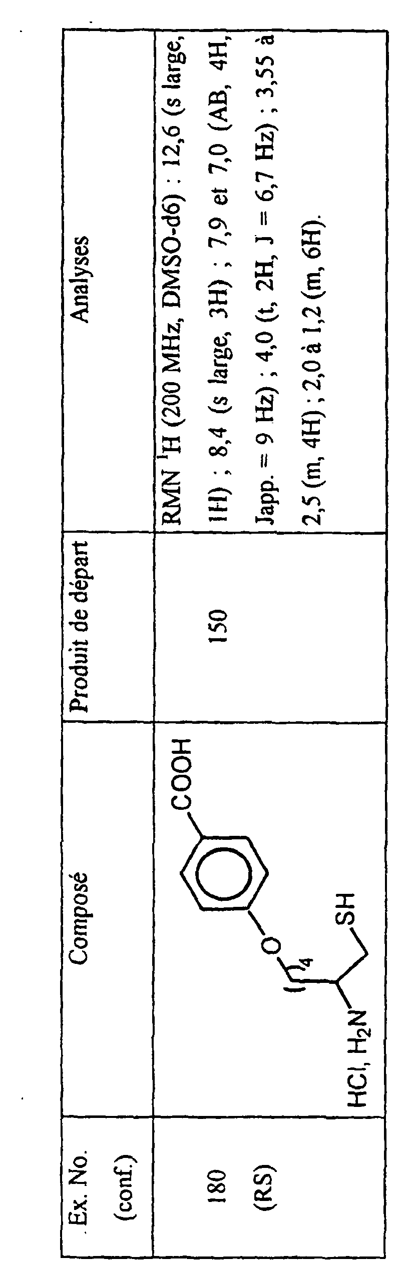

### Exemple 181

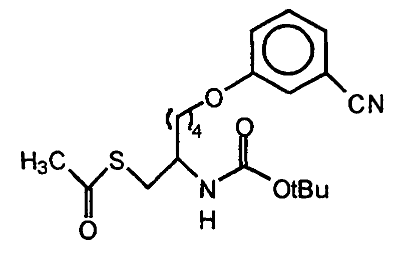

A 2,04 g (6,35 mmol) d'acide de l'exemple 150 en solution dans 16 ml de tBuOH, on ajoute successivement 1,74 g (6,35 mmol) de DPPA puis 0,64 g (6,35 mmol) de triéthylamine. On chauffe au reflux pendant une nuit puis on concentre le milieu réactionnel sous vide. Le résidu est repris par 40 ml d'acétate d'éthyle puis est successivement lavé avec de l'eau (1 fois 10 ml), une solution aqueuse saturée de NaHCO₃ (2 fois 10 ml), puis à l'eau (1 fois 10 ml).

La phase organique est séchée sur MgSO₄, filtrée puis concentrée sous vide. On obtient un résidu huileux que l'on purifie par chromatographie éclair sur silice en utilisant le mélange éther - éther de pétrole (4/7) puis l'éther comme éluants. On obtient 1,6 g (Rendement 64 %) d'un résidu huileux.

### Exemple 182

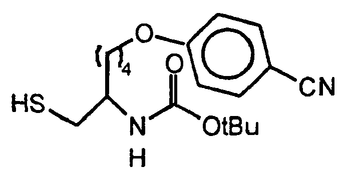

A 1,6 g (4,08 mmol) du thioester de l'exemple 181 sous atmosphère inerte en solution dans 10 ml de MeOH, on ajoute 4,9 ml d'une solution aqueuse de soude 1N. On agite 45 minutes à température ambiante puis on concentre le milieu réactionnel sous vide.

On reprend le résidu avec de l'éther (30 ml) et on lave successivement avec de l'eau (1 fois 8 ml) puis avec une solution aqueuse d'acide chlorhydrique 1N (1 fois 8 ml). La phase éthérée est séchée sur MgSO₄, filtrée puis évaporée à sec. Le résidu huileux est purifié par chromatographie éclair sur silice en utilisant le mélange éther - éther de pétrole (1/1) comme éluant. On obtient 0,87 g (Rendement 60,8 %) du composé désiré sous forme d'une huile jaune.

### Exemple 183

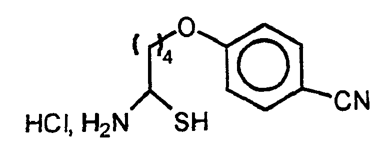

Une solution de 0,87 g (2,48 mmol) du composé de l'exemple 182 dans 9 ml de MeOH est refroidie à -50°C à l'aide d'un bain acétone/carboglace. On ajoute ensuite du HCl gazeux à la surface du milieu réactionnel de façon à ce que la température ne dépasse pas -10°C. Une fois que cette addition n'est plus exothermique, on revient à température ambiante et on concentre le milieu réactionnel sous vide. On obtient 0,66 g de solide blanc que l'on sèche sous vide sur P₂O₅.

Le solide est ensuite trituré dans 10 ml d'Et₂O anhydre. On filtre, on lave le solide avec 10 ml d'Et₂O et on sèche au dessicateur. On obtient 0,58 g (Rendement 81,5 %) de produit attendu.
RMN ¹H (200 MHz, CDCl₃) : 8,5 (s, 3H) ; 7,5 et 6,85 (AB, 4H, Japp. = 8 Hz) ; 3,95 (t, 2H, J = 6,5 Hz) ; 3,5 à 3,35 (m, 1H) ; 3,05 à 2,7 (m, 2H) ; 2,1 à 1,4 (m, 6H) ; 1,9 (t, 1H, J = 8 Hz).

Les exemples 184 à 218 sont préparés selon le même procédé que celui décrit pour l'exemple 183.

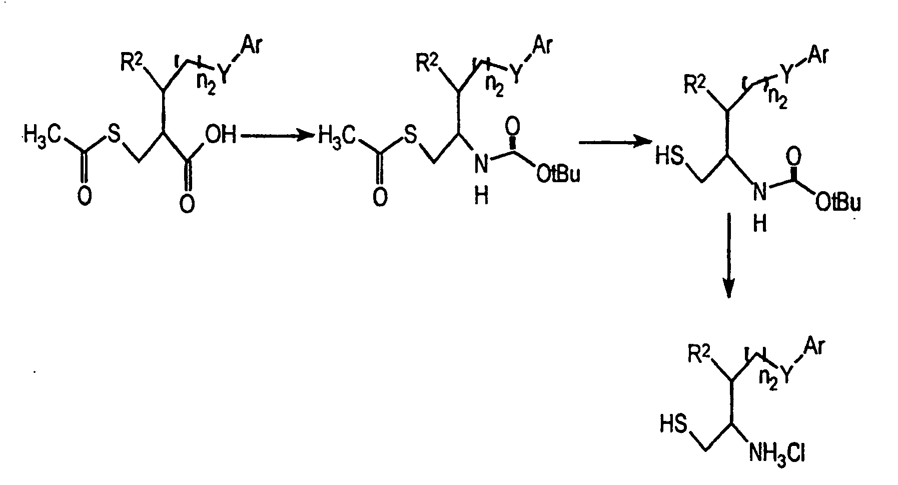

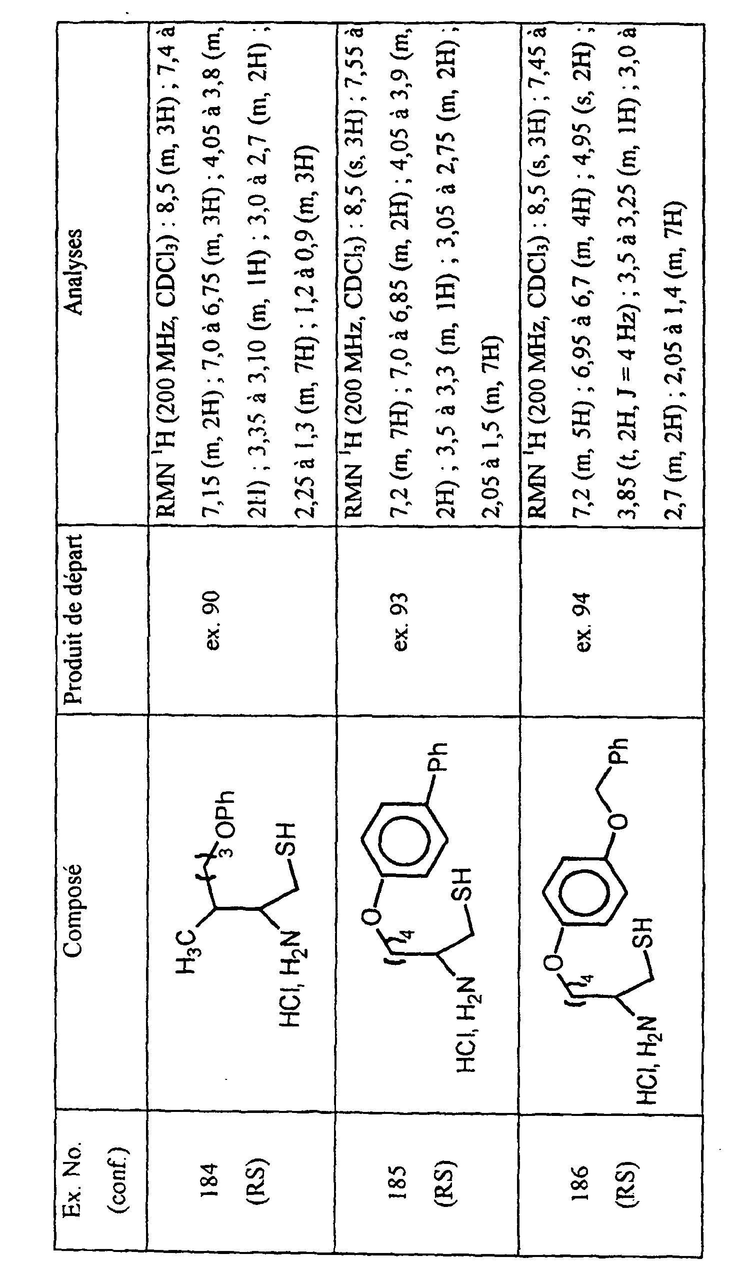

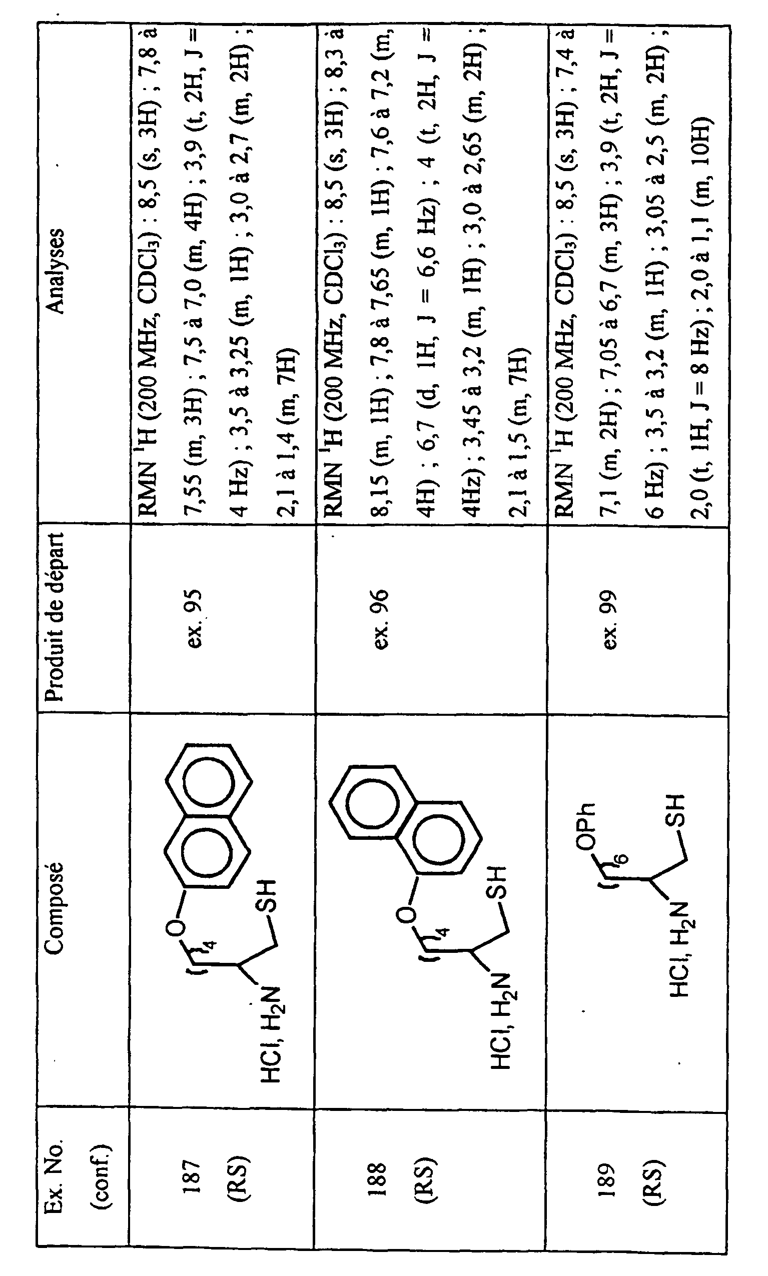

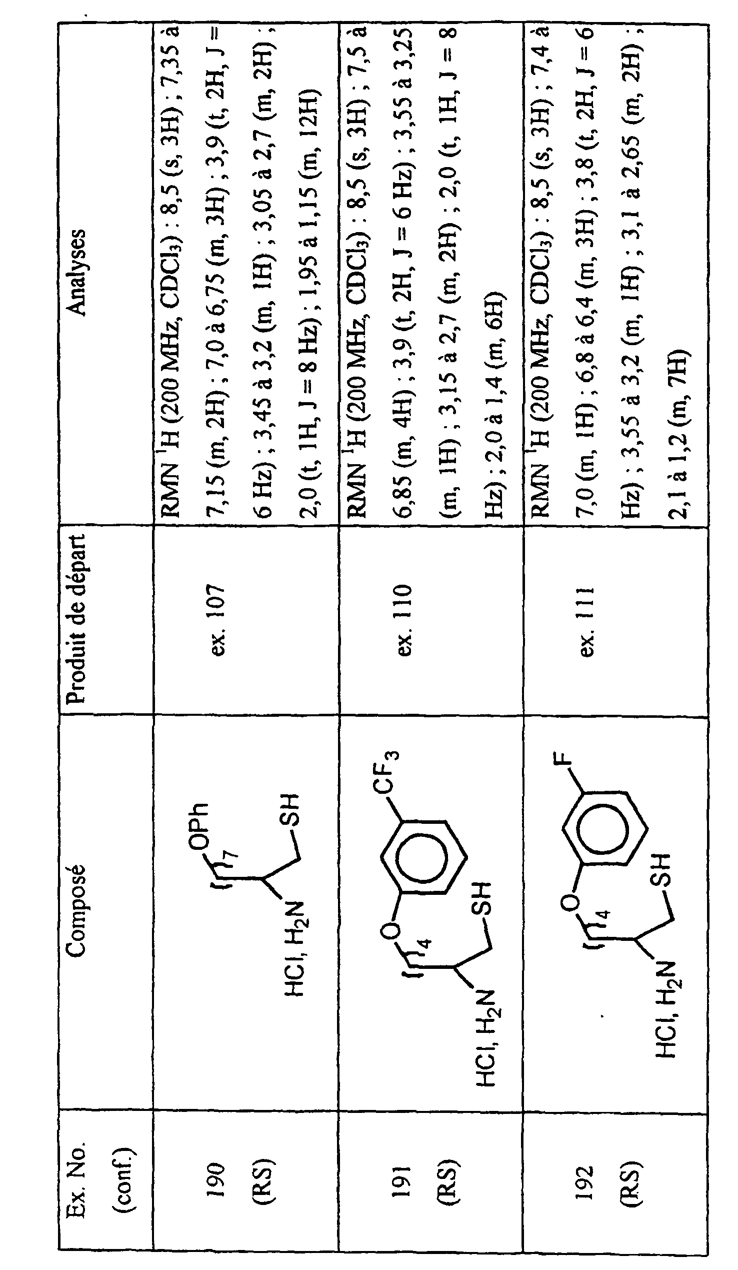

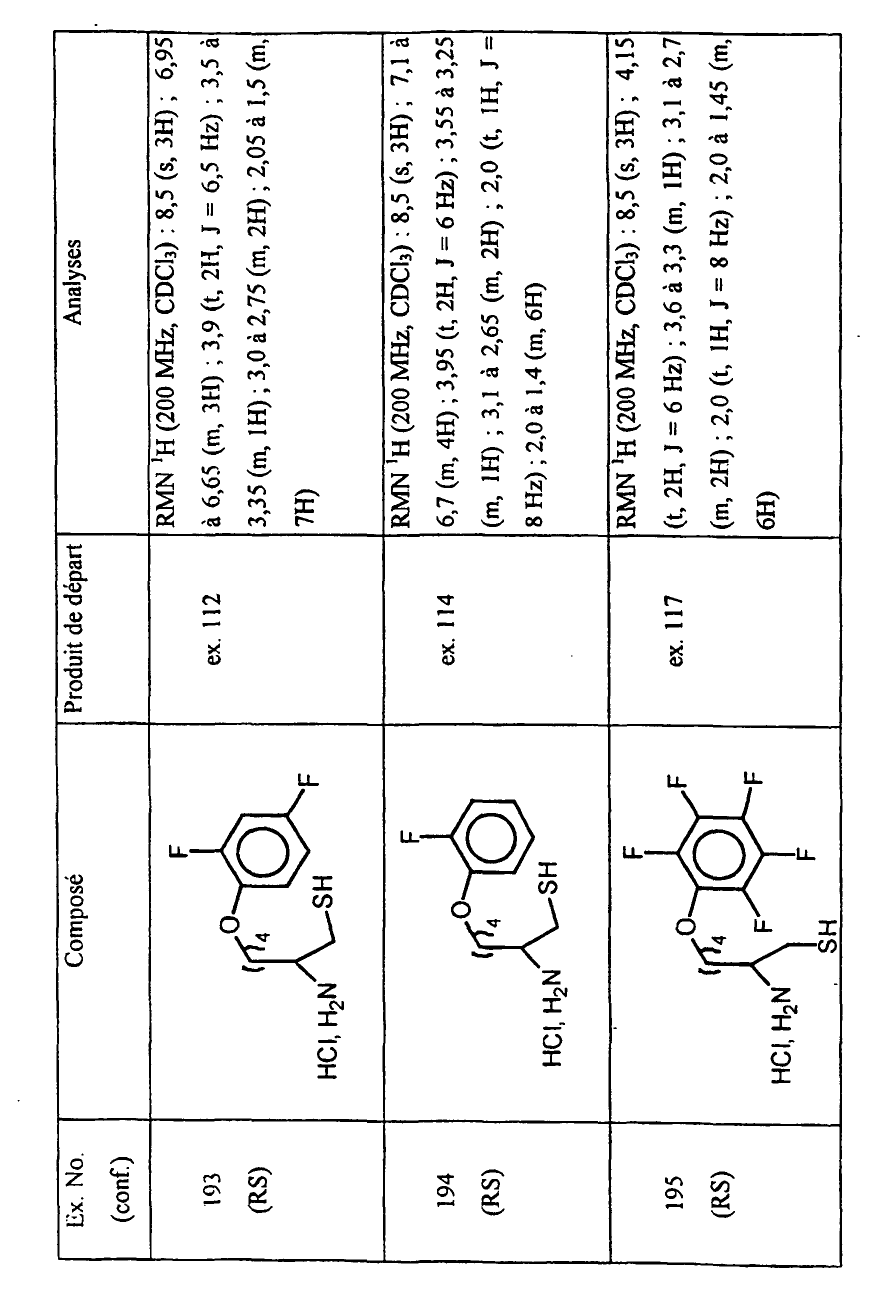

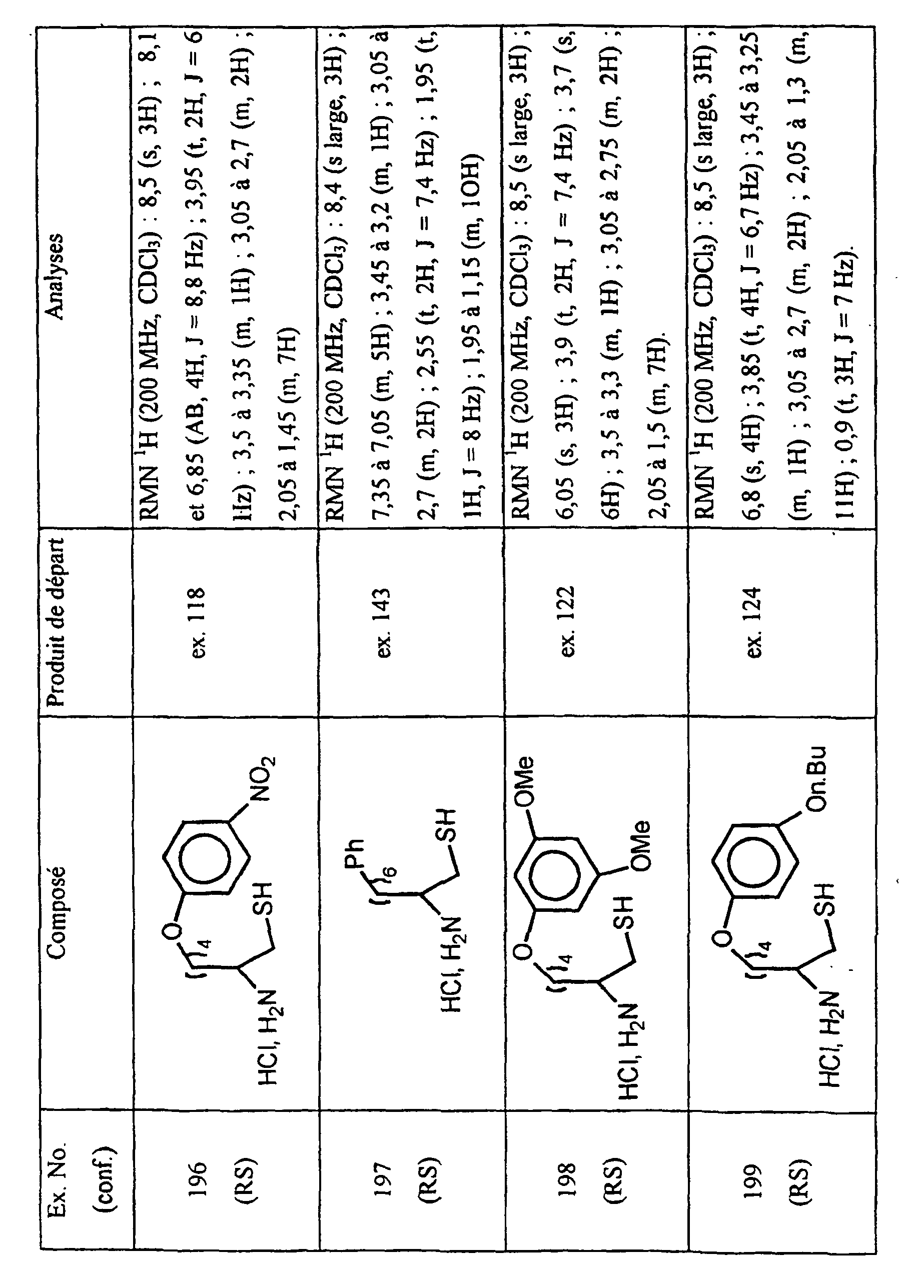

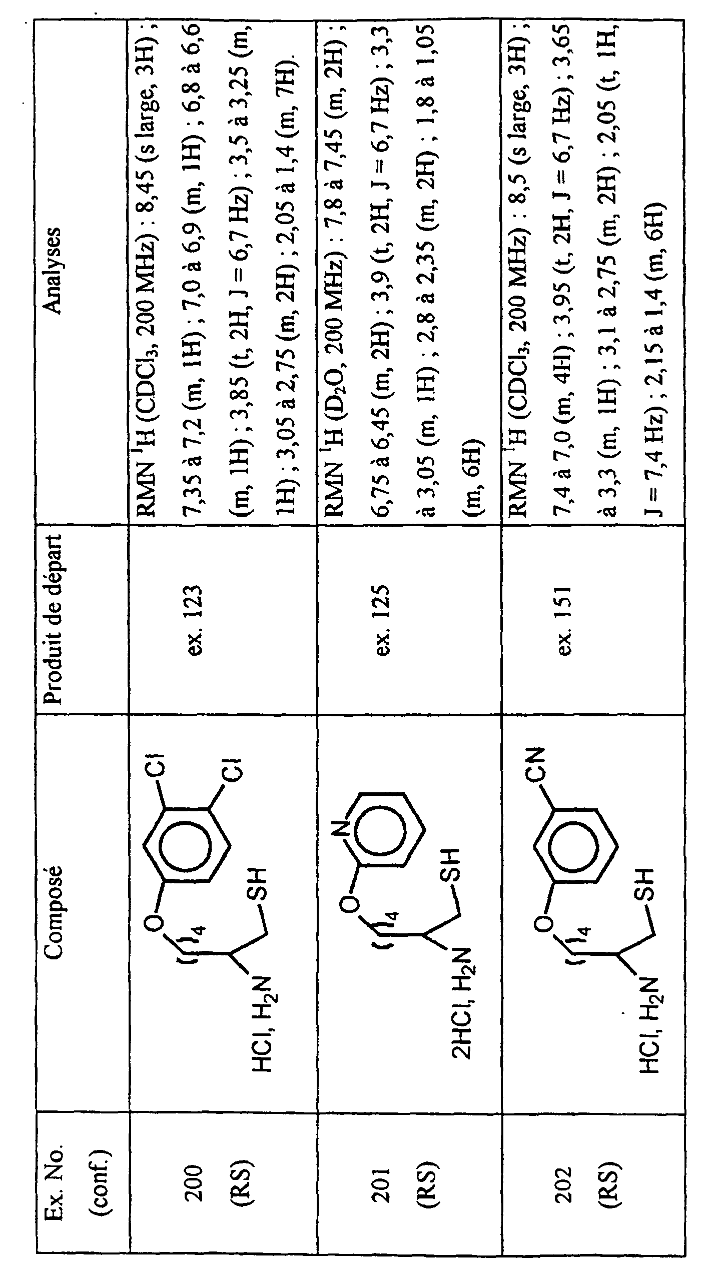

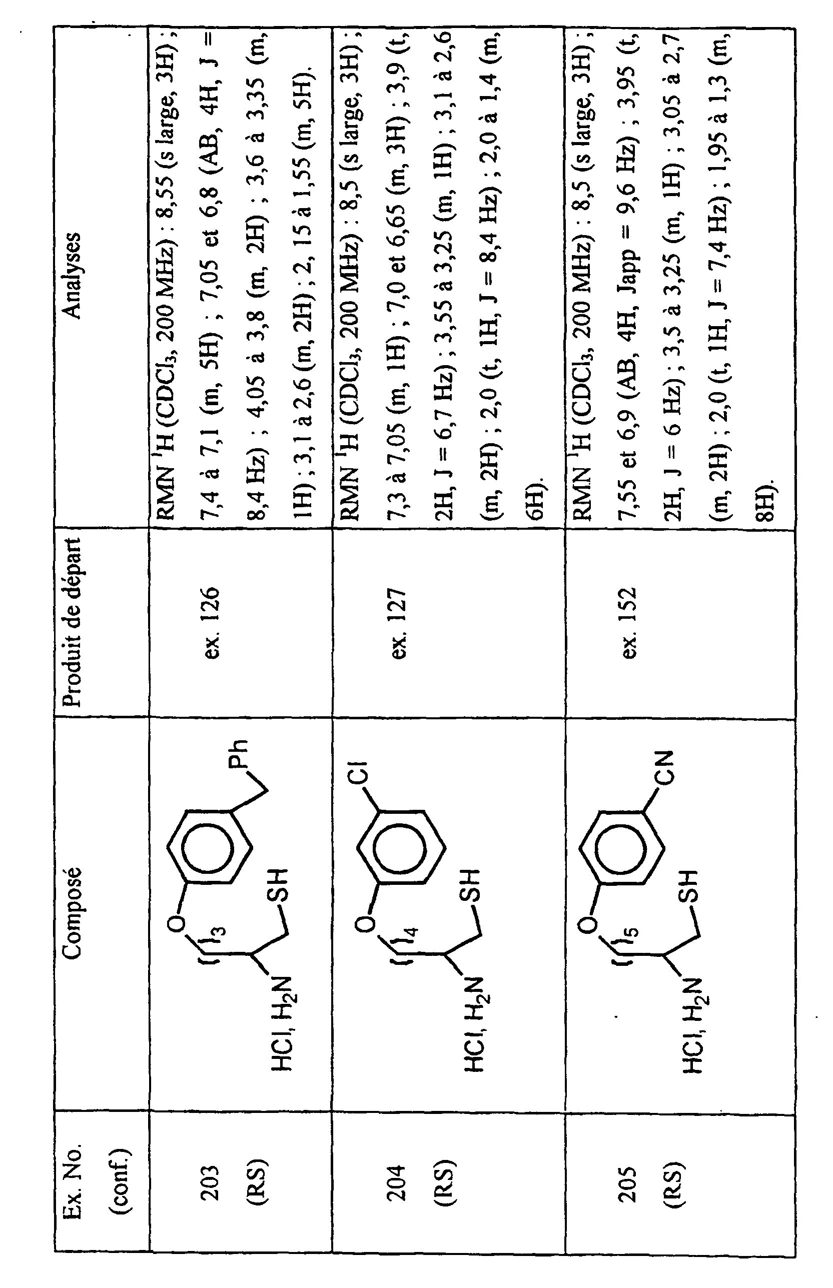

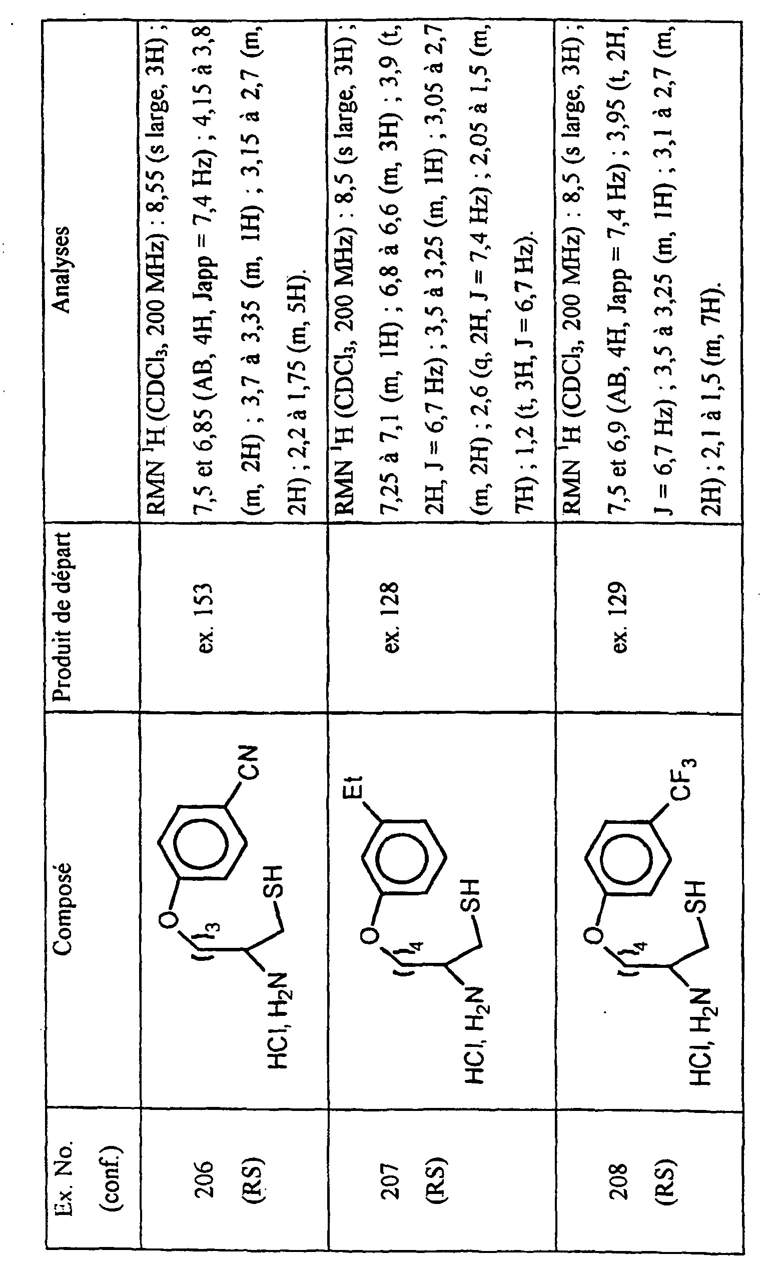

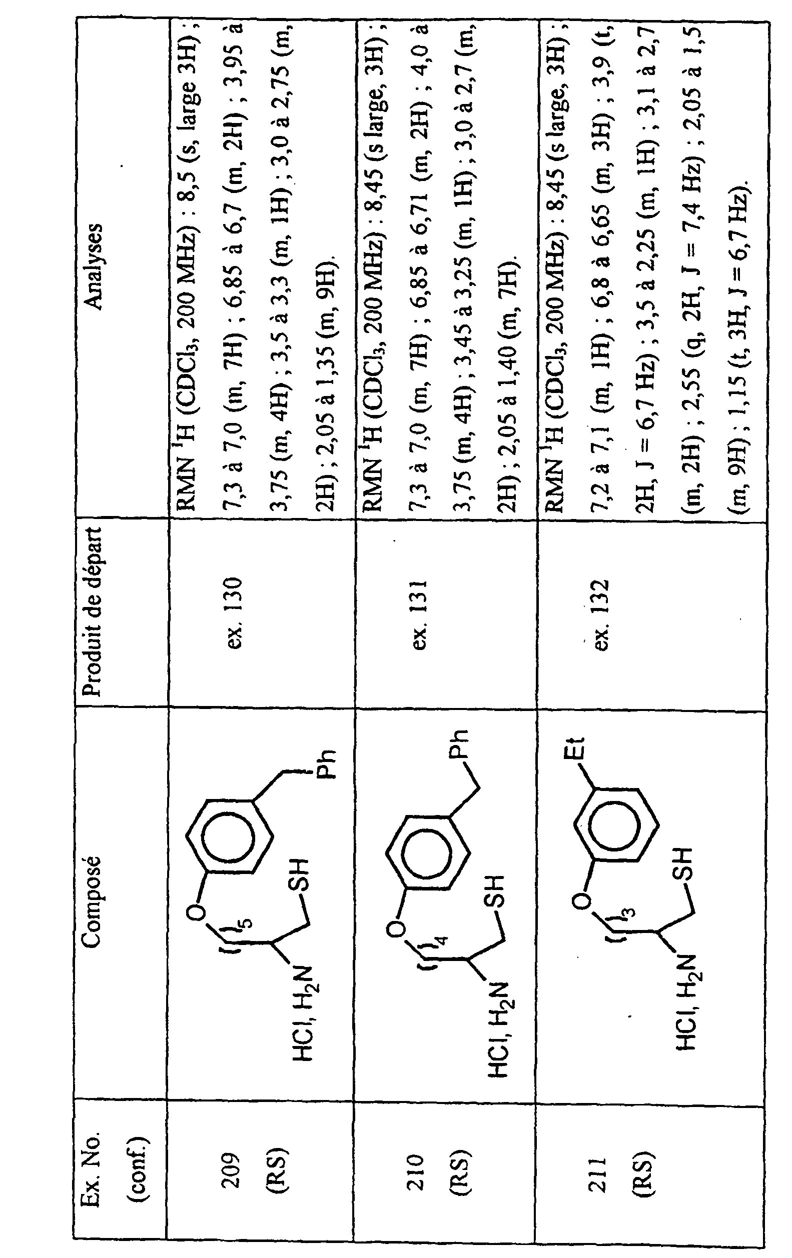

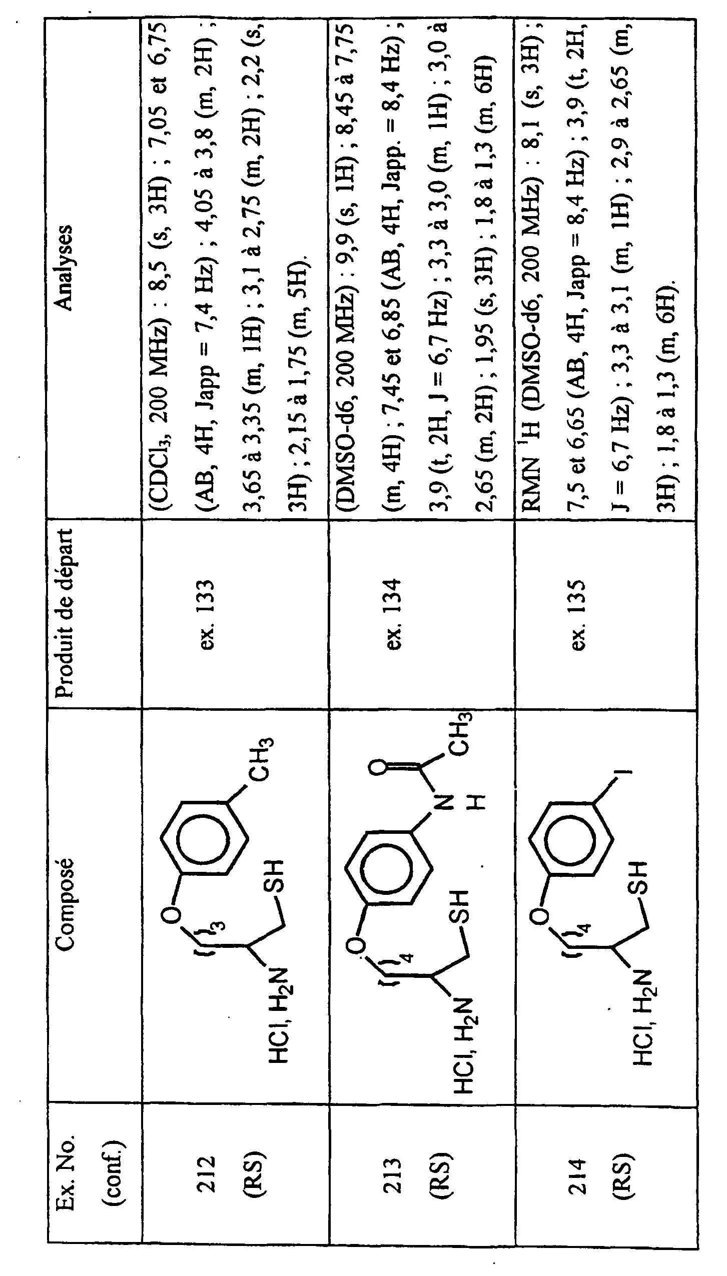

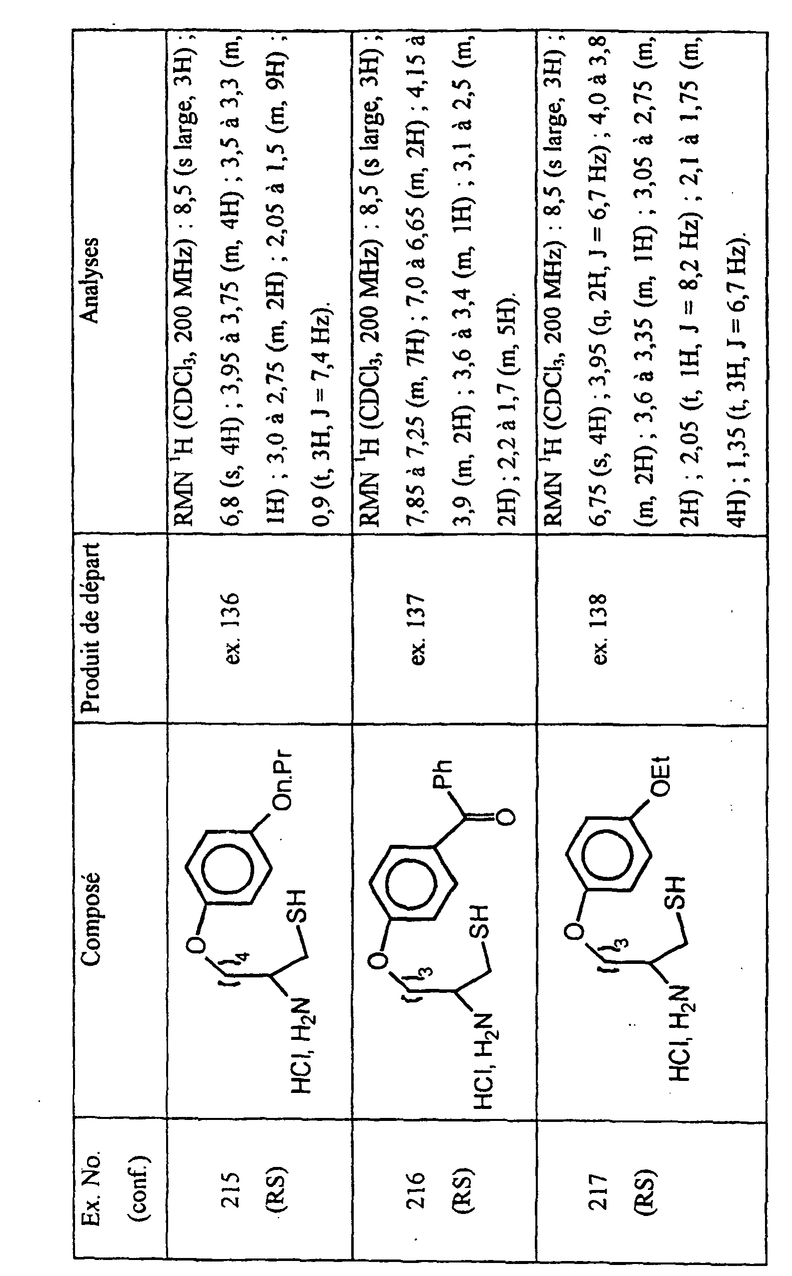

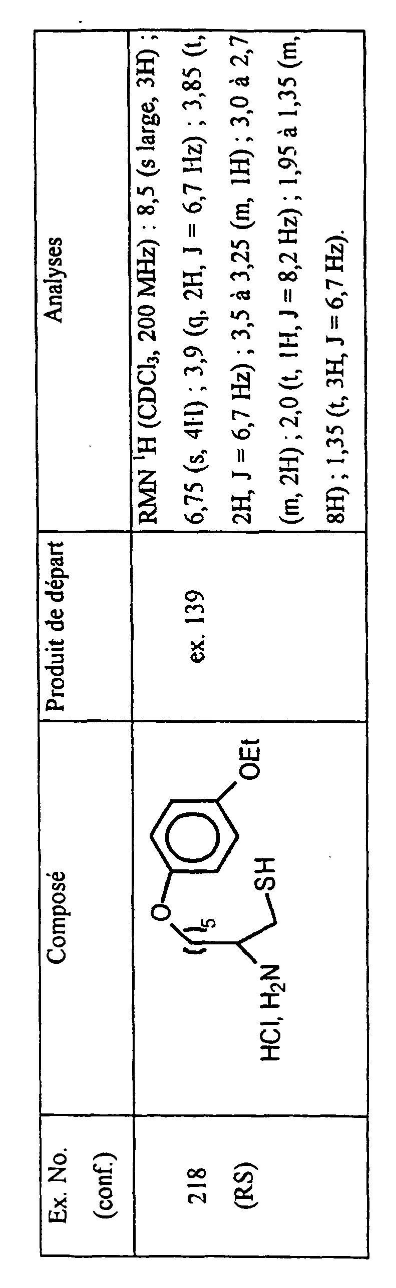

### Exemple 219

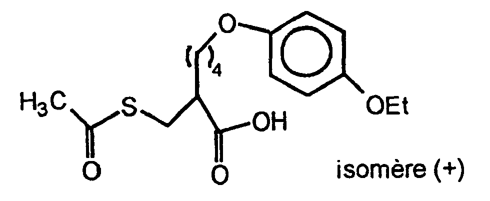

A une solution de 20 g (58,82 mmole) de l'acide racémique de l'exemple 115 dans 200 ml d'éther éthylique, on ajoute une solution de 9,70 g (58,82 mmol) de (1R, 2S)-(-)-éphédrine dans 50 ml d'éther éthylique. L'agitation est maintenue pendant 18 heures à température ambiante.

On filtre et on lave le sel avec 100 ml d'éther éthylique. Après séchage sous vide, on obtient 15,01 g (50 %) de sel blanc.
[α]_{D}²⁰ = -1,3° (c = 1,5 dans CHCl₃).

Ce sel est ensuite recristallisé par dissolution dans 40 ml de dichlorométhane et 300 ml d'éther éthylique à température ambiante. L'agitation est maintenue pendant une nuit à température ambiante.

On filtre, on lave avec de l'éther éthylique et on sèche sous vide. On obtient 6,84 g (86 %) de sel.
[α]_{D}²¹ = +4,7° (c = 1,2 dans le CHCl₃).

La recristallisation est répétée encore 5 fois afin d'obtenir un pouvoir rotatoire stable.
Rendement global des 6 recristallisations : 33 %.
Pouvoir rotatoire du sel final : [α]_{D}²¹= +7,8° (c = 1,2 dans le CHCl₃).

A une solution de 5,1 g (10,1 mmol) de sel (+) optiquement pur dans 30 ml de dichlorométhane, on ajoute, à environ 5°C, une solution d'HCl N jusqu'à pH = 1. La phase organique est lavée avec 10 ml d'eau, séchée sur MgSO₄, filtrée et concentrée sous vide. On obtient ainsi 3,26 g (95 %) de solide blanc.
F = +64°C.
[α]_{D}²² = +26,3° (c = 1,4 dans le CHCl₃).
Rendement global du dédoublement : 16 %.

### Exemple 220

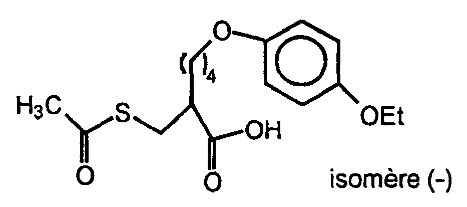

L'acide racémique de l'exemple 115 est dédoublé avec la (1S, 2R)-(+)-éphédrine dans l'éther éthylique comme décrit à l'exemple 119.
Rendement : 63 %.
[α]_{D}²² = +1,1° (c = 1,2 dans le CHCl₃).

Le sel est ensuite recristallisé 5 fois dans un mélange de dichlorométhane et d'éther éthylique suivant le même mode opératoire que celui décrit à l'exemple 119.
Rendement global des 5 recristallisations : 36 %.
Pouvoir rotatoire du sel final : [α]_{D}²¹ = -7,8° (c = 1,2 dans le CHCl₃).

L'acide est ensuite libéré comme décrit à l'exemple 219. Rendement : 97 %.
F : + 64°C.
[α]_{D}²¹ = -26,2° (c = 1,4 dans le CHCl₃).
Rendement global du dédoublement : 22 %.

### Exemple 221

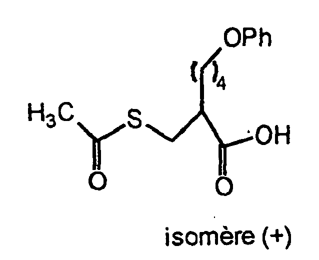

L'isomère (+) est obtenu selon la même suite réactionnelle que celle décrite à l'exemple 219 mais en partant de l'acide racémique de l'exemple 89 et de la (1R, 2S)-(-)-éphédrine.

Pouvoir rotatoire du sel obtenu après l'étape de dédoublement : [α]_{D}²¹ = -5,6° (c = 1,2 dans le CHCl₃).

Pouvoir rotatoire du sel obtenu après 6 recristallisations : [α]_{D}²¹ = +6,1° (c = 1,2 dans le CHCl₃).
Pouvoir rotatoire de l'acide libéré : [α]_{D}²¹ = +26,4° (c = 1,2 dans le CHCl₃).
Point de fusion de l'acide (+) : < 50°C.
Rendement global du dédoublement : 12 %.

### Exemple 222

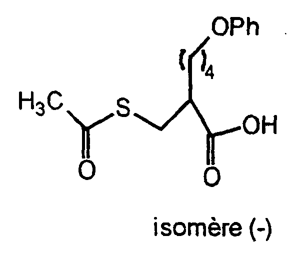

L'isomère (-) est obtenu selon la même suite réactionnelle que celle décrite à l'exemple 220 mais en partant de l'acide racémique de l'exemple 89 et de la (1S, 2R)-(+)-éphédrine.

Pouvoir rotatoire du sel obtenu après l'étape de dédoublement : [α]_{D}²¹ = +6,1° (c = 1,2 dans le CHCl₃).

Pouvoir rotatoire du sel après 8 recristallisations :
[α]_{D}²¹= -8,3° (c = 1,2 dans le CHCl₃).
Pouvoir rotatoire de l'acide libéré : [α]_{D}²¹ = -29,6° (c = 1,2 dans le CHCl₃).
Point de fusion de l'acide (-) : < 50°C.
Rendement global du dédoublement : 10 %.

Les exemples 223 à 226 sont préparés selon le même procédé que celui décrit pour l'exemple 183. 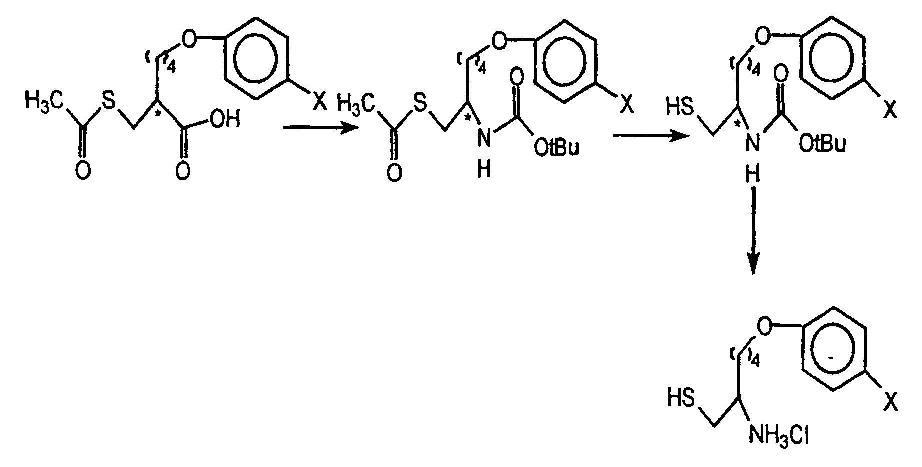
X = H, OEt

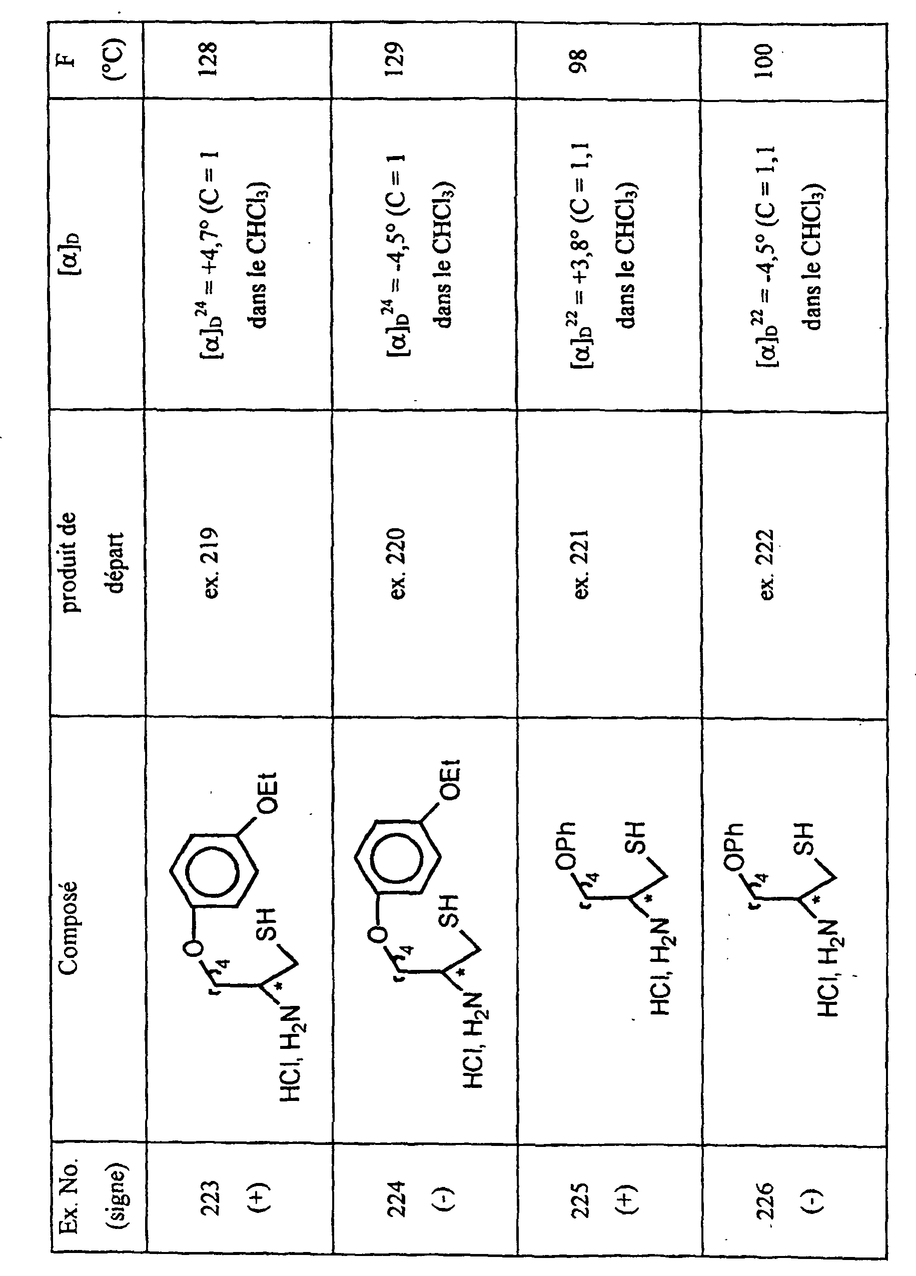

### Exemple 227

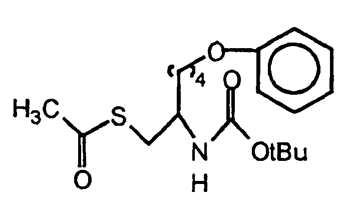

L'acide de l'exemple 89 est traité par le DPPA, NEt₃ et l'alcool terbutylique selon le même procédé que celui décrit à l'exemple 181.

### Exemple 228

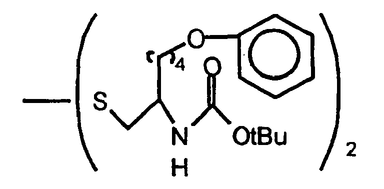

A une solution de 2,05 g (5,58 mmol) du carbamate de l'exemple 227 dans 10 ml d'EtOH, on ajoute à 0°C 11,1 ml de NaOH 1N. On agite pendant une nuit à température ambiante. On ajoute 10 ml d'une solution 0,3 M d'iode dans l'éthanol. On évapore les solvants et on reprend avec 50 ml d'Et₂O. On lave à l'eau (1 fois 20 ml), avec une solution aqueuse saturée de thiosulfate de sodium (2 fois 20 ml) et avec une solution aqueuse saturée de NaCl (1 fois 15 ml). On sèche sur MgSO₄, on filtre et on concentre. On obtient 1,85 g de solide blanc que l'on chromatographie sur silice (éluant : éther-éther de pétrole 2/8). On obtient-1,5 g (Rendement 84 %) de solide blanc.

### Exemple 229

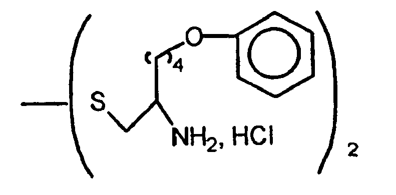

Le carbamate de l'exemple 228 est traité par HCl gaz dans le MeOH selon le même procédé que celui décrit à l'exemple 183.
RMN ¹H (DMSO-d6, 200 MHz) : 8,3 (s large, 3H) ; 7,35 à 7,1 (m, 2H) ; 7,0 à 6,75 (m, 3H) ; 3,9 (t, 2H, J = 5 Hz) ; 3,6 à 3,5 (m, 1H) ; 3,2 à 2,95 (m, 2H) ; 1,95 à 1,35 (m, 6H).

Les exemples 230 à 236 sont préparés selon le même procédé que celui décrit pour l'exemple 229.

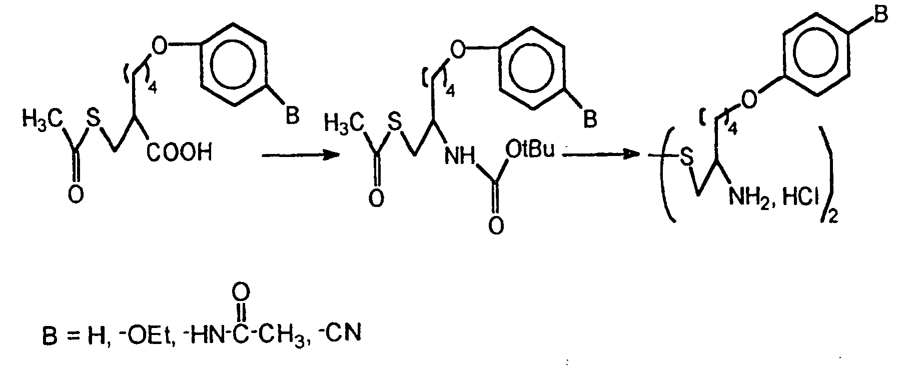

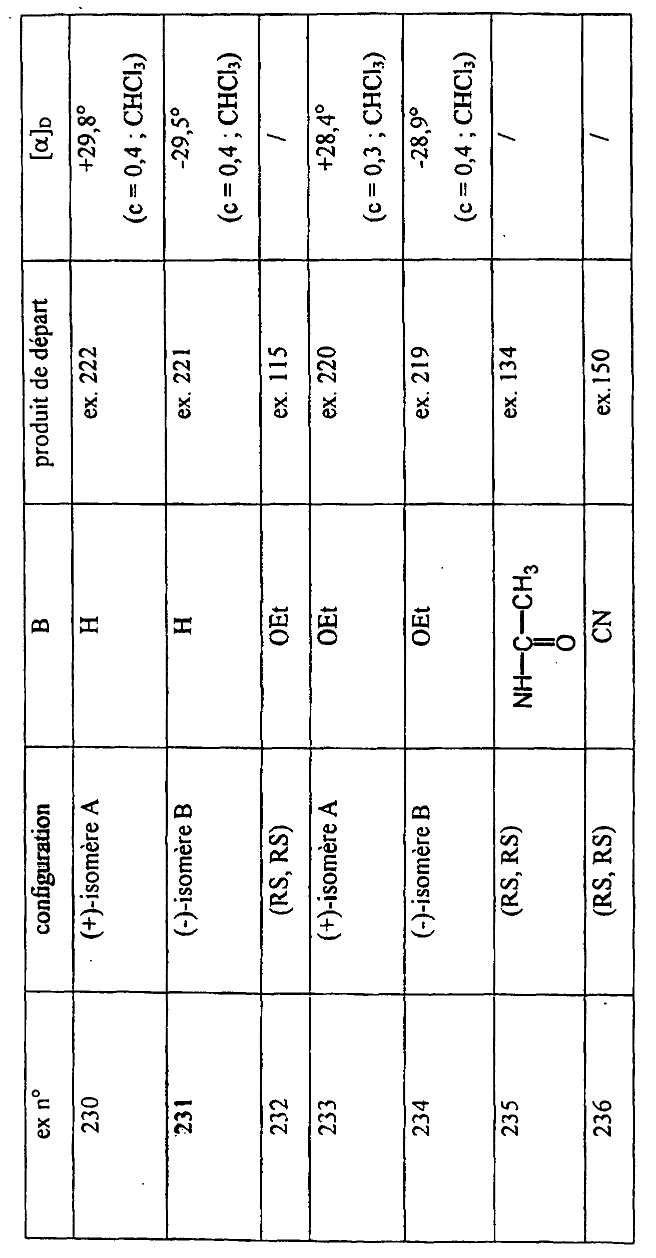

### Exemple 237

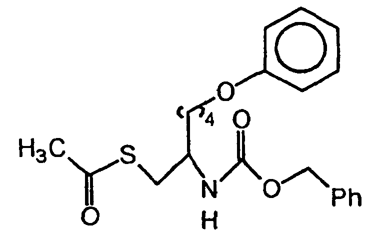

L'acide de l'exemple 89 est traité par le DPPA, NEt₃ et l'alcool benzylique selon le même procédé que celui décrit à l'exemple 9.

### Exemple 238

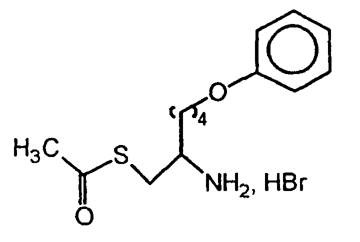

On met en solution 0,5 g (1,24 mmol) du carbamate de l'exemple 230 dans 6 ml d'une solution d'acide bromhydrique à 30 % dans l'acide acétique. On agite pendant 15 minutes à température ambiante puis on évapore à sec. Après trituration dans l'éther éthylique, le résidu huileux est séché sous vide sur P₂O₅.

On obtient 0,3 g (70 %) d'huile.
RMN ¹H (CDCl₃, 200 MHz) : 8,2 (s large, 3H) ; 7,3 à 7,15 (m, 2H) ; 6,95 à 6,75 (m, 3H) ; 3,9 (t, 5 Hz, 2H) ; 3,65 à 3,05 (m, 3H) ; 2,3 (s, 3H); 2,2 à 1,5 (m, 6H).

### ACTIVITE BIOLOGIOUE

### Essais biologiques des composés selon l'invention

### 1) Inhibition de l'activité aminopeptidase de la LTA₄ hydrolase recombinante

Les composés ont été testés en utilisant la LTA₄ hydrolase humaine recombinante (Minami et coll., FEBS Letters, 1988, 229 : 279). La LTA₄ hydrolase exprimée par E. Coli JM109 est purifiée principalement selon Minami et coll. (J. Biol. Chem., 1987, 262: 13873).

L'inhibition de l'activité aminopeptidase de l'enzyme est mesurée au moyen d'une méthode fluorimétrique en microplaques 96 puits. L'enzyme recombinante (0,5 µg dans 50 µl de Tris-HCl 50 mM pH 7,4) est préincubée 10 minutes à 37°C en présence d'inhibiteur et de dithiothréitol (DTT, 10⁻⁵M). Le substrat Alanyl-amido-méthylcoumarine (Ala-AMC, 25 µM -Tris HCl 50 mM, pH 7,4) est ajouté et l'incubation poursuivie 15 minutes à 37°C. La libération d'AMC est mesurée par fluorimétrie.

Afin d'évaluer la spécificité des composés selon l'invention, certains d'entre eux ont aussi été testés pour leur capacité à inhiber l'activité de l'aminopeptidase M membranaire (EC 3.4.11.2). Le même test est réalisé avec 0,1 µg d'aminopeptidase M (Pierce, USA).

Des résultats typiques sont donnés au tableau I ci-dessous :

**Tableau 1 INHIBITION DE LA LTA₄ HYDROLASE IN VITRO**

| Composé n° | Inhibition de l'activité aminopeptidase de la LTA₄ hydrolase Ki | Inhibition de l'aminopeptidase M Ki |
|---|---|---|
| 23 | 62 nM | - |
| 172 | 15 nM | - |
| 173 | 33 nM | 0,2 µM |

### 2) Inhibition de la biosynthèse de LTB₄ in vitro

La biosynthèse de LTB₄ est mesurée dans le sang total humain en présence d'inhibiteurs de la LTA₄ hydrolase selon l'invention. Un échantillon de 50 µl de sang prélevé sur héparinate de sodium est préincubé 10 minutes à 37°C en présence d'inhibiteur (Tris-HCl 50 mM, NaCl 0,15 M, DTT 10⁻⁵M, pH 7,4).

Le substrat LTA₄ a été préparé extemporanément par hydrolyse alcaline du LTA₄ méthyl ester (Cayman Chemical Co., USA). Après 10 minutes d'incubation en présence de LTA₄ (1 µM dans Tris-HCl 50 mM, NaCl 0,15 M, BSA, 0,5 %, pH 7,4), la réaction est stoppée par dilution au 1/20^{ème} dans du tampon phosphate de potassium 0,1M, NaN₂, 1,5 mM, NaCl 0,4 M, EDTA 1 mM, BSA 0,1 %, pH 7,4 -4°C).

Le LTB₄ est dosé par enzymo-immunodosage (Cayman Chemical Co, USA).

Les résultats sont donnés dans le tableau II ci-dessous.

**Tableau II INHIBITION DE LA BIOSYNTHESE DE LTB₄ IN VITRO**

| **Composé n°** | **Inhibition de l'activité LTA**_{**4**} **hydrolase dans le sang total humain Ki** |
|---|---|
| 172 | 190 nM |
| 173 | 200 nM |

### 3) Inhibition de la biosynthèse de LTB₄ ex vivo

Les composés inhibiteurs de la LTA₄ hydrolase selon l'invention sont mis en suspension dans la méthylcellulose 1,25 % et adminitrés aux souris par voie orale à la dose de 10 mg/kg. Trente minutes après, les souris sont sacrifiées et le sang prélevé sur héparinate de lithium. Le sang est alors comme précédemment incubé 10 minutes à 37°C en présence de LTA₄ puis le LTB₄ formé, dosé par enzymo-immunodosage.

Les résultats obtenus sont indiqués dans le tableau III ci-dessous :

**Tableau III INHIBITION DE LA BIOSYNTHESE DE LTB₄ EX VIVO**

| **Composé n°** | **Inhibition (%)** |
|---|---|
| 24 | 28 |
| 177 | 68 |

### 4) Activité anti-inflammatoire

### a) Oedème de la patte induit par le Zymosan chez la souris

Le zymosan est un polysaccharide de la membrane cellulaire de levure. Injecté dans la patte des animaux, il provoque une réaction inflammatoire locale sous forme d'un oedème. Une heure après administration de l'inhibiteur testé par voie orale à des souris, à la dose de 30 mg/kg, 25 µl de zymosan (0,05 % dans NaCl 0,15M) sont injectés dans la patte arrière gauche et 25 µl de sérum physiologique dans la patte droite comme contrôle. L'animal est sacrifié après 4 heures, les pattes coupées à la cheville et pesées afin d'évaluer l'oedème développé.

Les résultats sont donnés dans le tableau IV ci-dessous :

**Tableau IV EFFET INHIBITEUR SUR L'OEDEME DE LA PATTE INDUIT PAR LE ZYMOSAN**

| **Composé n°** | **Inhibition (%)** |
|---|---|
| 156 | 35 |

### b) Inflammation induite par la carragenine dans le modèle de poche d'air chez la souris

Les poches d'air sont formées chez la souris selon Dawson et coll. (Int. J. Tiss. React., 1991, XIII: 171). Au jour J, 5 ml d'air sont injectés par voie sous-cutanée dans le dos de la souris. A J + 3, la poche est maintenue en réinjectant 3 ml d'air. A J + 6, soit un inhibiteur de cyclooxygénase (indométhacine 5 mg/kg), soit un inhibiteur de LTA₄ hydrolase selon l'invention (25 mg/kg), ou les deux en association sont administrés par voie orale. Après 1 h, 1 ml de carragenine 1% est injecté dans la poche d'air. Les animaux sont sacrifiés 4 h après l'injection de carragenine. La poche d'air est lavée par 1 ml de tampon phosphate de potassium 50 mM, NaCl 0,15 M, pH 7,4. L'exudant est récupéré, centrifugé à 10,000 g, 5 minutes. Le LTB₄ est dosé dans le surnageant par enzymo-immunodosage.

Les résultats sont indiqués dans le tableau V ci-dessous :

**Tableau V INHIBITION DE LA BIOSYNTHESE DE LTB₄ DANS LE MODELE DE LA POCHE D'AIR CHEZ LA SOURIS (inflammation par la carragénine)**

| **Traitement** | **% LTB**_{**4**} **par rapport au témoin non enflammé** |
|---|---|
| Carragénine | +87% |
| Carragénine + Indométhacine | +516% |
| Carragénine + Composé n° 156 | -24% |
| Carragénine + Indométhacine + Composé n° 156 | -28% |

### c) Effet anti-arthritique

L'arthrite au collagène a été induite chez le rat "Dark Aganti" selon le protocole de Kamada et coll. (Jpn J. Pharmacol., 1997, 74: 313). Les inhibiteurs de LTA₄ hydrolase sont administrés soit de manière "préventive" (à partir d'un jour après le collagène), soit de manière curative (lors des premières manifestations pathologiques soit à partir de 15 jours après le collagène). Les résultats du tableau VI rapportent l'effet du composé n° 229 administré 2 fois/jour par voie i.p., à la dose de 10 mg/kg sur le score d'arthrite au 23^{ème} jour après collagène. L'effet est comparé à celui de l'indométhacine (1,5 mg/kg, p.o.).

Les résultats sont idiqués dans le tableau VI ci-dessous :

**Tableau VI**

| **Traitement** | **score** |
|---|---|
| Véhicule | 3,1 ±0,90 |
| Composé 229 (curatif) | 0,3 8 ± 0,19 |
| Composé 229 (préventif) | 0 |
| Indométhacine (curatif) | 1,35 ± 0,61 |

Par ailleurs, les composé selon l'invention, en particulier les composés de formule (II) ou (III), présentent une bonne biodisponibilité, supérieure à celle des composés connus dans la technique, en particulier du document WO 94/00420.

## Revendications

1. Composés répondant à la formule (1) suivante : 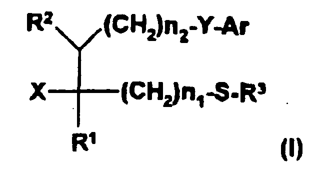
dans laquelle :
• X représente NH₂ ;
• R¹ et R² sont indépendamment choisis parmi les groupes suivants :
i) un atome d'hydrogène
ii) un groupe alkyle à chaîne linéaire ou ramifiée contenant de 1 à 6 atomes de carbone ;
• n₁ varie de 1 à 4
• n₂ varie de 0 à 10
• R³ est choisi parmi les groupes suivants :
i) un atome d'hydrogène
ii) 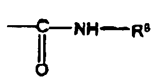
iii) 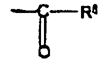
iv) 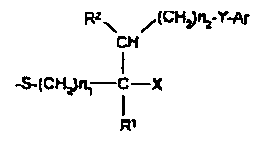
• Y est choisi parmi les groupes suivants :
i) -O-
ii) -CH₂-
iii) -S-
iv) -OCH₂-
v) -SCH₂-
• Ar est choisi parmi les groupes suivants :
i) un groupe phényle, non substitué, ou mono ou polysubstitué avec des substituants choisis parmi les atomes d'halogène et les groupes CF₃, alkyle à chaîne linéaire ou ramifiée contenant de 1 à 6 atomes de carbone, alcoxy à chaîne linéaire ou ramifiée contenant de 1 à 6 atomes de carbone, NH₂, NO₂, CN, OH, CO₂H, OPh, OCH₂Ph, SMe, SEt, Ph, CH₂Ph et NHCOR⁷ où R⁷ est un groupe alkyle à chaîne linéaire ou ramifiée contenant de 1 à 6 atomes de carbone,
ii) 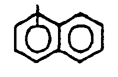
iii) 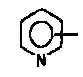
iv) 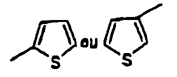
v) 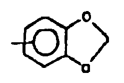
vi) 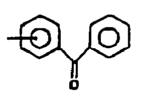
• R⁶ représente un groupe alkyle à chaîne linéaire ou ramifiée contenant de 1 à 6 atomes de carbone ou un groupe phényle ;
à l'exclusion des composés de formule I dans laquelle X est -NH₂, R¹ et R² sont chacun un atome d'hydrogène, n₁ = 1, n₂ = 0, Y est un groupe -OCH₂- ou -SCH₂-, Ar est un groupe phényle et R³ est un atome d'hydrogène ou les radicaux iv) correspondants,
ainsi que leurs isomères, diastéréoisomères et énantiomères et leurs sels pharmaceutiquement acceptables.

2. Composés de formule générale (I) 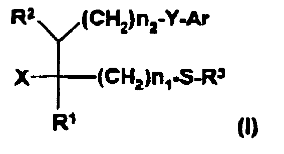
dans laquelle :
• X représente -NH₂ ;
• R¹ et R² sont indépendamment choisis parmi les groupes suivants :
i) un atome d'hydrogène
ii) un groupe alkyle à chaîne linéaire ou ramifiée contenant de 1 à 6 atomes de carbone ;
• n₁ varie de 1 à 4
• n₂ varie de 0 à 10
• R³ est choisi parmi les groupes suivants :
i) un atome d'hydrogène
ii) 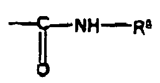
iii) 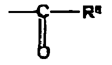
iv) 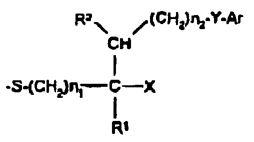
• Ar est choisi parmi les groupes suivants :
i) un groupe phényle, non substitué, ou mono ou polysubstitué avec des substituants choisis parmi les atomes d'halogène et les groupes CF₃, alkyle à chaîne linéaire ou ramifiée contenant de 1 à 6 atomes de carbone, alcoxy à chaîne linéaire ou ramifiée contenant de 1 à 6 atomes de carbone, NH₂, NO₂, CN, OH, CO₂H, OPh, OCH₂Ph, SMe, SEt, Ph, CH₂Ph et NHCOR⁷ où R⁷ est un groupe alkyle à chaîne linéaire ou ramifiée contenant de 1 à 6 atomes de carbone,
ii) 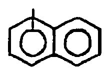
iii) 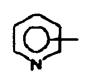
iv) 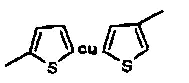
v) 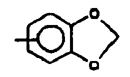
vi) 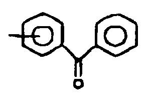
• R⁶ représente un groupe alkyle à chaîne linéaire ou ramifiée contenant de 1 à 6 atomes de carbone ou un groupe phényle
et Y est choisi parmi
i) -O-
ii) -CH₂-
iii) -S-

3. Composés selon la revendication 1 ou 2, **caractérisés en ce que** R¹ représente un atome d'hydrogène.

4. Composés selon la revendication 1 ou 2, **caractérisés en ce que** R¹ est différent de l'hydrogène.

5. Composés selon l'une des revendications 1 à 4, **caractérisés en ce que** n₁ est égal à 1.

6. Composés selon l'une des revendications 1 à 4, **caractérisés en ce que** n₁ est différent de 1.

7. Composés selon l'une des revendications 1 à 5, **caractérisés en ce qu'**ils répondent à la formule (II) suivante : 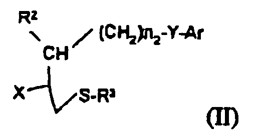
dans laquelle X, R², R³, Y, Ar et n₂ ont la signification donnée à la revendication 1 ou 2.

8. Composés selon l'une des revendications 1 à 7, **caractérisés en ce que** X représente NH₂.

9. Composés selon l'une des revendications 1 à 8, **caractérisés en ce que** R³ représente un atome d'hydrogène.

10. Composés selon l'une des revendications 1 à 9, **caractérisés en ce que** R² est différent d'un atome d'hydrogène.

11. Composés selon la revendication 10 dans laquelle R² représente un groupe méthyle.

12. Composés selon l'une des revendications 1 à 9, **caractérisés en ce que** R² est un atome d'hydrogène.

13. Composés selon l'une des revendications 1 à 12, **caractérisés en ce qu'**ils répondent à la formule (III) suivante : 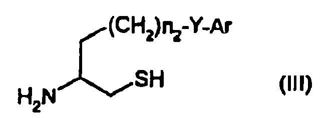
dans laquelle Y, Ar et n₂ ont la signification indiquée à la revendication 1 ou 2.

14. Composés selon l'une des revendications 1 à 13, **caractérisés en ce que** n₂ est égal à zéro.

15. Composés selon l'une des revendications 1 à 13, **caractérisés en ce que** n₂ varie de 1 à 4.

16. Composés selon la revendication 15, dans laquelle n₂ varie de 2 à 4.

17. Composés selon l'une des revendications 1 à 13, **caractérisés en ce que** n₂ est égal à 3.

18. Composés selon l'une des revendications 1 à 13, **caractérisés en ce que** n₂ est supérieur à 4.

19. Composés selon l'une des revendications 1 à 18, **caractérisés en ce que** Y représente un atome d'oxygène.

20. Composés selon l'une des revendications 1 à 18, **caractérisés en ce que** Y représente -CH₂-.

21. Composés selon l'une des revendications 1 à 18, **caractérisés en ce que** Y représente un atome de soufre.

22. Composés selon l'une des revendications 1, 3 à 18, **caractérisés en ce que** Y représente -OCH₂- ou -SCH₂-.

23. Composés selon l'une des revendications 1 à 18, **caractérisés en ce que** n₂ est égal à zéro et Y est choisi parmi -O-, -S-, -OCH₂- et -SCH₂-.

24. Composés selon l'une des revendications 1 à 23, **caractérisés en ce que** Ar représente un groupe phényle non substitué.

25. Composés selon l'une des revendications 1 à 23, **caractérisés en ce que** Ar représente un groupe phényle substitué.

26. Composés selon la revendication 25 dans laquelle Ar est un groupe phényle monosubstitué.

27. Composés selon l'une des revendications 1 à 23, **caractérisés en ce que** Ar représente un groupe phényle mono- ou polysubstitué par un groupe choisi parmi les atomes d'halogène, CF₃, alkyle à chaîne linéaire ou ramifiée contenant de 1 à 6 atomes de carbone, O(alkyle à chaîne linéaire ou ramifiée contenant de 1 à 6 atomes de carbone), NO₂, CN, CO₂H, OPh, OCH₂Ph, Ph et CH₂Ph.

28. Composés selon l'une des revendications 1 à 23, **caractérisés en ce que** Ar représente un groupe phényle mono- ou polysubstitué par un atome d'halogène, un groupe alkyle à chaîne linéaire ou ramifiée contenant de 1 à 6 atomes de carbone ou -O(alkyle à chaîne linéaire ou ramifiée contenant de 1 à 6 atomes de carbone).

29. Composés selon l'une des revendications 1 à 23 **caractérisés en ce que** Ar représente un groupe phényle substitué par un groupe -OPh, -OCH₂Ph, -Ph ou -CH₂Ph.

30. Composés selon l'une des revendications 1 à 23, **caractérisés en ce que** Ar représente un groupe naphtyle.

31. Composés selon l'une des revendications 1 à 23, **caractérisés en ce que** Ar représente le groupe

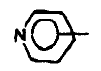

32. Composés selon l'une des revendications 1 à 23, **caractérisés en ce que** Ar représente le groupe

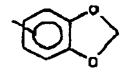

33. Composés selon l'une des revendications précédentes, **caractérisés en ce qu'**ils sont choisis parmi les :
- chlorhydrate du (S) 3-amino 3-phénoxy 1-propanethiol
- chlorhydrate du (S) 2-amino 3-benzyloxy 1-propanethiol
- chlorhydrate du (R) 2-amino 3-benzyloxy 1-propanethiol
- chlorhydrate du (2S,3R) 2-amino 3-méthyl 3-benzyloxy 1-propanethiol
- chlorhydrate du (2R,3S) 2-amino 3-méthyl 3-benzyloxy 1-propanethiol
- chlorhydrate du (S) 2-amino 3-benzylthio 1-propanethiol
- chlorhydrate du (R,S) 2-amino 7-phényl 1-heptanethiol
- chlorhydrate du (R,S) 2-amino 2-methyl 6 phénoxy 1-hexanethiol
- chlorhydrate du (S) 2-amino 3-(4-benzylphenoxy)-1-propanethiol
- chlorhydrate du (R,S) 2-amino 4-phényl 1-butanethiol
- chlorhydrate du (R,S) 2-amino 6-phénoxy 1-hexanethiol
- chlorhydrate du (R,S) 2-amino 7-phénoxy 1-heptanethiol
- chlorhydrate du (R,S) 2-amino 7-(4-méthoxy phénoxy) 1-heptanethiol
- chlorhydrate du (R,S) 2-amino 6-(4-méthoxy phénoxy) 1-hexanethiol
- chlorhydrate du (R,S) 2-amino 6-(4-méthyl phénoxy)-1-hexanethiol
- chlorhydrate du (R,S) 2-amino 6-(3-méthyl phénoxy) 1-hexenethiol
- chlorhydrate du (R,S) 2-amino 6-(3-méthoxy phénoxy) 1-hexanethiol
- chlorhydrate du (R,S) 2-amino 6-(4-chloro phénoxy)-1-hexanethiol
- chlorhydrate du (R,S) 2-amino 6-(4-bromo phénoxy)-1-hexanethiol
- chlorhydrate du (R,S) 2-amino 6-(4-fluoro phénoxy)-1-hexanethiol
- chlorhydrate du (R,S) 2-amino 6-(2-méthoxy phénoxy) 1-hexanethiol
- chlorhydrate du (R,S) 2-amino 6-phénylthio 1-hexanethiol
- chlorhydrate du (R,S) 2-amino 6-(3,4-dioxyméthylène phénoxy)-1-hexanethiol
- chlorhydrate du (R,S) 2-amino 6-(4-éthoxy phénoxy)-1-hexanethiol
- chlorhydrate du (R,S) 2-amino 6-(4-éthyl phénoxy)-1-hexanethiol
- chlorhydrate du (R,S) 2-amino 5-phényl-1-pentanethiol
- chlorhydrate du (R,S) 2-amino 4-phénoxy 1-butanethiol
- chlorhydrate du (R,S) 2-amino 6-phényl 1-hexanethiol
- chlorhydrate du (R,S) 2-amino 4-phenylthio 1-butanethiol
- chlorhydrate du (R,S) 2-amino 6-(2,6-diméthyl phénoxy) 1-hexanethiol
- chlorhydrate du (R,S) 2-amino 4-benzylthio 1-butanethiol
- chlorhydrate du (R,S) 2-amino 5-phénoxy 1-pentanethiol
- chlorhydrate du (R,S) 3-amino 6-(2-méthylphénoxy) 1-hexanethiol
- chlorhydrate du (R,S) 2-amino 6-(4-phénoxyphénoxy) 1-hexanethiol
- chlorhydrate du (R,S) 2-amino 6-(4-carboxyphénoxy) 1-hexanethiol
- chlorhydrate du (R,S) 2-amino 6-(4-cyano phénoxy) 1-hexanethiol
- chlorhydrate du [2(R,S)-3(R,S)] 2-amino 3-méthyl 6-phénoxy 1-hexanethiol
- chlorhydrate du (R,S) 2-amino 6-(4-phényl phénoxy) 1-hexanethiol
- chlorhydrate du (R,S) 2-amino 6-(4-benzyloxy phénoxy) 1-hexanethiol
- chlorhydrate du (R,S) 2-amino 6-(2-naphtyloxy) 1-hexanethiol
- chlorhydrate du (R,S) 2-amino 6-(1-naphtyloxy) 1-hexanethiol
- chlorhydrate du (R,S) 2-amino 8-phénoxy 1-octanethiol
- chlorhydrate du (R,S) 2-amino 9-phénoxy 1-nonanethiol
- chlorhydrate du (R,S) 2-amino 6-(3-trifluoro méttryl phénoxy) 1-hexanethiol
- chlorhydrate du (R,S) 2-amino 6-(3-fluoro phénoxy) 1-hexanethiol.
- chlorhydrate du (R,S) 2-amino 6-(2,4-difluoro phénoxy) 1-hexanethiol
- chlorhydrate du (R,S) 2-amino 6-(2-fluoro phénoxy) 1-hexanethiol
- chlorhydrate du (R,S) 2-amino 6-pentafluoro phénoxy 1-hexanethiol
- chlorhydrate du (R,S) 2-amino 6-(4-nitro phénoxy) 1-hexanethiol
- chlorhydrate du (R,S) 2-amino 8-phényl 1-octanethiol
- chlorhydrate du (R,S) 3-amino 6-(3,5-diméthoxy phénoxy) 1-hexanethiol
- chlorhydrate du (R,S) 2-amino 6-(4-butoxy phénoxy) 1-hexanethiol
- chlorhydrate du (R,S) 2-amino 6-(4,5-dichloro phénoxy)1-hexanethiol
- chlorhydrate du (R,S) 2-amino 6-(2-pyridoxy) 1-hexanethiol
- chlorhydrate du (R,S) 2-amino 6-(3-cyano phénoxy) 1-hexanethiol
- chlorhydrate du (R,S) 2-amino 5-(4-benzyl phénoxy) 1-pentanethiol
- chlorhydrate du (R,S) 2-amino 6-(3-chloro phénoxy) 1-hexanethiol
- chlorhydrate du (R,S) 2-amino 7-(4-cyano phénoxy) 1-heptanethiol
- chlorhydrate du (R,S) 2-amino 5-(4-cyano phénoxy) 1-pentanethiol
- chlorhydrate du (R,S) 2-amino 6-(3-éthyl phénoxy) 1-hexanethiol
- chlorhydrate du (R,S) 2-amino 6-(4-trifluorométhyl phénoxy) 1-hexanethiol
- chlorhydrate du (R,S) 2-amino 7-(4-benzylphénoxy) 1-heptanethiol
- chlorhydrate du (R,S) 2-amino 6-(4-benzylphénoxy) 1-hexanethiol
- chlorhydrate du (R,S) 2-amino 5-(3-éthyl phénoxy) 1-pentanethiol
- chlorhydrate du (R,S) 2-amino 5-(4-méthyl phénoxy) 1-pentanethiol
- chlorhydrate du (R,S) 2-amiao 6-(4-acétamido phénoxy) 1-hexanethiol
- chlorhydrate du (R,S) 2-amino 6-(4-iodo phénoxy) 1-hexanethiol
- chlorhydrate du (R,S) 2-amino 6-(4-propoxy phénoxy) 1-hexanethiol
- chlorhydrate du (R,S) 2-amino 5-(4-benzoyl phenoxy) 1-pentanethiol
- chlorhydrate du (R,S) 2-amino 5-(4-éthoxy phenoxy) 1-pentanethiol
- chlorhydrate du (R,S) 2-amino 7-(4-éthoxy phénoxy) 1-heptanethiol
- chlorhydrate du (+) 2-amino 6-(4-éthoxy phénoxy) 1-hexanethiol
- chlorhydrate du (-) 2-amino 6-(4-éthoxy phénoxy) 1-hexanethiol
- chlorhydrate du (+) 2-amino 6-phénoxy 1-hexanethiol
- chlorhydrate du (-) 2-amino 6-phénoxy 1-hexanethiol.

34. Composés selon l'une des revendications 1 à 8, 10, 11, 12, 14 à 32 et 33, **caractérisés en ce que** R³ représente le groupe 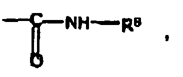 R⁶ ayant la signification donnée à la revendication 1.

35. Composés selon l'une des revendications 1 à 8, 10, 11, 12, 14 à 32 et 33, **caractérisés en ce que** R³ représente le groupe 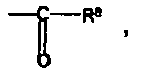 R⁶ ayant la signification donnée à la revendication 1.

36. Composés selon l'une des revendications 8, 10, 11, 12, 14 à 32 et 33, **caractérisés en ce que** R³ représente le groupe

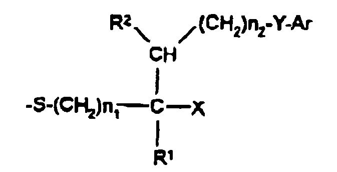

37. Composés selon l'une des revendications 34 à 36, **caractérisés en ce qu'**ils sont choisis parmi les :
- dichlorhydrate du 1,1-dithio bis (2-(R,S)-amino 6-phénoxy hexane)
- bromhydrate du (R,S) 2-amino 6-phénoxy 1-S-acétylthio hexane
- dichlorhydrate du 1,1-dithivbis (2-(+)-amino-6-phénoxy-hexane) (isomère A)
- dichlorhydrate du 1,1-dithiobis (2-(-)-amino-6-phénoxy-hexane) (isomère B)
- dichlorhydrate du 1,1-dithiobis (2-(R,S)-amino-6-(4-éthoxyphénoxy)-1 hexane)
- dichlorhydrate du 1,1-dithiobis (2-(+)-amino-6-(4-éthoxyphénoxy)-1 hexane) (isomère A)
- dichlorhydrate du 1,1-dithiobis (2-(-)-amino-6-(4-éthoxyphénoxy)-1-hexane) (isomère B)
- dichlorhydrate du 1,1-dithiobis (2-(R,S)-amino-6-(4-acétamidophénoxyl)-1-hexane
- dichlorhydrate du 1.1-dithiobis (2-(R,S)-amino-6-(4-cyanophénoxy)-1 hexane

38. Composition pharmaceutique **caractérisée en ce qu'**elle comprend à titre de principe actif une quantité thérapeutiquement efficace d'un composé selon l'une des revendications 1 à 37, en combinaison avec un véhicule ou excipient physiologiquement acceptable.

39. Utilisation d'un composé de formule générale (I) 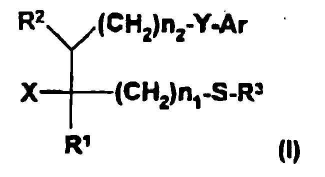
dans laquelle:
• X représente -NH₂;
• R¹ et R² sont indépendamment choisis parmi les groupes suivants :
i) un atome d'hydrogène
ii) un groupe alkyle à chaîne linéaire ou ramifiée contenant de 1 à 6 atomes de carbone ;
• n₁ varie de 1 à 4
• n₂ varie de 0 à 10
• R³ est choisi parmi les groupes suivants :
i) un atome d'hydrogène
ii) 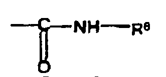
iii) 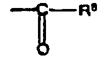
iv) 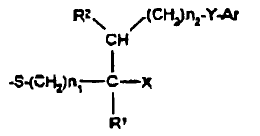
• Y est choisi parmi les groupes suivants :
i) -O-
ii) -CH₂-
iii) -S-
iv) -OCH₂-
v) -SCH₂-
vi) -NH
• Ar est choisi parmi les groupes suivants :
i) un groupe phényle, non substitué, ou mono ou polysubstitué avec des substituants choisis parmi les atomes d'halogène et les groupes CF₃, alkyle à chaîne linéaire ou ramifiée contenant de 1 à 6 atomes de carbone, alcoxy à chaîne linéaire ou ramifiée contenant de 1 à 6 atomes de carbone, NH₂, NO₂, CN, OH, CO₂H, OPh, OCH₂Ph, SMe, SEt, Ph, CH₂Ph et NHCOR⁷ où R⁷ est un groupe alkyle à chaîne linéaire ou ramifiée contenant de 1 à 6 atomes de carbone,
ii) 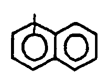
iii) 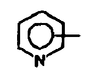
iv) 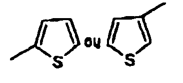
v) 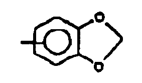
vi) 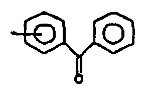
• R⁶ représente un groupe alkyle à chaîne linéaire ou ramifiée contenant de 1 à 6 atomes de carbone ou un groupe phényle pour la fabrication d'un médicament destiné à inhiber l'activité de la LTA₄.

40. Utilisation d'un composé de formule (1) selon la revendication 39 pour la fabrication d'un médicament destiné à un traitement anti-inflammatoire.

41. Utilisation d'un composé de formule (I) selon la revendication 39 pour la fabrication d'un médicament destiné à un traitement anti-arthritique.

42. Utilisation d'un composé de formule (I) selon la revendication 39 pour la fabrication d'un médicament destiné à un traitement anti-psoriasique.

43. Utilisation d'un composé de formule (I) selon la revendication 39 pour la fabrication d'un médicament destiné à un traitement hépato-protecteur.

44. Utilisation d'un composé de formule (I) selon la revendication 39 pour la fabrication d'un médicament destiné à un traitement antimitotique.

45. Utilisation d'un composé de formule (I) selon la revendication 39 pour la fabrication d'un médicament destiné à un traitement d'une surproduction de LTB₄ induite notamment par un inhibiteur de cyclooxygénase.

46. Composition pharmaceutique, **caractérisée en ce qu'**elle comprend, à titre de principe actif, une quantité thérapeutiquement efficace d'un composé de formule (1) selon la revendication 39 et une quantité thérapeutiquement efficace d'un inhibiteur de cyclooxygénase.

47. Composition selon la revendication 46 comprenant en outre en véhicule ou excipient physiologiquement acceptable.

## Patentansprüche

1. Verbindungen der folgenden Formel (I): 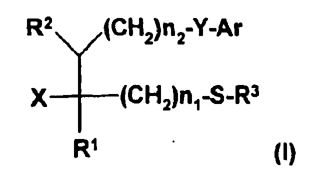
in der:
● X -NH₂ bedeutet;
● R¹ und R² unabhängig voneinander ausgewählt sind aus den folgenden Gruppen:
i) dem Wasserstoffatom,
ii) einer Alkylgruppe mit gerader oder verzweigter Kette enthaltend 1 bis 6 Kohlenstoffatome;
● n₁ einen Wert von 1 bis 4 besitzt,
● n₂ einen Wert von 0 bis 10 besitzt,
● R³ aus den folgenden Gruppen ausgewählt ist:
i) dem Wasserstoffatom
ii) 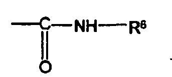
iii) 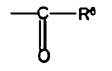
iv) 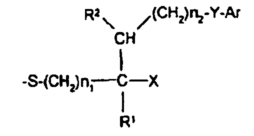
● Y ausgewählt ist aus den folgenden Gruppen:
i) -O-
ii) -CH₂-
iii) -S-
iv) -OCH₂-
v) -SCH₂-
● Ar ausgewählt ist aus den folgenden Gruppen:
i) eine Phenylgruppe, die nichtsubstituiert oder einfach oder mehrfach substituiert ist mit Substituenten ausgewählt aus Halogenatomen und den Gruppen CF₃, Alkyl mit gerader oder verzweigter Kette enthaltend 1 bis 6 Kohlenstoffatome, Alkoxy mit gerader oder verzweigter Kette enthaltend 1 bis 6 Kohlenstoffatome, NH₂, NO₂, CN, OH, CO₂H, OPh, OCH₂Ph, SMe, SEt, Ph, CH₂Ph und NHCOR⁷, worin R⁷ eine Alkylgruppe mit gerader oder verzweigter Kette enthaltend 1 bis 6 Kohlenstoffatome, darstellt,
ii) 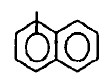
iii) 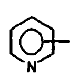
iv) 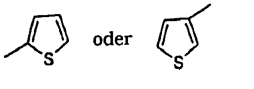
v) 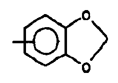
vi) 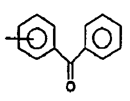
● R⁶ eine Alkylgruppe mit gerader oder verzweigter Kette enthaltend 1 bis 6 Kohlenstoffatome oder eine Phenylgruppe bedeutet;
mit Ausnahme der Verbindungen der Formel I, in der X -NH₂ bedeutet, R¹ und R² jeweils ein Wasserstoffatom darstellen, n₁ = 1, n₂ = 0, Y eine Gruppe -OCH₂- oder -SCH₂- bedeutet, Ar eine Phenylgruppe darstellt und R³ ein Wasserstoffatom oder die entsprechenden Reste iv) bedeutet,
sowie deren Isomere, Diastereoisomere und Enantiomere und deren pharmazeutisch annehmbare Salze.

2. Verbindungen der allgemeinen Formel (I): 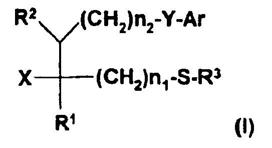
in der:
● X -NH₂ bedeutet;
● R¹ und R² unabhängig voneinander ausgewählt sind aus den folgenden Gruppen:
i) dem Wasserstoffatom,
ii) einer Alkylgruppe mit gerader oder verzweigter Kette enthaltend 1 bis 6 Kohlenstoffatome,
● n₁ einen Wert von 1 bis 4 besitzt
● n₂ einen Wert von 0 bis 10 besitzt
● R³ ausgewählt ist aus den folgenden Gruppen:
i) dem Wasserstoffatom
ii) 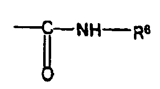
iii) 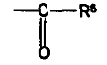
iv) 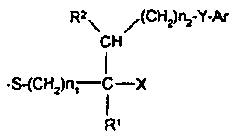
● Ar ausgewählt ist aus den folgenden Gruppen:
i) einer Phenylgruppe, die nicht substituiert ist oder einfach oder mehrfach substituiert ist mit Substituenten ausgewählt aus Halogenatomen und den Gruppen CF₃, Alkyl mit gerader oder verzweigter Kette enthaltend 1 bis 6 Kohlenstoffatome, Alkoxy mit gerader oder verzweigter Kette enthaltend 1 bis 6 Kohlenstoffatome, NH₂, NO₂, CN, OH, CO₂H, OPh, OCH₂Ph, SMe, SEt, Ph, CH₂Ph und NHCOR⁷, worin R⁷ eine Alkylgruppe mit gerader oder verzweigter Kette enthaltend 1 bis 6 Kohlenstoffatome darstellt,
ii) 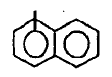
iii) 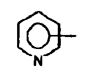
iv) 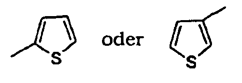
v) 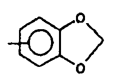
vi) 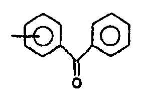
● R⁶ eine Alkylgruppe mit gerader oder verzweigter Kette enthaltend 1 bis 6 Kohlenstoffatome oder eine Phenylgruppe bedeutet
und Y ausgewählt ist aus
i) -O-
ii) -CH₂-
iii) -S-.

3. Verbindungen nach Anspruch 1 oder 2, **dadurch gekennzeichnet, daß** R¹ ein Wasserstoffatom bedeutet.

4. Verbindungen nach Anspruch 1 oder 2, **dadurch gekennzeichnet, daß** R¹ von Wasserstoff verschieden ist.

5. Verbindungen nach einem der Ansprüche 1 bis 4, **dadurch gekennzeichnet, daß** n₁ 1 bedeutet.

6. Verbindungen nach einem der Ansprüche 1 bis 4, **dadurch gekennzeichnet, daß** n₁ von 1 verschieden ist.

7. Verbindungen nach einem der Ansprüche 1 bis 5, **dadurch gekennzeichnet, daß** sie der folgenden Formel (II) entsprechen: 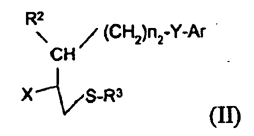
in der X, R², R³, Y, Ar und n₂ die in Anspruch 1 oder 2 angegebenen Bedeutungen besitzen.

8. Verbindungen nach einem der Ansprüche 1 bis 7, **dadurch gekennzeichnet, daß** X NH₂ bedeutet.

9. Verbindungen nach einem der Ansprüche 1 bis 8, **dadurch gekennzeichnet, daß** R³ ein Wasserstoffatom bedeutet.

10. Verbindungen nach einem der Ansprüche 1 bis 9, **dadurch gekennzeichnet, daß** R² von dem Wasserstoffatom verschieden ist.

11. Verbindungen nach Anspruch 10, **dadurch gekennzeichnet, daß** R² eine Methylgruppe bedeutet.

12. Verbindungen nach einem der Ansprüche 1 bis 9, **dadurch gekennzeichnet, daß** R² ein Wasserstoffatom bedeutet.

13. Verbindungen nach einem der Ansprüche 1 bis 12, **dadurch gekennzeichnet, daß** sie der folgenden Formel (III) entsprechen: 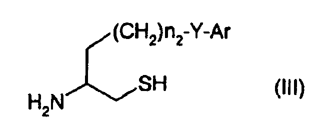
in der Y, Ar und n₂ die in Anspruch 1 oder 2 angegebenen Bedeutungen besitzen.

14. Verbindungen nach einem der Ansprüche 1 bis 13, **dadurch gekennzeichnet, daß** n₂ Null bedeutet.

15. Verbindungen nach einem der Ansprüche 1 bis 13, **dadurch gekennzeichnet, daß** n₂ einen Wert von 1 bis 4 besitzt.

16. Verbindungen nach Anspruch 15, worin n₂ den Wert von 2 bis 4 besitzt.

17. Verbindungen nach einem der Ansprüche 1 bis 13, **dadurch gekennzeichnet, daß** n₂ 3 bedeutet.

18. Verbindungen nach einem der Ansprüche 1 bis 13, **dadurch gekennzeichnet, daß** n₂ größer ist als 4.

19. Verbindungen nach einem der Ansprüche 1 bis 18, **dadurch gekennzeichnet, daß** Y ein Sauerstoffatom bedeutet.

20. Verbindungen nach einem der Ansprüche 1 bis 18, **dadurch gekennzeichnet, daß** Y -CH₂- bedeutet.

21. Verbindungen nach einem der Ansprüche 1 bis 18, **dadurch gekennzeichnet, daß** Y ein Schwefelatom bedeutet.

22. Verbindungen nach einem der Ansprüche 1 und 3 bis 18, **dadurch gekennzeichnet, daß** Y -OCH₂- oder -SCH₂- bedeutet.

23. Verbindungen nach einem der Ansprüche 1 bis 18, **dadurch gekennzeichnet, daß** n₂ Null bedeutet und Y ausgewählt ist aus -O-, -S-, -OCH₂- und -SCH₂-.

24. Verbindungen nach einem der Ansprüche 1 bis 23, **dadurch gekennzeichnet, daß** Ar eine nichtsubstituierte Phenylgruppe bedeutet.

25. Verbindungen nach einem der Ansprüche 1 bis 23, **dadurch gekennzeichnet, daß** Ar eine substituierte Phenylgruppe bedeutet.

26. Verbindungen nach Anspruch 25, worin Ar eine monosubstituierte Phenylgruppe bedeutet.

27. Verbindungen nach einem der Ansprüche 1 bis 23, **dadurch gekennzeichnet, daß** Ar eine Phenylgruppe bedeutet, die einfach oder mehrfach substituiert ist durch eine Gruppe ausgewählt aus Halogenatomen, CF₃, Alkyl mit gerader oder verzweigter Kette enthaltend 1 bis 6 Kohlenstoffatome, O(Alkyl mit gerader oder verzweigter Kette enthaltend 1 bis 6 Kohlenstoffatome). NO₂, CN, CO₂H, OPh. OCH₂Ph, Ph und CH₂Ph.

28. Verbindungen nach einem der Ansprüche 1 bis 23, **dadurch gekennzeichnet, daß** Ar eine Phenylgruppe bedeutet, die einfach oder mehrfach substituiert ist durch ein Halogenatom, eine Alkylgruppe mit gerader oder verzweigter Kette enthaltend 1 bis 6 Kohlenstoffatome oder -O(Alkyl mit gerader oder verzweigter Kette enthaltend 1 bis 6 Kohlenstoffatome).

29. Verbindungen nach einem der Ansprüche 1 bis 23, **dadurch gekennzeichnet, daß** Ar eine Phenylgruppe bedeutet, die durch eine Gruppe -OPh, -OCH₂Ph, -Ph oder -CH₂Ph substituiert ist.

30. Verbindungen nach einem der Ansprüche 1 bis 23, **dadurch gekennzeichnet, daß** Ar eine Naphthylgruppe bedeutet.

31. Verbindungen nach einem der Ansprüche 1 bis 23, **dadurch gekennzeichnet, daß** Ar die Gruppe 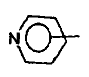
bedeutet.

32. Verbindungen nach einem der Ansprüche 1 bis 23, **dadurch gekennzeichnet, daß** Ar die Gruppe 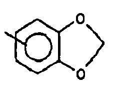
bedeutet.

33. Verbindungen nach einem der vorhergehenden Ansprüche, **dadurch gekennzeichnet, daß** sie ausgewählt sind aus:
- (S)-2-Amino-3-phenoxy-1-propanthiol, Hydrochlorid
- (S)-2-Amino-3-benzyloxy-1-propanthiol, Hydrochlorid
- (R)-2-Amino-3-benzyloxy-1-propanthiol, Hydrochlorid
- (2S,3R)-2-Amino-3-methyl-3-benzyloxy-1-propanthiol, Hydrochlorid
- (2R,3S)-2-Amino-3-methyl-3-benzyloxy-1-propanthiol, Hydrochlorid
- (S)-2-Amino-3-benzylthio-1-propanthiol, Hydrochlorid
- (R,S)-2-Amino-7-phenyl-1-heptanthiol, Hydrochlorid
- (R,S)-2-Amino-2-methyl-6-phenoxy-1-hexanthiol, Hydrochlorid
- (S)-2-Amino-3-(4-benzylphenoxy)-1-propanthiol, Hydrochlorid
- (R,S)-2-Amino-4-phenyl-1-butanthiol, Hydrochlorid
- (R,S)-2-Amino-6-phenoxy-1-hexanthiol, Hydrochlorid
- (R,S)-2-Amino-7-phenoxy-1-heptanthiol, Hydrochlorid
- (R,S)-2-Amino-7-(4-methoxyphenoxy)-1-heptanthiol, Hydrochlorid
- (R,S)-2-Amino-6-(4-methoxyphenoxy)-1-hexanthiol, Hydrochlorid
- (R,S)-2-Amino-6-(4-methylphenoxy)-1-hexanthiol, Hydrochlorid
- (R,S)-2-Amino-6-(3-methylphenoxy)-1-hexanthiol, Hydrochlorid
- (R,S)-2-Amino-6-(3-methoxyphenoxy)-1-hexanthiol, Hydrochlorid
- (R,S)-2-Amino-6-(4-chlorphenoxy)-1-hexanthiol, Hydrochlorid
- (R,S)-2-Amino-6-(4-bromphenoxy)-1-hexanthiol, Hydrochlorid
- (R,S)-2-Amino-6-(4-fluorphenoxy)-1-hexanthiol, Hydrochlorid
- (R,S)-2-Amino-6-(2-methoxyphenoxy)-1-hexanthiol, Hydrochlorid
- (R,S)-2-Amino-6-phenylthio-1-hexanthiol, Hydrochlorid
- (R,S)-2-Amino-6-(3,4-dioxymethylenphenoxy)-1-hexanthiol, Hydrochlorid
- (R,S)-2-Amino-6-(4-ethoxyphenoxy)-1-hexanthiol, Hydrochlorid
- (R,S)-2-Amino-6-(4-ethylphenoxy)-1-hexanthiol, Hydrochlorid
- (R,S)-2-Amino-5-phenyl-1-pentanthiol, Hydrochlorid
- (R,S)-2-Amino-4-phenoxy-1-butanthiol, Hydrochlorid
- (R,S)-2-Amino-6-phenyl-1-hexanthiol, Hydrochlorid
- (R,S)-2-Amino-4-phenylthio-1-butanthiol, Hydrochlorid
- (R,S)-2-Amino-6-(2,6-dimethylphenoxy)-1-hexanthiol, Hydrochlorid
- (R,S)-2-Amino-4-benzylthio-1-butanthiol, Hydrochlorid
- (R,S)-2-Amino-5-phenoxy-1-pentanthiol, Hydrochlorid
- (R,S)-2-Amino-6-(2-methylphenoxy)-1-hexanthiol, Hydrochlorid
- (R,S)-2-Amino-6-(4-phenoxyphenoxy)-1-hexanthiol, Hydrochlorid
- (R,S)-2-Amino-6-(4-carboxyphenoxy)-1-hexanthiol, Hydrochlorid
- (R,S)-2-Amino-6-(4-cyanophenoxy)-1-hexanthiol, Hydrochlorid
- [2(R,S)-3(R,S)]-2-Amino-3-methyl-6-phenoxy-1-hexanthiol, Hydrochlorid
- (R,S)-2-Amino-6-(4-phenylphenoxy)-1-hexanthiol, Hydrochlorid
- (R,S)-2-Amino-6-(4-benzyloxyphenoxy)-1-hexanthiol, Hydrochlorid
- (R,S)-2-Amino-6-(2-naphthyloxy)-1-hexanthiol, Hydrochlorid
- (R,S)-2-Amino-6-(1-naphthyloxy)-1-hexanthiol, Hydrochlorid
- (R,S)-2-Amino-8-phenoxy-1-octanthiol, Hydrochlorid
- (R,S)-2-Amino-9-phenoxy-1-nonanthiol, Hydrochlorid
- (R,S)-2-Amino-6-(3-trifluormethylphenoxy)-1-hexanthiol, Hydrochlorid
- (R,S)-2-Amino-6-(3-fluorphenoxy)-1-hexanthiol, Hydrochlorid
- (R,S)-2-Amino-6-(2,4-difluorphenoxy)-1-hexanthiol. Hydrochlorid
- (R,S)-2-Amino-6-(2-fluorphenoxy)-1-hexanthiol, Hydrochlorid
- (R,S)-2-Amino-6-pentafluorphenoxy-1-hexanthiol, Hydrochlorid
- (R,S)-2-Amino-6-(4-nitrophenoxy)-1-hexanthiol, Hydrochlorid
- (R,S)-2-Amino-8-phenyl-1-octanthiol, Hydrochlorid
- (R,S)-2-Amino-6-(3,5-dimethoxyphenoxy)-1-hexanthiol, Hydrochlorid
- (R,S)-2-Amino-6-(4-butoxyphenoxy)-1-hexanthiol, Hydrochlorid
- (R,S)-2-Amino-6-(4,5-dichlorphenoxy)-1-hexanthiol, Hydrochlorid
- (R,S)-2-Amino-6-(2-pyridoxy)-1-hexanthiol, Hydrochlorid
- (R,S)-2-Amino-6-(3-cyanophenoxy)-1-hexanthiol, Hydrochlorid
- (R,S)-2-Amino-5-(4-benzylphenoxy)-1-pentanthiol, Hydrochlorid
- (R,S)-2-Amino-6-(3-chlorphenoxy)-1-hexanthiol, Hydrochlorid
- (R,S)-2-Amino-7-(4-cyanophenoxy)-1-heptanthiol, Hydrochlorid
- (R,S)-2-Amino-5-(4-cyanophenoxy)-1-pentanthiol, Hydrochlorid
- (R,S)-2-Amino-6-(3-ethylphenoxy)-1-hexanthiol, Hydrochlorid
- (R,S)-2-Amino-6-(4-trifluormethylphenoxy)-1-hexanthiol, Hydrochlorid
- (R,S)-2-Amino-7-(4-benzylphenoxy)-1-heptanthiol, Hydrochlorid
- (R,S)-2-Amino-6-(4-benzylphenoxy)-1-hexanthiol, Hydrochlorid
- (R,S)-2-Amino-5-(3-ethylphenoxy)-1-pentanthiol, Hydrochlorid
- (R,S)-2-Amino-5-(4-methylphenoxy)-1-pentanthiol, Hydrochlorid
- (R,S)-2-Amino-6-(4-acetamidophenoxy)-1-hexanthiol, Hydrochlorid
- (R,S)-2-Amino-6-(4-iodphenoxy)-1-hexanthiol, Hydrochlorid
- (R,S)-2-Amino-6-(4-propoxyphenoxy)-1-hexanthiol, Hydrochlorid
- (R,S)-2-Amino-5-(4-benzoylphenoxy)-1-pentanthiol, Hydrochlorid
- (R,S)-2-Amino-5-(4-ethoxyphenoxy)-1-pentanthiol, Hydrochlorid
- (R,S)-2-Amino-7-(4-ethoxyphenoxy)-1-heptanthiol, Hydrochlorid
- (+)-2-Amino-6-(4-ethoxyphenoxy)-1-hexanthiol, Hydrochlorid
- (-)-2-Amino-6-(4-ethoxyphenoxy)-1-hexanthiol, Hydrochlorid
- (+)-2-Amino-6-phenoxy-1-hexanthiol, Hydrochlorid
- (-)-2-Amino-6-phenoxy-1-hexanthiol, Hydrochlorid.

34. Verbindungen nach einem der Ansprüche 1 bis 8, 10, 11, 12, 14 bis 32 und 33, **dadurch gekennzeichnet, daß** R³ die Gruppe 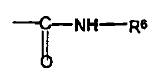 bedeutet, worin R⁶ die in Anspruch 1 angegebenen Bedeutungen besitzt.

35. Verbindungen nach einem der Ansprüche 1 bis 8, 10, 11, 12, 14 bis 32 und 33, **dadurch gekennzeichnet, daß** R³ die Gruppe 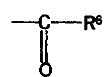 bedeutet, worin R⁶ die in Anspruch 1 angegebenen Bedeutungen besitzt.

36. Verbindungen nach einem der Ansprüche 8, 10, 11, 12, 14 bis 32 und 33, **dadurch gekennzeichnet, daß** R³ die Gruppe 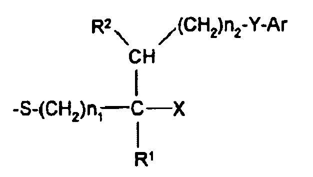
bedeutet.

37. Verbindungen nach einem der Ansprüche 34 bis 36, **dadurch gekennzeichnet, daß** sie ausgewählt sind aus:
- 1,1-Dithiobis(2-(R,S)-amino-6-phenoxyhexan), Dihydrochlorid
- (R,S)-2-Amino-6-phenoxy-1-S-acetylthiohexan, Hydrobromid
- 1,1-Dithiobis(2-(+)-amino-6-phenoxyhexan), Dihydrochlorid (Isomeres A)
- 1,1-Dithiobis(2-(-)-amino-6-phenoxyhexan), Dihydrochlorid (Isomeres B)
- 1,1-Dithiobis(2-(R,S)-amino-6-(4-ethoxyphenoxy)-1-hexan), Dihydrochlorid
- 1,1-Dithiobis(2-(+)-amino-6-(4-ethoxyphenoxy)-1-hexan), Dihydrochlorid (Isomeres A)
- 1,1-Dithiobis(2-(-)-amino-6-(4-ethoxyphenoxy)-1-hexan), Dihydrochlorid (Isomeres B)
- 1,1-Dithiobis(2-(R,S)-amino-6-(4-acetamidophenoxy)-1-hexan, Dihydrochlorid
- 1,1-Dithiobis(2-(R,S)-amino-6-(4-cyanophenoxy)-1-hexan, Dihydrochlorid.

38. Pharmazeutische Zubereitung, **dadurch gekennzeichnet, daß** sie als Wirkstoff eine therapeutisch wirksame Menge einer Verbindung nach einem der Ansprüche 1 bis 37 in Kombination mit einem physiologisch annehmbaren Trägermaterial oder Hilfsstoff enthält.

39. Verwendung einer Verbindung der allgemeinen Formel (I) 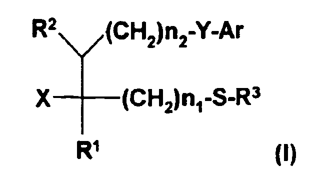
in der:
● X -NH₂ bedeutet;
● R¹ und R² unabhängig voneinander ausgewählt sind aus den folgenden Gruppen:
i) dem Wasserstoffatom,
ii) einer Alkylgruppe mit gerader oder verzweigter Kette enthaltend 1 bis 6 Kohlenstoffatome;
● n₁ einen Wert von 1 bis 4 besitzt,
● n₂ einen Wert von 0 bis 10 besitzt,
● R³ aus den folgenden Gruppen ausgewählt ist:
i) dem Wasserstoffatom
ii) 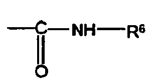
iii) 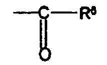
iv) 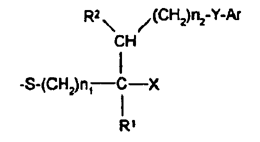
● Y ausgewählt ist aus den folgenden Gruppen:
i) -O-
ii) -CH₂-
iii) -S-
iv) -OCH₂-
v) -SCH₂-
● Ar ausgewählt ist aus den folgenden Gruppen:
i) eine Phenylgruppe, die nichtsubstituiert oder einfach oder mehrfach substituiert ist mit Substituenten ausgewählt aus Halogenatomen und den Gruppen CF₃, Alkyl mit gerader oder verzweigter Kette enthaltend 1 bis 6 Kohlenstoffatome, Alkoxy mit gerader oder verzweigter Kette enthaltend 1 bis 6 Kohlenstoffatome, NH₂, NO₂, CN, OH, CO₂H, OPh, OCH₂Ph, SMe, SEt, Ph, CH₂Ph und NHCOR⁷, worin R⁷ eine Alkylgruppe mit gerader oder verzweigter Kette enthaltend 1 bis 6 Kohlenstoffatome darstellt,
ii) 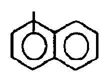
iii) 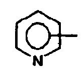
iv) 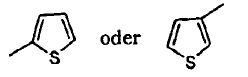
v) 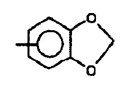
vi) 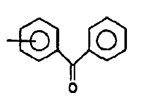
● R⁶ eine Alkylgruppe mit gerader oder verzweigter Kette enthaltend 1 bis 6 Kohlenstoffatome oder eine Phenylgruppe bedeutet, für die Herstellung eines Arzneimittels zur Inhibierung der Aktivität von LTA₄.

40. Verwendung einer Verbindung der Formel (I) nach Anspruch 39 für die Herstellung eines Arzneimittels für eine intiinflammatorische Behandlung.

41. Verwendung einer Verbindung der Formel (I) nach Anspruch 39 für die Herstellung eines Arzneimittels für eine antiarthritische Behandlung.

42. Verwendung einer Verbindung der Formel (I) nach Anspruch 39 für die Herstellung eines Arzneimittels für eine Antipsoriasis-Behandlung.

43. Verwendung einer Verbindung der Formel (I) nach Anspruch 39 für die Herstellung eines Arzneimittels für eine Leberschutz-Behandlung.

44. Verwendung einer Verbindung der Formel (I) nach Anspruch 39 für die Herstellung eines Arzneimittels für eine antimitotische Behandlung.

45. Verwendung einer Verbindung der Formel (I) nach Anspruch 39 für die Herstellung eines Arzneimittels zur Behandlung einer insbesondere durch einen Cyclooxygenase-Inhibitor induzierten Überproduktion von LTB₄.

46. Pharmazeutische Zubereitung, **dadurch gekennzeichnet, daß** sie als Wirkstoff eine therapeutisch wirksame Menge einer Verbindung der Formel (I) nach Anspruch 39 und eine therapeutisch wirksame Menge eines Cyclooxygenase-Inhibitors umfaßt.

47. Zubereitung nach Anspruch 46, enthaltend zusätzlich ein physiologisch annehmbares Trägermaterial oder Hilfsmittel.

## Claims

1. Compounds corresponding to the following formula (I): 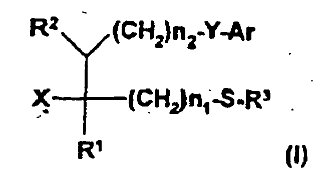
wherein:
• X denotes -NH₂;
• R¹ and R² are independently selected from among the following groups:
i) a hydrogen atom
ii) a straight-chain or branched alkyl group containing 1 to 6 carbon atoms;
• n₁ varies from 1 to 4
• n₂ varies from 0 to 10
• R³ is selected from among the following groups:
i) a hydrogen atom
ii) 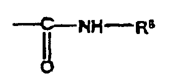
iii) 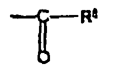
iv) 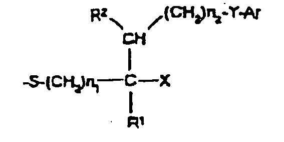
• Y is selected from among the following groups:
i) -O-
ii) -CH₂-
iii) -S-
iv) -OCH₂-
v) -SCH₂-
• Ar is selected from among the following groups:
i) a phenyl group which is unsubstituted or mono- or polysubstituted by substituents selected from among the halogen atoms and the groups CF₃, straight-chain or branched alkyl containing 1 to 6 carbon atoms, straight-chain or branched alkoxy containing 1 to 6 carbon atoms, NH₂, NO₂, CN, OH, CO₂H, OPh, OCH₂Ph, SMe, SEt, Ph, CH₂Ph and NHCOR⁷ where R⁷ is a straight-chain or branched alkyl group containing 1 to 6 carbon atoms,
ii) 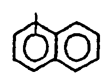
iii) 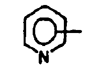
iv) 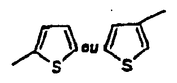
v) 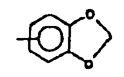
vi) 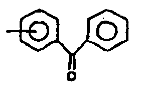
• R⁶ denotes a straight-chain or branched alkyl group containing 1 to 6 carbon atoms or a phenyl group;
with the exception of compounds of formula I wherein X is -NH₂, R¹ and R² each denote a hydrogen atom, n₁ = 1, n₂ = 0, Y is a group -OCH₂- or -SCH₂-, Ar is a phenyl group and R³ is a hydrogen atom or the corresponding radicals iv),
as well as the isomers, diastereoisomers and enantiomers thereof and the pharmaceutically acceptable salts thereof.

2. Compounds of general formula I 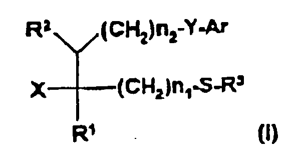
wherein:
• X denotes -NH₂
• R¹ and R² are independently selected from among the following groups:
i) a hydrogen atom
ii) a straight-chain or branched alkyl group containing 1 to 6 carbon atoms;
• n₁ varies from 1 to 4
• n₂ varies from 0 to 10
• R³ is selected from among the following groups:
i) a hydrogen atom
ii) 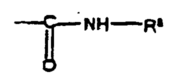
iii) 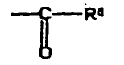
iv) 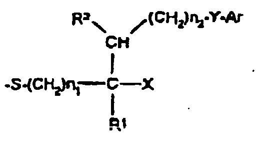
• Ar is selected from among the following groups:
i) a phenyl group which is unsubstituted or mono- or polysubstituted by substituents selected from among the halogen atoms and the groups CF₃, straight-chain or branched alkyl containing 1 to 6 carbon atoms, straight-chain or branched alkoxy containing 1 to 6 carbon atoms, NH₂, NO₂, CN, OH, CO₂H, OPh, OCH₂Ph, SMe, SEt, Ph, CH₂Ph and NHCOR⁷ where R⁷ is a straight-chain or branched alkyl group containing 1 to 6 carbon atoms,
ii) 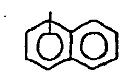
iii) 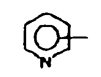
iv) 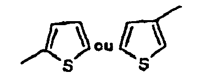
v) 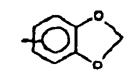
vi) 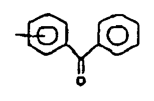
• R⁶ denotes a straight-chain or branched alkyl group containing 1 to 6 carbon atoms or a phenyl group;
and Y is selected from among
i) -O-
ii) -CH₂-
iii) -S-

3. Compounds according to claim 1 or 2, **characterised in that** R¹ denotes a hydrogen atom.

4. Compounds according to claim 1 or 2, **characterised in that** R¹ is different from hydrogen.

5. Compounds according to one of claims 1 to 4, **characterised in that** n₁ is equal to 1.

6. Compounds according to one of claims 1 to 4, **characterised in that** n₁ is different from 1.

7. Compounds according to one of claims 1 to 5, **characterised in that** they correspond to the following formula (II): 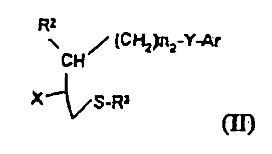
wherein X, R², R³, Y, Ar and n₂ have the meanings given in claim 1 or 2.

8. Compounds according to one of claims 1 to 7, **characterised in that** X denotes NH₂.

9. Compounds according to one of claims 1 to 8, **characterised in that** R³ denotes a hydrogen atom.

10. Compounds according to one of claims 1 to 9, **characterised in that** R² is different from a hydrogen atom.

11. Compounds according to claim 10, wherein R² denotes a methyl group.

12. Compounds according to one of claims 1 to 9, **characterised in that** R² is a hydrogen atom.

13. Compounds according to one of claims 1 to 12, **characterised in that** they correspond to the following formula (III): 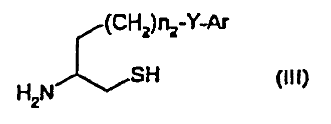
wherein Y, Ar and n₂ have the meanings given in claim 1 or 2.

14. Compounds according to one of claims 1 to 13, **characterised in that** n₂ is equal to zero.

15. Compounds according to one of claims 1 to 13, **characterised in that** n₂ varies from 1 to 4.

16. Compounds according to claim 15, wherein n₂ varies from 2 to 4.

17. Compounds according to one of claims 1 to 13, **characterised in that** n₂ is equal to 3.

18. Compounds according to one of claims 1 to 13, **characterised in that** n₂ is greater than 4.

19. Compounds according to one of claims 1 to 18, **characterised in that** Y denotes an oxygen atom.

20. Compounds according to one of claims 1 to 18, **characterised in that** Y denotes -CH₂-.

21. Compounds according to one of claims 1 to 18, **characterised in that** Y denotes a sulphur atom.

22. Compounds according to one of claims 1, 3 to 18, **characterised in that** Y denotes -OCH₂- or -SCH₂-.

23. Compounds according to one of claims 1 to 18, **characterised in that** n₂ is equal to zero and Y is selected from among -O-, -S-, -OCH₂- and -SCH₂-.

24. Compounds according to one of claims 1 to 23, **characterised in that** Ar denotes an unsubstituted phenyl group.

25. Compounds according to one of claims 1 to 23, **characterised in that** Ar denotes a substituted phenyl group.

26. Compounds according to claim 25, wherein Ar is a monosubstituted phenyl group.

27. Compounds according to one of claims 1 to 23, **characterised in that** Ar denotes a phenyl group mono- or polysubstituted by a group selected from among the halogen atoms, CF₃, straight-chain or branched alkyl containing 1 to 6 carbon atoms, O(straight-chain or branched alkyl containing 1 to 6 carbon atoms), NO₂, CN, CO₂H, OPh, OCH₂Ph, Ph and CH₂Ph.

28. Compounds according to one of claims 1 to 23, **characterised in that** Ar denotes a phenyl group mono- or polysubstituted by a halogen atom, a straight-chain or branched alkyl group containing 1 to 6 carbon atoms or -O(straight-chain or branched alkyl containing 1 to 6 carbon atoms).

29. Compounds according to one of claims 1 to 23, **characterised in that** Ar denotes a phenyl group substituted by a group -OPh, -OCH₂Ph, -Ph or -CH₂Ph.

30. Compounds according to one of claims 1 to 23, **characterised in that** Ar denotes a naphthyl group.

31. Compounds according to one of claims 1 to 23, **characterised in that** Ar denotes the group

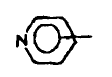

32. Compounds according to one of claims 1 to 23, **characterised in that** Ar denotes the group

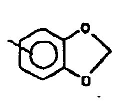

33. Compounds according to one of the preceding claims, **characterised in that** they are selected from among:
(S) 2-amino-3-phenoxy-1-propanethiol hydrochloride
(S) 2-amino-3-benzyloxy-1-propanethiol hydrochloride
(R) 2-amino-3-benzyloxy-1-propanethiol hydrochloride
(2S,3R) 2-amino-3-methyl-3-benzyloxy-1-propanethiol hydrochloride
(2R,3S) 2-amino-3-methyl-3-benzyloxy-1-propanethiol hydrochloride
(S) 2-amino-3-benzylthio-1-propanethiol hydrochloride
(R,S) 2-amino-7-phenyl-1-heptanethiol hydrochloride
(R,S) 2-amino-2-methyl-6-phenoxy-1-hexanethiol hydrochloride
(S) 2-amino-3-(4-benzylphenoxy)-1-propanethiol hydrochloride
(R,S) 2-amino-4-phenyl-1-butanethiol hydrochloride
(R,S) 2-amino-6-phenoxy-1-hexanethiol hydrochloride
(R,S) 2-amino-7-phenoxy-1-heptanethiol hydrochloride
(R,S) 2-amino-7-(4-methoxy phenoxy)-1-heptanethiol hydrochloride
(R,S) 2-amino-6-(4-methoxy phenoxy)-1-hexanethiol hydrochloride
(R,S) 2-amino-6-(4-methyl phenoxy)-1-hexanethiol hydrochloride
(R,S) 2-amino-6-(3-methyl phenoxy)-1-hexanethiol hydrochloride
(R,S) 2-amino-6-(3-methoxy phenoxy)-1-hexanethiol hydrochloride
(R,S) 2-amino-6-(4-chloro phenoxy)-1-hexanethiol hydrochloride
(R,S) 2-amino-6-(4-bromo phenoxy)-1-hexanethiol hydrochloride
(R,S) 2-amino-6-(4-fluoro phenoxy)-1-hexanethiol hydrochloride
(R,S) 2-amino-6-(2-methoxy phenoxy)-1-hexanethiol hydrochloride
(R,S) 2-amino-6-phenylthio-1-hexanethiol hydrochloride
(R,S) 2-amino-6-(3,4-dioxymethylenephenoxy)-1-hexanethiol hydrochloride
(R,S) 2-amino-6-(4-ethoxy phenoxy)-1-hexanethiol hydrochloride
(R,S) 2-amino-6-(4-ethyl phenoxy)-1-hexanethiol hydrochloride
(R,S) 2-amino-5-phenyl-1-pentanethiol hydrochloride
(R,S) 2-amino-4-phenoxy-1-butanethiol hydrochloride
(R,S) 2-amino-6-phenyl-1-hexanethiol hydrochloride
(R,S) 2-amino-4-phenylthio-1-butanethiol hydrochloride
(R,S) 2-amino-6-(2,6-dimethyl phenoxy)-1-hexanethiol hydrochloride
(R,S) 2-amino-4-benzylthio-1-butanethiol hydrochloride
(R,S) 2-amino-5-phenoxy-1-pentanethiol hydrochloride
(R,S) 2-amino-6-(2-methylphenox-y)-1-hexanethiol hydrochloride
(R,S) 2-amino-6-(4-phenoxyphenoxy)-1-hexanethiol hydrochloride
(R,S) 2-amino-6-(4-carboxyphenoxy)-1-hexanethiol hydrochloride
(R,S) 2-amino-6-(4-cyano phenoxy)-1-hexanethiol hydrochloride
[2(R,S)-3(R,S)] 2-amino-3-methyl-6-phenoxy-1-hexanethiol hydrochloride
(R,S) 2-amino-6-(4-phenyl phenoxy)-1-hexanethiol hydrochloride
(R,S) 2-amino-6-(4-benzyloxy phenoxy)-1-hexanethiol hydrochloride
(R,S) 2-amino-6-(2-naphthyloxy)-1-hexanethiol hydrochloride
(R,S) 2-amino-6-(1-naphthyloxy)-1-hexanethiol hydrochloride
(R,S) 2-amino-8-phenoxy-1-octanethiol hydrochloride
(R,S) 2-amino-9-phenoxy-1-nonanethiol hydrochloride
(R,S) 2-amino-6-(3-trifluoromethyl phenoxy)-1-hexanethiol hydrochloride
(R,S) 2-amino-6-(3-fluorophenoxy)-1-hexanethiol hydrochloride
(R,S) 2-amino-6-(2,4-difluorophenoxy)-1-hexanethiol hydrochloride
(R,S) 2-amino-6-(2-fluoro-phenoxy)-1-hexanethiol hydrochloride
(R,S) 2-amino-6-pentafluorophenoxy-1-hexanethiol hydrochloride
(R,S) 2-amino-6-(4-nitrophenoxy)-1-hexanethiol hydrochloride
(R,S) 2-amino-8-phenxyl-1-octanethiol hydrochloride
(R,S) 2-amino-6-(3,5-dimethoxyphenoxy)-1-hexanethiol hydrochloride
(R,S) 2-amino-6-(4-butoxyphenoxy)-1-hexanethiol hydrochloride
(R,S) 2-amino-6-(4,5-dichlorophenoxy)-1-hexanethiol hydrochloride
(R,S) 2-amino-6-(2-pyridoxy)-1-hexanethiol hydrochloride
(R,S) 2-amino-6-(3-cyanophenoxy)-1-hexanethiol hydrochloride
(R,S) 2-amino-5-(4-benzylphenoxy)-1-pentanethiol hydrochloride
(R,5) 2-amino-6-(3-chlorophenoxy)-1-hexanethiol hydrochloride
(R,S) 2-amino-7-(4-cyanophenoxy)-1-heptanethiol hydrochloride
(R,S) 2-amino-5-(4-cyanophenoxy)-1-pentanethiol hydrochloride
(R,S) 2-amino-6-(3-ethylphenoxy)-1-hexanethiol hydrochloride
(R,S) 2-amino-6-(4-triffuoromethylphenoxy)-1-hexanethiol hydrochloride
(R,S) 2-amino-7-(4-benzylphenoxy)-1-heptanethiol hydrochloride
(R,S) 2-amino-6-(4-benzylphenoxy)-1-hexanethiol hydrochloride
(R,S) 2-amino-5-(3-ethylphenoxy)-1-pentanethiol hydrochloride
(R,S) 2-amino-5-(4-methylphenoxy)-1-pentanethiol hydrochloride
(R,S) 2-amino-6-(4-acetamidophenoxy)-1-hexanethiol hydrochloride
(R,S) 2-amino-6-(4-iodophenoxy)-1-hexanethiol hydrochloride
(R,S) 2-amino-6-(4-propoxyphenoxy)-1-hexanethiol hydrochloride
(R,S) 2-amino-5-(4-benzoylphenoxy)-1-pentanethiol hydrochloride
(R,S) 2-amino-5-(4-ethoxyphenoxy)-1-pentanechiol hydrochloride
(R,S) 2-amino-7-(4-ethoxyphenoxy)-1-heptanethiol hydrochloride
(+) 2-amino-6-(4-ethoxyphenoxy)-1-hexanethiol hydrochloride
(-) 2-amino-6-(4-ethoxyphenoxy)-1-hexanethiol hydrochloride
(+) 2-amino-6-phenoxy-1-hexanethiol hydrochloride
(-) 2-amino-6-phenoxy)-1-hexanethiol hydrochloride.

34. Compounds according to one of claims 1 to 8, 10, 11, 12, 14 to 32 and 33, **characterised in that** R³ denotes the group 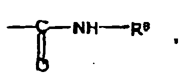 where R⁶ has the meaning given in claim 1.

35. Compounds according to one of claims 1 to 8, 10, 11, 12, 14 to 32 and 33, **characterised in that** R³ denotes the group 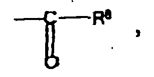 where R⁶ has the meaning given in claim 1.

36. Compounds according to one of claims 8, 10, 11, 12, 14 to 32 and 33, **characterised in that** R³ denotes the group

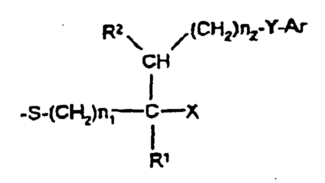

37. Compounds according to one of claims 34 to 36, **characterised in that** they are selected from among:
1,1-dithiobis (2-(R,S)-amino-6-phenoxy hexane) dihydrochloride
(R,S) 2-amino-6-phenoay-1-S-acetylthio hexane hydrobromide
1,1-dithiobis (2-(+)-amino-6-phenoay-hexane) dihydrochloride (isomer A)
1,1-dithiobis (2-(-)-amino-6-phenoxy-hexane) dihydrochloride (isomer A)
1,1-dithiobis (2-(R,S)-amino-6-(4-ethoxyphenoxy)-1-hexane) dihydrochloride
1,1-dithiobis (2-(+)-amino-6-(4-ethoxyphenoxy)-1-hexane) dihydrochloride (isomer A)
1,1-dithiobis (2-(-)-amino-6-(4-ethoxyphenoxy)-1-hexane) dihydrochloride (isomer B)
1,1-dithiobis (2-(R,S)-amino-6-(4-acetamido-phenoxyl)-1-hexane dihydrochloride
1,1-dithiobis (2-(R,S)-amino-6-(4-cyanophenoxy)-1-hexane dihydrochloride

38. Pharmaceutical composition, **characterised in that** it contains as active ingredient a therapeutically effective quantity of a compound according to one of claims 1 to 37, combined with a physiologically acceptable carrier or excipient.

39. Use of a compound of general formula I 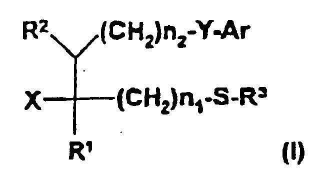
wherein:
• X denotes -NH₂
• R¹ and R² are independently selected from among the following groups:
i) a hydrogen atom
ii) a straight-chain or branched alkyl group containing 1 to 6 carbon atoms;
• n₁ varies from 1 to 4
• n₂ varies from 0 to 10
• R³ is selected from among the following groups:
i) a hydrogen atom
ii) 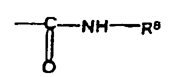
iii) 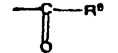
iv) 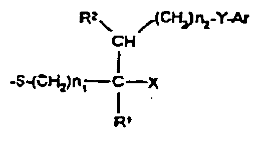
• Y is selected from among the following groups:
i) -O-
ii) -CH₂-
iii) -S-
iv) -OCH₂-
v) -SCH₂-
vi) -NH
• Ar is selected from among the following groups:
i) a phenyl group which is unsubstituted or mono- or polysubstituted by substituents selected from among the halogen atoms and the groups CF₃, straight-chain or branched alkyl containing 1 to 6 carbon atoms, straight-chain or branched alkoxy containing 1 to 6 carbon atoms, NH₂, NO₂, CN, OH, CO₂H, OPh, OCH₂Ph, SMe, SEt, Ph, CH₂Ph and NHCOR⁷ where R⁷ is a straight-chain or branched alkyl group containing 1 to 6 carbon atoms,
ii) 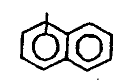
iii) 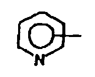
iv) 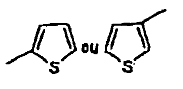
v) 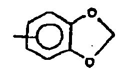
vi) 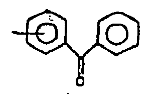
• R⁶ denotes a straight-chain or branched alkyl group containing 1 to 6 carbon atoms or a phenyl group, for the manufacture of a medicament intended to inhibit the activity of LTA₄.

40. Use of a compound of formula I according to claim 39 for the production of a medicament intended for anti-inflammatory treatment.

41. Use of a compound of formula I according to claim 39 for the production of a medicament intended for anti-arthritic treatment.

42. Use of a compound of formula I according to claim 39 for the production of a medicament intended for anti-psoriatic treatment.

43. Use of a compound of formula I according to claim 39 for the production of a medicament intended for hepato-protective treatment.

44. Use of a compound of formula I according to claim 39 for the production of a medicament intended for anti-mitotic treatment.

45. Use of a compound of formula I according to claim 39 for the production of a medicament intended to treat overproduction of LTB₄ induced in particular by a cyclooxygenase inhibitor.

46. Pharmaceutical composition, **characterised in that** it contains, as active ingredient, a therapeutically effective quantity of a compound of formula (I) according to claim 39 and a therapeutically effective quantity of a cyclooxygenase inhibitor.

47. Composition according to claim 46, further comprising a physiologically acceptable carrier or excipient.
